(19) Europäisches Patentamt
European Patent Office
Office européen des brevets

(11) Veröffentlichungsnummer: **0 226 196**
**B1**

(12) **EUROPÄISCHE PATENTSCHRIFT**

(45) Veröffentlichungstag der Patentschrift: **22.08.90**

(21) Anmeldenummer: **86117347.4**

(22) Anmeldetag: **12.12.86**

(51) Int. Cl.[5]: **C 07 D 471/04,**
C 07 D 495/14,
C 07 D 513/04,
C 07 D 513/14,
C 07 D 513/20,
C 07 D 513/22, A 61 K 31/50
// (C07D471/04, 237:00,
221:00),(C07D495/14, 333:00,
237:00, 221:00),(C07D513/04,
277:00, 237:00),(C07D513/14,
333:00, 277:00,
237:00),(C07D513/20, 333:00,
277:00, 237:00),(C07D513/22,
333:00)

(54) **Tricyclische Pyridonderivate.**

(30) Priorität: **13.12.85 CH 5324/85**
**01.10.86 CH 3922/86**

(43) Veröffentlichungstag der Anmeldung:
**24.06.87 Patentblatt 87/26**

(45) Bekanntmachung des Hinweises auf die Patenterteilung:
**22.08.90 Patentblatt 90/34**

(84) Benannte Vertragsstaaten:
**AT BE CH DE ES FR GB GR IT LI LU NL SE**

(56) Entgegenhaltungen:
**EP-A-0 183 994**

(73) Patentinhaber: **F. HOFFMANN-LA ROCHE AG**
**Postfach 3255**
**CH-4002 Basel (CH)**

(72) Erfinder: **Fischer, Ulf, Dr.**
**Madlenweg 2**
**CH-4402 Frenkendorf (CH)**
Erfinder: **Schneider, Fernand, Dr.**
**Marignanostrasse 28**
**CH-4059 Basel (CH)**
Erfinder: **Widmer, Ulrich, Dr.**
**Alleeweg 13**
**CH-4310 Rheinfelden (CH)**

(74) Vertreter: **Lederer, Franz, Dr. et al**
**Patentanwalt Dr. Franz Lederer Lucile-Grahn-Strasse 22**
**D-8000 München 80 (DE)**

**EP 0 226 196 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Verbindungen der allgemeinen Formel

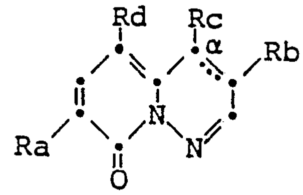

I

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_\alpha—S—CH=CH—$ (a), $>C_\alpha—CH=CH—S—$ (b) oder $>C_\alpha—CH=CH—CH=CH—$ (c), die gestrichelte Linie eine zusätzliche Bindung, Rd die Gruppe der Formel $—(A^1)_m—(CO)_n—(Q^1A^2)_q—R^1$, m, n und q je die Zahl 0 oder 1, $A^1$ niederes Alkylen, $A^2$ niederes Alkylen, eine direkte Bindung oder die Gruppe —CO—, $Q^1$ ein Sauerstoffatom oder die Gruppe $—NR^2—$, $R^1$ Wasserstoff, Hydroxy, Cyano, Nitro, Halogen, niederes Alkoxy, niederes Alkyl, niederes Alkoxycarbonyl, Aryl, eine Gruppe der Formel $—NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N—R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Massgabe, dass n die Zahl 0 bedeutet, wenn q die Zahl 1 und $A^2$ die Gruppe —CO— bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die Gruppe —CO— bedeuten, dass $R^1$ eine von Hydroxy, Cyano, Nitro, Halogen, niederes Alkoxycarbonyl, niederes Alkoxy und $—NR^3R^4$ verschiedene Bedeutung hat, wenn q die Zahl 1 und $A^2$ eine direkte Bindung bedeuten, und dass Rd eine von Acetyl verschiedene Bedeutung hat, wenn Ra Phenyl und Rb und Rc zusammen die Gruppe —CH=CH—CH=CH— bedeuten, und pharmazeutisch annehmbare Säureadditionssalze von Verbindungen der Formel I mit einem oder mehreren basischen Substituenten.

Diese tricyclischen Pyridonderivate besitzen wertvolle pharmakologische Eigenschaften und können zur Bekämpfung oder Verhütung von Krankheiten verwendet werden. Sie sind insbesondere muskelrelaxierend, sedative-hypnotisch, anxiolytisch und/oder antikonvulsiv wirksam und können demnach bei der Bekämpfung oder Verhütung von Muskelspannungen, Spannungszuständen, Schlaflosigkeit, Angstzuständen und/oder Konvulsionen verwendet werden.

In der EP—A—183 994 werden Verbindungen beschrieben, welche die gleichen Eigenschaften besitzen und welche der Formel I ensprechen, jedoch austelle des Stickstoffatoms im Pyridazinring eine Methingruppe besitzen.

Gegenstand der vorliegenden Erfindung sind: Die obigen Verbindungen der Formel I und die erwähnten Salze davon als solche; eine Verfahren und Zwischenprodukte zu deren Herstellung; die Verwendung der Zwischenprodukte zur Herstellung von therapeutischen Wirkstoffen; die obigen Verbindungen der Formel I und die erwähnten Salze davon zur Anwendung als therapeutische Wirkstoffe; Arzneimittel auf der Basis dieser neuen Wirkstoffe und deren Herstellung; die Verwendung dieser Wirkstoffe zur Herstellung von muskelrelaxierend, sedativ-hypnotisch, anxiolytisch und/oder antikonvulsiv wirksamen Mitteln.

Der Ausdruck "nieder" bezeichnet Reste und Verbindungen mit höchstens sieben, vorzugsweise höchstens vier Kohlenstoffatomen. Der Ausdruck "Alkyl" für sich allein genommen oder in Kombinationen, wie Alkanoyl, Alkanoyloxy und Alkoxyalkyl, bezeichnet geradkettige oder verzweigte, gesättigte Kohlenwasserstoffreste, wie Methyl, Aethyl, Propyl, Isopropyl und t-Butyl. Der Ausdruck "Cycloalkyl" bezeichnet

cyclische, gesättigte Kohlenwasserstoffreste, wie Cyclohexyl. Der Ausdruck "Alkoxy" bezeichnet über ein Sauerstoffatom gebundene Alkylgruppen, wie Methoxy und Aethoxy. Der Ausdruck "Hydroxyalkyl" bezeichnet durch Hydroxy substituierte Alkylgruppen, wie 2-Hydroxyäthyl. Die Ausdrücke "Alkanoyl" und "Alkanoyloxy" bezeichnen Fettsäurereste, wie Acetyl und Acetoxy. Der Ausdruck "Alkylen" bezeichnet geradkettige oder verzweigte, gesättigte Kohlenwasserstoffreste mit zwei freien Valenzen, wie Methylen, 1,2-Aethylen und 1,3-Propylen. Der Ausdruck "Halogen" bezeichnet die vier Formen Fluor, Chlor, Brom und Jod.

Der Ausdruck "Aryl" bezeichnet vorzugsweise Phenylgruppen, welche gegebenenfalls durch Halogen, Trifluormethyl, niederes Alkyl, niederes Alkoxy, Nitro, Amino oder mono- oder di(niederes Alkyl)amino substituiert sind.

Die über ein Kohlenstoffatom gebundenen, 5-gliedrigen, gesättigten, partiell ungesättigten oder aromatischen Heterocyclen enthalten als Heteroringglieder vorzugsweise ein Sauerstoff- oder Schwefelatom oder eine Imino- oder niedere Alkyliminogruppe und gegebenenfalls ein oder zwei Stickstoffatome, wobei das Kohlenstoffatom, über das der Heterocyclus gebunden ist, sich vorzugsweise neben einem oder zwischen zwei Heteroatomen befindet. Beispiele solcher Heterocyclen, welche noch wie erwähnt substituiert sein können, sind: 2-Oxazolin-2-yl, 3-Methyl-1,2,4-oxadiazol-5-yl, 3-Cyclopropyl-1,2,4-oxadiazol-5-yl, 2-Thiazolin-2-yl, 2-Tetrahydrofuryl und 2-Thiazolyl.

Der Ausdruck "3- bis 7-gliedrigen, gesättigter N-Heterocyclus, der als Ringglied noch ein Sauerstoff- oder Schwefelatom oder die Gruppe $>N-R^5$ enthalten kann" als Bedeutungsmöglichkeit für $-NR^3R^4$ bezeichnet einerseits Heterocyclen mit nur einem Heteroatom, nämlich dem Stickstoffatom, über das sie gebunden sind, und andererseits Heterocyclen mit zwei Heteroatomen, nämlich dem besagten Stickstoffatom und einem Sauerstoff- oder Schwefelatom oder einem zweiten Stickstoffatom. Beispiele derartiger Heterocyclen, welche noch wie erwähnt substituiert sein können, sind: 2-(niederes Alkoxyalkyl)-1-azetidinyl, 3-(niederes Alkoxy)-1-azetidinyl, 3-Hydroxy-1-azetidinyl, 2-(niederes Hydroxyalkyl)-1-azetidinyl, 2-(niederes Alkanoyloxyalkyl)-1-pyrrolidinyl, 3-Oxo-1-pyrrolidinyl, 2-(niederes Alkoxycarbonyl)-1-pyrrolidinyl, 3-(niederes Alkoxy)-1-pyrrolidinyl, 3-Hydroxy-1-pyrrolidinyl, 2-(niederes Alkoxyalkyl)-1-pyrrolidinyl, 2-(niederes Hydroxyalkyl)-1-pyrrolidinyl, 2-(niederes Hydroxyalkyl)-4-hydroxy-1-pyrrolidinyl, 2-(niederes Alkoxyalkyl)-4-(niederes Alkoxy)-1-pyrrolidinyl, 4-Morpholinyl, 2,6-Di(niederes Alkyl)-4-morpholinyl, 4-Thiomorpholinyl, 1-Piperazinyl, 1-(niederes Alkyl)-4-piperazinyl, 1-(niederes Alkoxylalkyl)-4-piperazinyl, 1-(niederes Alkanoyl)-4-piperazinyl, 4-(niederes Hydroxyalkyl)-1-piperidinyl, 4-Oxo-1-piperidinyl, 4-(niederes Alkoxy)-1-piperidinyl, 4-niederes Alkoxycarbonyl)-1-piperidinyl, 4-Hydroxy-1-piperidinyl, 4-(niederes Alkylcarbamoyl)-1-piperidinyl, 4-(niederes Alkanoyloxy)-1-piperidinyl, 2-(niederes Alkoxyalkyl)-1-piperidinyl, 2-(niederes Hydroxyalkyl)-1-piperidinyl, 3-(niederes Alkoxy)-1-piperidinyl, 4,4-(niederes Alkylendioxy)-1-piperidinyl und 3-Hydroxy-1-piperidinyl.

Das Symbol Ra bedeutet vorzugsweise eine gegebenenfalls durch m-Halogen oder m-Trifluormethyl substituierte Phenylgruppe, wobei die Bedeutungsmöglichkeit Phenyl besonders bevorzugt ist.

Die Symbole Rb und Rc bedeuten zusammen mit dem mit α bezeichneten Kohlenstoffatom vorzugsweise eine gegebenenfalls durch Halogen substituierte Gruppe der Formel $>C_\alpha-S-CH=CH-$ (a) oder $>C_\alpha-CH=CH-CH=CH-$ (c), insbesondere die Gruppe der Formel $>C_\alpha-S-CH=CH-$ oder $>C_\alpha-CH=CCl-CH=CH-$, wobei die gestrichelte Linie eine zusätzliche Bindung bedeutet.

In einer bevorzugten Ausführungsform bedeuten $Q^1$ ein Sauerstoffatom, $A^2$ die Gruppe $-CO-$, $R^1$ die Gruppe $-NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann, und entweder m und q die Zahl 0 und n die Zahl 1 oder m und q die Zahl 1 und n die Zahl 0. In einer besonders bevorzugten Ausführungsform bedeuten $A^1$ Methylen, $Q^1$ ein Sauerstoffatom, $A^2$ die Gruppe $-CO-$, $R^1$ die Gruppe $-NR^3R^4$, $R^3$ niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten 1-Azetidinyl-, 1-Pyrrolidinyl-, 1-Piperidinyl- oder 4-Morpholinylgruppe und entweder m und q die Zahl 0 und n die Zahl 1 oder m und q die Zahl 1 und n die Zahl 0. In einer ganz besonders bevorzugten Ausführungsform bedeuten $A^1$ Methylen, $Q^1$ ein Sauerstoffatom, $A^2$ die Gruppe $-CO-$, $R^1$ die Gruppe $-NR^3R^4$, $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl, 2-(niederes Hydroxyalkyl)-1-pyrrolidinyl, 4-Hydroxy-1-piperidinyl, 4-(niederes Alkoxy)-1-piperidinyl, 4-Morpholinyl oder 2,6-Di(niederes Alkyl)-4-morpholinyl und entweder m und q die Zahl 0 und n die Zahl 1 oder m und q die Zahl 1 und n die Zahl 0. In einer speziellen Ausführungsform bedeuten m und q die Zahl 0 und n die Zahl 1. In einer weiteren speziellen Ausführungsform bedeuten m und q die Zahl 0, n die Zahl 1 und $R^1$ Hydroxy oder niederes Alkoxy.

Die nachfolgend aufgeführten Verbindung sind bevorzugte Vertreter der durch die Formel I definierten Stoffklasse:

3-Methoxy-1-[(4-oxo-3-phenyl-4H-pyrido[2,1-a]phthalazin-1-yl)carbonyl]azetidin,

N,N-Diäthyl-4-oxo-3-phenyl-4H-pyrido[2,1-a]phthalazin-1-carboxamid,

4-[(4-Oxo-3-phenyl-4H-pyrido[2,1-a]phthalazin-1-yl)carbonyl]morpholin,

(S)-2-(Methoxymethyl)-1-[(10-chlor-4-oxo-3-phenyl-4H-pyrido[2,1-a]phthalazin-1-yl)carbonyl]-pyrrolidin,
N-Aethyl-N-(2-methoxyäthyl)-10-chlor-4-oxo-3-phenyl-4H-pyrido[2,1-a]phthalazin-1-carboxamid,
1-[(10-Chlor-4-oxo-3-phenyl-4H-pyrido[2,1-a]phthalazin-1-yl)carbonyl]-3-methoxyazetidin,
N,N-Dimethyl-10-chlor-4-oxo-3-phenyl-4H-pyrido[2,1-a]phthalazin-1-carboxamid,
N,N-Diäthyl-10-chlor-4-oxo-3-phenyl-4H-pyrido[2,1-a]phthalazin-1-carboxamid,
4-[(10-Chlor-4-oxo-3-phenyl-4H-pyrido[2,1-a]phthalazin-1-yl)carbonyl]morpholin,
rac-3-Methoxy-1-[(10-chlor-4-oxo-3-phenyl-4H-pyrido[2,1-a]phthalazin-1-yl)carbonyl]pyrrolidin,
(R)-2-(Methoxymethyl)-1-[(10-chlor-4-oxo-3-phenyl-4H-pyrido[2,1-a]phthalazin-1-yl)carbonyl]-pyrrolidin,
N,N-Diäthyl-7-oxo-8-phenyl-7H-pyrido[1,2-b]thieno[2,3-d]pyridazin-10-carboxamid,
3-Methoxy-1-[(7-oxo-8-phenyl-7H-pyrido[1,2-b]thieno[2,3-d]pyridazin-10-yl)carbonyl]azetidin,
cis-2,6-Dimethyl-4-[(7-oxo-8-phenyl-7H-pyrido[1,2-b]thieno[2,3-d]pyridazin-10-yl)carbonyl]morpholin,
(R)-2-(Methoxymethyl)-1-[(7-oxo-8-phenyl-7H-pyrido[1,2-b]thieno[2,3-d]pyridazin-10-yl)carbonyl]-pyrrolidin,
(S)-2-(Methoxymethyl)-1-[(7-oxo-8-phenyl-7H-pyrido[1,2-b]thieno[2,3-d]pyridazin-10-yl)carbonyl]-pyrrolidin,
rac-3-Methoxy-1-[(7-oxo-8-phenyl-7H-pyrido[1,2-b]thieno[2,3-d]pyridazin-10-yl)carbonyl]pyrrolidin,
N-(2-Methoxyäthyl)-7-oxo-8-phenyl-7H-pyrido[1,2-b]thieno[2,3-d]pyridazin-10-carboxamid,
4-[(7-Oxo-8-phenyl-7H-pyrido[1,2-b]thieno[2,3-d]pyridazin-10-yl)carbonyl]morpholin,
N-Aethyl-N-(2-methoxyäthyl)-7-oxo-8-phenyl-7H-pyrido[1,2-b]thieno[2,3-d]pyridazin-10-carboxamid,
N-(3-Methoxypropyl)-7-oxo-8-phenyl-7H-pyrido[1,2-b]thieno[2,3-d]pyridazin-10-carboxamid,
N,N-Dimethyl-7-oxo-8-phenyl-7H-pyrido[1,2-b]thieno[2,3-d]pyridazin-10-carboxamid und
N-Methyl-7-oxo-8-phenyl-7H-pyrido[1,2-b]thieno[2,3-d]pyridazin-10-carboxamid.

Weitere Vertreter der erfindungsgemässen Stoffklasse sind:
4-Oxo-3-phenyl-4H-pyrido[2,1-a]phthalazin-1-carbonsäuremethylester,
7-Oxo-8-phenyl-7H-pyrido[1,2-b]thieno[2,3-d]pyridazin-10-carbonsäuremethylester,
10-chlor-4-oxo-3-phenyl-4H-pyrido[2,1-a]phthalazin-1-carbonsäuremethylester,
4-Oxo-3-phenyl-4H-pyrido[2,1-a]phthalazin-1-carbonsäure,
7-Oxo-8-phenyl-7H-pyrido[1,2-b]thieno[2,3-d]pyridazin-10-carbonsäure,
10-chlor-4-oxo-3-phenyl-4H-pyrido[2,1-a]phthalazin-1-carbonsäure,
3-Hydroxy-1-[(4-oxo-3-phenyl-4H-pyrido[2,1-a]phthalazin-1-yl)carbonyl]azetidin,
(R)-1-[(7-Oxo-8-phenyl-7H-pyrido[1,2-b]thieno[2,3-d]pyridazin-10-yl)carbonyl]-2-pyrrolidinmethanol,
(S)-1-[(7-Oxo-8-phenyl-7H-pyrido[1,2-b]thieno[2,3-d]pyridazin-10-yl)carbonyl]-2-pyrrolidinmethanol
und
(R)-1-[(10-Chlor-4-oxo-3-phenyl-4H-pyrido[2,1-a]phthalazin-1-yl)carbonyl]-2-pyrrolidinmethanol.

Die neuen Verbindungen der Formel I und die pharmazeutisch annehmbaren Säureadditionssalze derselben mit einem basischen Substituenten können erfindungsgemäss hergestellt werden, indem man
a) eine Verbindung der allgemeinen Formel

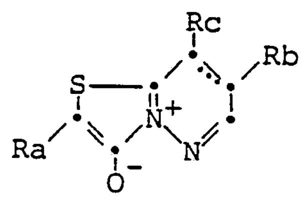

II

worin Ra, Rb, Rc und die gestrichelte Linie die obige Bedeutung besitzen,
bei erhöhter Temperatur mit einer Verbindung der allgemeinen Formel

$$HC{\equiv}C{-}Rd' \qquad III$$

oder

$$H_2C{=}CH{-}Rd' \qquad IV$$

worin Rd' Cyano, Nitro oder die Gruppe der Formel $-CO-(Q^1A^2)_q-R^1$ bedeutet, und q, $A^2$, $Q^1$ und $R^1$ die obige Bedeutung besitzen,
oder mit Phenylvinylsulfoxid umsetzt und das erhaltene Cycloadditionsprodukt gegebenenfalls mit einer starken Base behandelt, oder

b) eine Verbindung der allgemeinen Formel

$$ROOC—C(Ra)=CHR' \qquad V$$

worin R niederes Alkyl und R' Wasserstoff oder niederes Alkoxy bedeuten, und Ra die obige Bedeutung besitzt,
wenn R' Wasserstoff bedeutet, bei erhöhter Temperatur oder, wenn R' niederes Alkoxy bedeutet, in Gegenwart einer starken Base mit einer Verbindung der allgemeinen Formel

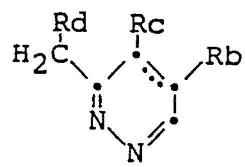

VI

worin Rb, Rc, Rd und die gestrichelte Linie die obige Bedeutung besitzen,
umsetzt und das erhaltene Cyclokondensationsprodukt, wenn R' Wasserstoff bedeutet, dehydriert, oder
c) eine Verbindung der Formel I, welche eine veresterte Carboxygruppe besitzt, hydrolysiert, oder
d) eine Carbonsäure der allgemeinen Formel

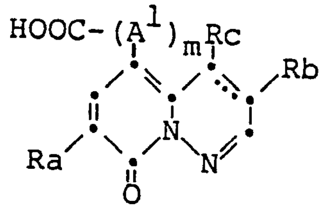

Ia

worin $A^1$, Ra, Rb, Rc, m und die gestrichelte Linie die obige Bedeutung besitzen,
mit einem Alkohol der allgemeinen Formel

$$HO—A^{21}—R^1 \qquad VII$$

worin $A^{21}$ niederes Alkylen oder eine direkte Bindung bedeutet, und $R^1$ die obige Bedeutung besitzt, verestert, oder
e) eine Carbonsäure der obigen Formel Ia oder eine Carbonsäure der allgemeinen Formel

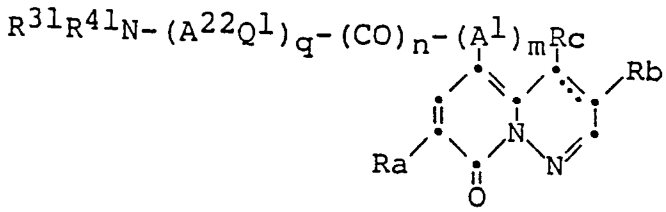

VIIIa

worin $A^{22}$ niederes Alkylen oder die Gruppe —CO— und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- oder Schwefelatom oder die Gruppe $>N—R^5$ enthalten kann, und $A^1$, $Q^1$, Ra, Rb, Rc, $R^5$, m, n, q und die gestrichelte Linie die obige Bedeutung besitzen,
oder ein reaktives Derivat davon mit einem Amin der allgemeinen Formel

$$HNR^2—A^{21}—R^1 \qquad IX$$

oder

$$HNR^3R^4 \qquad X$$

worin $A^{21}$, $R^1$, $R^2$, $R^3$ und $R^4$ die obige Bedeutung besitzen,
bzw. mit Ammoniak oder einem mono- oder di(niederen Alkyl)amin in das entsprechende Amid überführt, oder

f) eine Verbindung der allgemeinen Formel

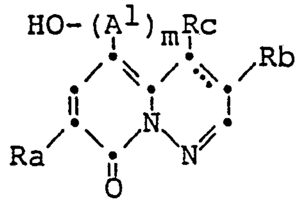

Ib'

worin $A^1$, Ra, Rb, Rc, $R^5$, m und die gestrichelte Linie die obige Bedeutung besitzen, in Gegenwart einer Base mit einer Verbindung der allgemeinen Formel

$$X—A^{21}—R^1 \qquad XI$$

worin X eine Abgangsgruppe bedeutet, und $A^{21}$ und $R^1$ die obige Bedeutung besitzen, bzw. eine Verbindung der Formel I, welche eine freie Hydroxygruppe besitzt, mit einer Verbindung der allgemeinen Formel

$$R—X \qquad XII$$

worin R niederes Alkyl bedeutet, und X obige Bedeutung besitzt, umsetzt, oder

g) eine Verbindung der allgemeinen Formel

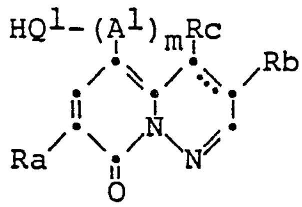

Ib

worin $A^1$, $Q^1$, Ra, Rb, Rc, m und die gestrichelte Linie die obige Bedeutung besitzen, in Gegenwart eines säurebindenden Mittels mit einem reaktiven Derivat einer Carbonsäure der allgemeinen Formel

$$R^1—COOH \qquad XIII$$

worin $R^1$ die obige Bedeutung besitzt, umsetzt, oder

h) eine Verbindung der allgemeinen Formel

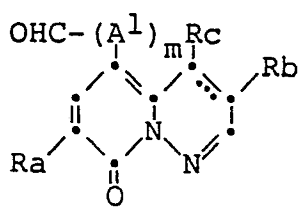

Ic

worin $A^1$, Ra, Rb, Rc, m und die gestrichelte Linie die obige Bedeutung besitzen, in Gegenwart eines Reduktionsmittels mit einem Amin der obigen Formel IX oder X umsetzt, oder

i) eine Verbindung der allgemeinen Formel

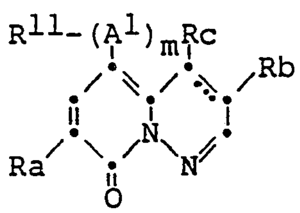

Id

worin $R^{11}$ Nitro, Cyano oder niederes Alkoxycarbonyl bedeutet, und $A^1$, Ra, Rb, Rc, m und die gestrichelte Linie die obige Bedeutung besitzen,

oder eine Verbindung der obigen Formel Ia oder ein reaktives Derivat davon reduziert, oder

j) einen Alkohol der obigen Formel Ib' oder einen Alkohol der allgemeinen Formel

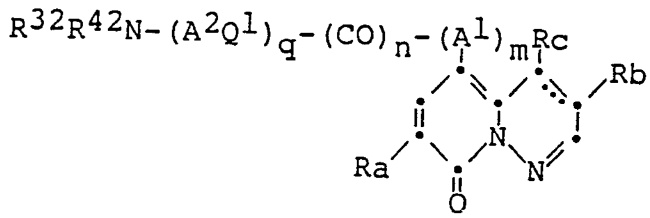

Ie

worin $A^1$, $A^2$, $Q^1$, Ra, Rb, Rc, m, n, q und die gestrichelte Linie die obige Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigten N-Heterocyclus bedeuten, der als Ringglied noch ein Sauerstoff- oder Schwefelatom oder die Gruppe >N—$R^5$ enthalten kann, und $R^5$ die obige Bedeutung besitzt,

oxidiert, oder

k) ein Isocyanat der allgemeinen Formel

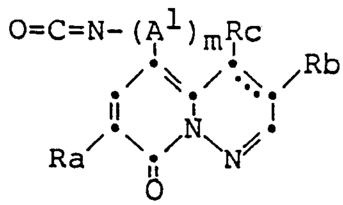

VIIIb

oder $O{=}C{-}N{-}R^{33}$ XIV

worin $A^1$, Ra, Rb, Rc, m und die gestrichelte Linie die obige Bedeutung besitzen, und $R^{33}$ Wasserstoff, niederes Alkyl oder $(C_{3-7})$-Cycloalkyl bedeutet,

mit einem niederen Alkohol oder einem Amin der obigen Formel X bzw. mit einer Verbindung der obigen Formel Ib umsetzt, oder

l) eine Verbindung der allgemeinen Formel

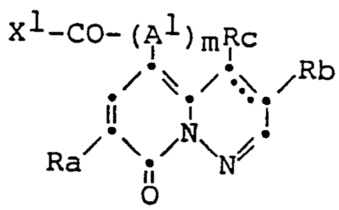

VIIIc

worin $X^1$ ein Halogenatom bedeutet, und $A^1$, Ra, Rb, Rc, m und die gestrichelte Linie die obige Bedeutung besitzen,

mit einem niederen Alkylmagnesiumhalogenid umsetzt, oder

m) eine Verbindung der allgemeinen Formel

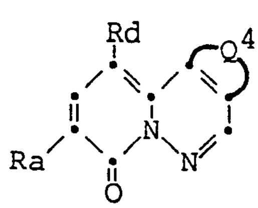

Ih

worin $Q^4$ die obige Gruppe (a) oder (b) bedeutet und Ra und Rd obige Bedeutung besitzen,

am Thiophenring halogeniert, oder

n) eine Verbindung der obigen Formel VIIIc in Gegenwart einer Base mit einer Verbindung der allgemeinen Formel

$$HYN{=}C(NH_2){-}R''$$ XV,

$$H_2N{-}CHR''{-}CHR'''{-}Y'H$$ XVI

oder

$H_2N—NH—C(R'')=Y''$ XVII

worin Y ein Sauerstoffatom oder die Gruppe —NR'''—, Y' ein Sauerstoffatom oder die Gruppe —NH—, Y'' ein Sauerstoff- oder Schwefelatom und R'' und R''' je Wasserstoff oder niederes Alkyl bedeuten, umsetzt und das erhaltene Produkt cyclisiert, oder

o) eine Verbindung der allgemeinen Formel

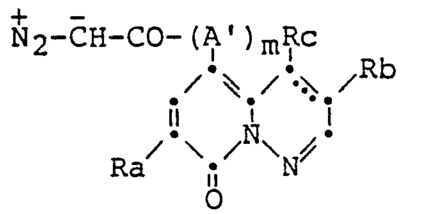

VIIId

worin A' $C_{1-6}$-Alkylen bedeutet, und Ra, Rb, Rc, die gestrichelte Linie und m die obige Bedeutung besitzen,

mit einem niederen Alkohol umsetzt, oder

p) eine Carbonsäure der Formel Ia, worin m die Zahl 0 bedeutet, decarboxyliert, oder

q) eine Verbindung der Formel I, worin Rd Wasserstoff bedeutet, am Pyridonring halogeniert, oder

r) in einer Verbindung der allgemeinen Formel

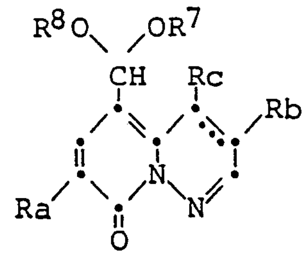

VIIIe

worin $R^7$ und $R^8$ je niederes Alkyl oder zusammen niederes Alkylen bedeuten, und Ra, Rb, Rc und die gestrichelte Linie die obige Bedeutung besitzen,

die Acetalgruppe spaltet, oder

s) eine Verbindung der allgemeinen Formel

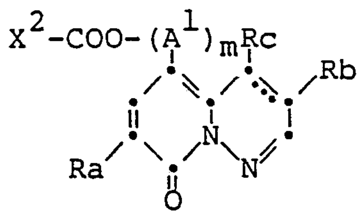

VIIIf

worin $X^2$ Phenoxy bedeutet, und $A^1$, Ra, Rb, Rc, die gestrichelte Linie und m die obige Bedeutung besitzen,

mit einem Amin der obigen Formel X umsetzt, und

t) erwünschtenfalls eine erhaltene Verbindung der Formel I mit einem basischen Substituenten in ein pharmazeutisch annehmbares Säureadditionssalz überführt.

Bei verschiedenen der obigen erfindungsgemässen Verfahren müssen die in den Ausgangsstoffen allfällig vorhandenen reaktionsfähigen Amino-, Carboxy- und/oder Hydroxylgruppen durch Schutzgruppen blockiert sein. Diese Fälle sind für den Fachmann ohne weiteres erkennbar, und auch die Auswahl der jeweils geeigneten Schutzgruppen bereitet ihm keine Schwierigkeiten.

Gemäss Verfahrensvariante a) können Verbindungen der Formel I hergestellt werden, worin Rd Wasserstoff, Cyano, Nitro oder die Gruppe der Formel $—CO—(Q^1A^2)_q—R^1$ bedeutet und q, $A^2$, $Q^1$ und $R^1$ obige Bedeutung besitzen. Die Umsetzung erfolgt zweckmässigerweise in einem inerten Lösungsmittel, das bei erhöhten Temperatur, vorzugsweise oberhalt 80°C siedet. Geeignete Lösungsmittel sind beispielsweise aromatische Kohlenwasserstoff, wie Benzol, Toluol und Xylol, wobei die Reaktion vorzugsweise bei der Rückflusstemperatur des Lösungsmittels durchgeführt wird.

Bei der Umsetzung einer Verbindung der Formel II mit einer Verbindung der Formel III oder mit Phenylvinylsulfoxid erhält man, wenn man bei erhöhter Temperatur arbeitet, direkt die entsprechende Verbindung der Formel I. Bei der Umsetzung einer Verbindung der Formel II mit einer Verbindung der Formel IV erhält man als Cycloadditionsprodukt zunächst die entsprechende Epithio-Verbindung der allgemeinen Formel

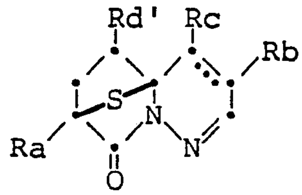

XVIII

worin Ra, Rb, Rc, Rd', und die gestrichelte Linie obige Bedeutung besitzen, welche anschliessend durch Behandeln mit einer starken Base in die entsprechende Verbindung der Formel I übergeführt wird. Geeignete Basen sind beispielsweise die niederen Alkalimetallalkoholate, wie Natriummethylat, wobei man als Lösungsmittel zweckmässigerweise den entsprechenden niederen Alkohol verwendet. Die Reaktion wird vorzugsweise bei der Rückflusstemperatur des Lösungsmittels durchgeführt.

Die Reaktion einer Verbindung der Formel V, worin R' Wasserstoff bedeutet, mit einer Verbindung der Formel VI gemäss Verfahrensvariante b) kann ohne Lösungsmittel oder in Gegenwart eines bei erhöhter Temperatur siedenden Lösungsmittels durchgeführt werden. Geeignete Lösungsmittel sind beispielsweise aromatische Kohlenwasserstoff, wie Benzol, Toluol und Xylol. Die Cyclokondensation wird jedoch vorzugsweise ohne Lösungsmittel in einem Temperaturbereich von etwa 80°C bis etwa 150°C durchgeführt. Das so erhaltene Cyclokondensationsprodukt, nämlich eine Verbindung der allgemeinen Formel

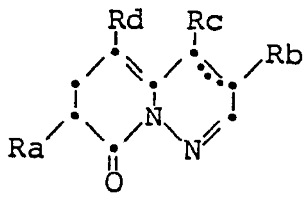

XIX

worin Ra, Rb, Rc, Rd, und die gestrichelte Linie obige Bedeutung besitzen, wird anschliessend mit einem geeigneten Oxidationsmittel, wie Mangandioxid, dehydriert. Geeignete Lösungsmittel sind beispielsweise aromatische Kohlenwasserstoffe, wie Benzol, Toluol und Xylol. Die Dehydrierung wird vorzugsweise in einem Temperaturbereich von etwa Raumtemperatur bis zur Siedetemperatur des gewählten Lösungsmittels, vorzugsweise bei der Siedetemperatur, durchgeführt.

Durch Reaktion einer Verbindung der Formel V, worin R' niederes Alkoxy bedeutet, in Gegenwart einer starken Base, wie Natriumhydrid, und in einem inerten Lösungsmittel, vorzugsweise in einem Aether, wie Tetrahydrofuran, mit einer Verbindung der Formel VI gemäss Verfahrensvariante b) erhält man die entsprechende Verbindung der Formel I. Die Reaktionstemperatur liegt in einem Bereich von Raumtemperatur bis zur Siedetemperatur der Reaktionsmischung.

Die Verbindungen der Formeln XVIII, sofern Rd' eine von Acetyl und Aethoxycarbonyl verschiedene Bedeutung hat, wenn Ra Phenyl, Rb und Rc zusammen die Gruppe —CH=CH—CH=CH— und die gestrichelte Linie eine zusätzliche Bindung bedeuten, und XIX sind neu und ebenfalls Gegenstand der vorliegenden Erfindung.

Verbindungen der Formel I, welche eine veresterte Carboxygruppe besitzen, können gemäss Verfahrensvariante c) hydrolysiert werden, wobei man die entsprechenden freien Carbonsäuren erhält. Die Hydrolyse kann nach an sich bekannten Methoden durchgeführt werden. Die Hydrolyse wird vorzugsweise mit einem Alkalimetallhydroxid, wie Natriumhydroxid und Kaliumhydroxid, in einem niederen Alkohol, wie Methanol und Aethanol, oder in einem Gemisch aus einem niederen Alkohol und Wasser durchgeführt. Die Reaktionstemperatur liegt zweckmässigerweise in einem Bereich von Raumtemperatur bis zur Siedetemperatur des Reaktionsgemisches, wobei man vorzugsweise bei der Siedetemperatur des Reaktionsgemisches arbeitet.

Durch Veresterung einer Carbonsäure der Formel Ia mit einem Alkohol der Formel VII gemäss Verfahrensvariante d) können Verbindungen der Formel I hergestellt werden, worin Rd die Gruppe der Formel $—(A^1)_m—CO—O—A^{21}—R^1$ bedeutet und $A^1$, $A^{21}$, $R^1$ und m obige Bedeutung besitzen. Die Veresterung kann beispielsweise in Gegenwart eines Veresterungsreagenzes in einem inerten organischen Lösungsmittel durchgeführt werden. Geeignete Reagenzien sind beispielsweise N-Methyl-2-chlorpyridiniumjodid und dergleichen, organische Sulfonsäurehalogenide, wie Methylsulfonylchlorid, p-Toluolsulfonylchlorid und Mesitylensulfonsäurechlorid, und dergleichen. Geeignete Lösungsmittel sind beispielsweise halogenierte Kohlenwasserstoffe, wie Methylenchlorid, Chloroform und dergleichen. Geeignete Basen sind beispielsweise tertiäre Amine, wie Triäthylamin, Tri-n-butylamin und dergleichen. Die Reaktion wird vorzugsweise in einem Temperaturbereich von Raumtemperatur bis zur Rückflusstemperatur des Lösungsmittels durchgeführt.

Die gewünschte Veresterung kann auch dadurch bewerkstelligt werden, dass man die Carbonsäure der Formel Ia zunächst in ein reaktives Derivat überführt und dieses dann in Gegenwart einer Base mit einem Alkohol der Formel VII umsetzt. Als reaktive Derivate verwendet man vorzugsweise die entsprechenden Carbonsäurechloride. Als Basen eignen sich beispielsweise die bereits erwähnten tertiären Amine. Die

Reaktion wird vorzugsweise in einem Temperaturbereich von etwa Raumtemperatur bis zur Rückflüsstemperatur des Reaktionsgemisches durchgeführt, wobei man zweckmässigerweise bei Raumtemperatur arbeitet.

Die Veresterung mit einem Alkohol der Formel VII, worin $A^{21}$ niederes Alkylen und $R^1$ Wasserstoff bedeuten, d.h. mit einem niederen Alkohol, kann auch durch Umsetzen der Carbonsäure mit einem di-(niederen Alkyl)acetal des N,N-Dimethylformamids durchgeführt werden. Die Umsetzung mit einem di-(niederen Alkyl)acetal des N,N-dimethylformamids wird vorzugsweise in einem inerten Lösungsmittel, beispielsweise in einem aromatischen Kohlenwasserstoff, wie Benzol, bei der Rückflusstemperatur des Reaktionsgemisches durchgeführt.

Durch Reaktion einer Carbonsäure der Formel Ia oder eines reaktiven Derivates davon mit einem Amin der Formel IX oder X gemäss Verfahrensvariante 3) können Verbindungen der Formel I hergestellt werden, worin Rd die Gruppe $-(A^1)_m-CO-NR^2-A^{21}-R^1$ oder $-(A^1)_m-CO-NR^3R^4$ bedeutet und $A^1$, $A^{21}$, $R^1$, $R^2$, $R^3$, $R^4$ und m obige Bedeutung besitzen.

Durch Umsetzen einer Carbonsäure der Formel VIIIa oder eines reaktiven Derivates davon mit Ammoniak oder einem mono- oder di(niederen Alkyl)amin gemäss Verfahrensvariante e) können entsprechende Verbindungen der Formel I hergestellt werden, worin $R^1$ eine Gruppe der Formel $-NR^3R^4$ und $R^3$ und $R^4$ zusammen mit dem Stickstoffatom einen 3- bis 7-gliedrigen, gegebenenfalls durch eine oder zwei niedere Alkylgruppen und durch eine Carbamoyl- oder mono- oder di(niedere Alkyl)carbamoylgruppe substituierten, gesättigten N-Heterocyclus bedeuten, der als Ringglied noch ein Sauerstoff- oder Schwefelatom oder die Gruppe $>N-R^5$ enthalten kann, und $R^5$ obige Bedeutung besitzt.

Verwendet man als Ausgangsstoff die freie Carbonsäure der Formel Ia oder VIIIa, so wird die Amidierungsreaktion vorzugsweise in Gegenwart eines Kondensationsmittels, wie N-Methyl-2-chlorpyridiniumjodid, in einem inerten organischen Lösungsmittel und in Gegenwart einer Base durchgeführt. Geeignete Lösungsmittel sind beispielsweise aromatische Kohlenwasserstoffe wie Benzol, Toluol und Xylol. Geeignete Basen sind beispielsweise die weiter oben erwähnten tertiären Amine. Bevorzugte reaktive Carbonsäurederivate, welche in Gegenwart einer Base direkt mit dem entsprechenden Amin umgesetzt werden können, sind die entsprechenden Carbonsäurechloride. Geeignete Basen sind wiederum die vorerwähnten tertiären Amine. Geeignete Lösungsmittel sind beispielsweise aromatische Kohlenwasserstoffe, wie Benzol, Toluol und Xylol, und Aether, wie Dioxan. Die Reaktion wird in beiden Fällen vorzugsweise in einem Bereich von Raumtemperatur bis Rückflusstemperatur des Reaktionsgemisches durchgeführt.

Gemäss Verfahrensvariante f) können einerseits Verbindungen der Formel I hergestellt werden, worin Rd eine Gruppe der Formel $-(A^1)_m-O-A^{21}-R^1$ bedeutet, und $A^1$, $A^{21}$, $R^1$ und m obige Bedeutung besitzen, und andererseits Verbindungen der Formel I, welche eine in Form eines niederen Alkyläthers verätherte Hydroxygruppe besitzen.

Die Umsetzung einer Verbindung der Formel Ib' mit einer Verbindung der Formel XI bzw. die Umsetzung einer Verbindung der Formel I, welche eine freie Hydroxygruppe besitzt, mit einer Verbindung der Formel XII wird zweckmässigerweise in einem inerten organischen Lösungsmittel, wie N,N-Dimethylformamid oder dergleichen, durchgeführt, wobei man als Base zweckmässigerweise eine starke Base, z.B. ein Alkalimetallhydrid oder -hydroxyd, wie Natriumhydrid, Kaliumhydroxyd und Natriumhydroxyd, verwendet. Man arbeitet zweckmässigerweise in einem Bereich von 0°C bis Raumtemperatur. Die mit X bezeichnete Abgangsgruppe ist vorzugsweise ein Halogenatom, insbesondere ein Chlor-, Brom- oder Jodatom, oder eine Alkyl- oder Arylsulfonyloxygruppe, beispielsweise eine Methansulfonyloxy- oder p-Toluolsulfonyloxygruppe. Im Falle der Herstellung von niederen Alkyläthern kann X auch eine niedere Alkoxysulfonyloxygruppe bedeuten, d.h. dass das Alkylierungsmittel in diesem Fall ein Di(niederes Alkyl)-sulfat, wie Dimethylsulfat, ist.

Gemäss Verfahrensvariante g) können Verbindungen der Formel I hergestellt werden, worin Rd eine Gruppe der Formel $-(A^1)_m-Q^1-CO-R^1$ bedeutet, und $A^1$, $Q^1$, $R^1$ und m obige Bedeutung besitzen.

Die Umsetzung einer Verbindung der Formel Ib mit einem reaktiven Derivat einer Carbonsäure der Formel XIII, beispielsweise einem Carbonsäurechlorid, wird zweckmässigerweise in einem inerten organischen Lösungsmittel in Gegenwart eines säurebindenden Mittels, beispielsweise einem tertiären Amin, durchgeführt. Als Lösungsmittel eignen sich beispielsweise aromatische Kohlenwasserstoffe, wie Benzol, Toluol und Xylol, und halogenierte Kohlenwasserstoffe, wie Methylenchlorid. Wenn $R^1$ niederes Alkyl bedeutet, können auch entsprechende Carbonsäureanhydride verwendet werden, wobei man in diesem Fall als Lösungsmittel und als säurebindendes Mittel zweckmässigerweise Pyridin verwendet. Die Reaktion wird vorzugsweise in einem Temperaturbereich von etwa 0°C bis Siedetemperatur des Lösungsmittels durchgeführt.

Gemäss Verfahrensvariante h) können Verbindungen der Formel I hergestellt werden, worin Rd eine Gruppe der Formel $-(A^1)_m-CH_2-NR^2A^{21}-R^1$ oder $-(A^1)_m-CH_2-NR^3R^4$ bedeutet und $A^1$, $R^{21}$, $R^1$, $R^2$, $R^3$, $R^4$ und m obige Bedeutung besitzen. Die Reaktion wird vorzugsweise in einem niederen Alkohol als Lösungsmittel und mit Natriumcyanoborhydrid als Reduktionsmittel durchgeführt, wobei man zweckmässigerweise bei Raumtemperatur arbeitet und das Amin in Form seines Hydrochlorides einsetzt.

Gemäss Verfahrensvariante i) können Verbindungen der Formel I hergestellt werden, worin Rd eine Gruppe der Formel $-(A^1)_m-R^{12}$ und $R^{12}$ Amino, Aminomethyl, Hydroxymethyl oder Methyl bedeuten und $A^1$ und m obige Bedeutung besitzen. Die Wahl des geeigneten Reduktionsmittels richtet sich einerseits

nach dem verwendeten Ausgangsstoff und andererseits nach dem gewünschten Produkt. Eine Verbindung der Formel Id, worin $R^{11}$ Cyano bedeutet kann beispielsweise mit Diboran in Tetrahydrofuran zur entsprechenden Aminomethylverbindung reduziert werden. Eine Verbindung der Formel Id, worin $R^{11}$ Nitro bedeutet, kann beispielsweise mit Natriumsulfid in einem niederen Alkohol, wie Methanol, zur entsprechenden Aminoverbindung reduziert werden. Eine Verbindung der Formel Id worin $R^{11}$ niederes Alkoxycarbonyl bedeutet, kann mit Lithiumborhydrid, und das Säurechlorid einer Verbindung der Formel Ia mit Natriumborhydrid in Tetrahydrofuran und/oder Dimethylformamid zur entsprechenden Hydroxymethylverbindung reduziert werden. Eine Carbonsäure der Formel Ia kann beispielsweise mit Boran/Tetrahydrofuran-Komplex oder Boran/Methylsulfid-Komplex in Tetrahydrofuran zur entsprechenden Methylverbindung reduziert werden.

Gemäss Verfahrensvariante j) können Verbindungen der Formel I hergestellt werden, worin Rd eine Gruppe der Formel $-(A^1)_m-CHO$ oder $-(A^1)_m-(CO)_n-(Q^1A^2)_q-NR^{34}R^{44}$ und $R^{34}$ und $R^{44}$ zusammen einen 3- bis 7-gliedrigen, gegebenenfalls durch eine oder zwei niedere Alkylgruppen und durch eine Oxogruppe substituierten, gesättigten N-Heterocyclus bedeuten, der als Ringglied noch ein Sauerstoff- oder Schwefelatom oder die Gruppe $>N-R^5$ enthalten kann, und $A^1$, $A^2$, $Q^1$, $R^5$, m, n und q obige Bedeutung besitzen. Die Oxidation von Alkoholen der Formeln Ib' und Ie kann nach an sich bekannten und jedem Fachmann geläufigen Methoden durchgeführt werden. Man kann die gewünschte Oxidation beispielsweise mit Mangandioxid in einem halogenierten Kohlenwasserstoff, wie Methylenchlorid bei Raumtemperatur bewerkstelligen. Die gewünschte Oxidation kann jedoch auch mit Pyridiniumchlorochromat in einem halogenierten Kohlenwasserstoff, wie Methylenchlorid, bei Raumtemperatur, oder mit Dimethylsulfoxid/Trifluoressigsäureanhydrid in einem halogenierten Kohlenwasserstoff, wie Methylenchlorid, bei Temperaturen von etwa $-70°C$ bewerkstelligt werden.

Gemäss Verfahrensvariante k) können durch Umsetzung eines Isocyanates der Formel VIIIb mit einem niederen Alkohol oder einem Amin der Formel X, bzw. durch Umsetzen eines Isocyanates der Formel XIV mit einer Verbindung der Formel Ib Verbindungen der Formel I hergestellt werden, worin Rd eine Gruppe der Formel $-(A^1)_m-NHCO-R^{13}$, $-(A^1)_m-NHCO-NR^3R^4$ oder $-(A^1)_m-Q^1-CO-NH-R^{33}$ und $R^{13}$ niederes Alkoxy bedeuten, und $A^1$, $Q^1$, $R^3$, $R^{33}$, $R^4$ und m obige Bedeutung besitzen. Diese Umsetzung wird zweckmässigerweise in einem inerten Lösungsmittel, beispielsweise in einem aromatischen Kohlenwasserstoff, wie Benzol, Toluol oder Xylol, in einem halogenierten Kohlenwasserstoff, wie Methylenchlorid, oder in einem Aether, wie Dioxan, durchgeführt. Die Reaktion wird vorzugsweise in einem Temperaturbereich von etwa Raumtemperatur bis zur Siedetemperatur des Reaktionsgemisches durchgeführt. Verwendet man ein Isocyanat der Formel XIV, worin $R^{33}$ Wasserstoff bedeutet, als Ausgangsstoff, so wird dieses zweckmässigerweise in geschützter Form eingesetzt. Eine besonders geeignete Schutzgruppe ist dabei die Trichloracetylgruppe, welche nach erfolgter Umsetzung beispielsweise mit Kaliumcarbonat in Wasser durch Hydrolyse wieder entfernt werden kann.

Gemäss Verfahrensvariante l) können Verbindungen der Formel I hergestellt werden, worin Rd eine Gruppe der Formel $-(A^1)_m-CO-R^{14}$ und $R^{14}$ niederes Alkyl bedeuten, und $A^1$ und m obige Bedeutung besitzen. Als Lösungsmittel verwendet man vorzugsweise Aether, wie Tetrahydrofuran. Die Reaktion wird vorzugsweise in einem Temperaturbereich von $-78°C$ bis Raumtemperatur durchgeführt.

Gemäss Verfahrensvariante m) können Verbindungen der Formel I hergestellt werden, worin Rb und Rc zusammen mit dem mit α bezeichneten Kohlenstoffatom eine durch Halogen substituierte Gruppe der Formel $>C_\alpha-S-CH=CH-$ (h) oder $>C_\alpha-CH=CH-S-$ (i) und die gestrichelte Linie eine zusätzliche Bindung bedeuten. Als Halogenierungsmittel verwendet man vorzugsweise elementares Halogen, beispielsweise elementares Brom. Geeignete Lösungsmittel sind beispielsweise halogenierte Kohlenwasserstoffe, wie Chloroform. Man arbeitet zweckmässigerweise in einem Temperaturbereich von 0°C bis etwa Raumtemperatur.

Gemäss Verfahrensvariante n) können Verbindungen der Formel I hergestellt werden, worin Rd eine Gruppe der Formel $-(A^1)_m-R^{15}$ und $R^{15}$ einen über ein Kohlenstoffatom gebundenen, 5-gliedrigen, gegebenenfalls durch eine oder zwei niedere Alkylgruppen substituierten, partiell ungesättigten oder aromatischen Heterocyclus bedeuten, und $A^1$ und m obige Bedeutung besitzen. Die Umsetzung einer Verbindung der Formel VIIIc mit einer Verbindung der Formel XVI, XVII oder XVIII wird zweckmässigerweise in einem inerten Lösungsmittel, beispielsweise in einem halogenierten Kohlenwasserstoff, wie Methylenchlorid, oder in einem aromatischen Kohlenwasserstoff, wie Benzol, Toluol oder Xylol, und in einem Temperaturbereich von etwa 0°C bis zur Rückflusstemperatur des Reaktionsgemisches durchgeführt. Geeignete Basen sind beispielsweise die bereits früher erwähnten tertiären Amine. Die Cyclisierung des so erhaltenen Produktes kann nach an sich bekannten und jedem Fachmann geläufigen Methoden durchgeführt werden. Die Cyclisierung kann beispielsweise in Gegenwart katalytischer Mengen einer starken Säure wie p-Toluolsulfonsäure, unter Entfernung des gebildeten Reaktionswassers mittels eines Schleppers, wie Toluol, bewerkstelligt werden. Die Cyclisierung kann aber auch mittels Azodicarbonsäurediäthylester/Triphenylphosphin in einem Aether, wie Tetrahydrofuran, bewerkstelligt werden.

Gemäss Verfahrensvariante o) können Verbindungen der Formel I hergestellt werden, worin Rd eine Gruppe der Formel $-(A^1)_m-CH_2-R^{16}$, A' $C_{1-6}$-Alkylen und $R^{16}$ niederes Alkoxycarbonyl bedeuten, und m obige Bedeutung besitzt. Die Reaktion eines Diazoketons der Formel VIIId mit einem niederen Alkohol wird vorzugsweise in Gegenwart eines Silberkatalysators, wie Silberoxid, durchgeführt, wobei man als

Lösungsmittel vorzugsweise den niederen Alkohol verwendet. Die Reaktion wird bei erhöhter Temperatur, vorzugsweise bei der Siedetemperatur des Reaktionsgemisches durchgeführt.

Gemäss Verfahrensvariante p) können Verbindungen der Formel I hergestellt werden, worin Rd Wasserstoff bedeutet. Die Decarboxylierung einer Carbonsäure der Formel Ia wird vorzugsweise durch trockenes Erhitzen, insbesondere durch trockenes Erhitzen im Vakuum auf Temperaturen von etwa 200° bis etwa 300°C bewerkstelligt.

Gemäss Verfahrensvariante q) können Verbindungen der Formel I hergestellt werden, worin Rd Halogen bedeutet. Für die Vorliegende Halogenierung geeignete Halogenierungsmittel sind N-Halogenimide und -amide, wie N-Chlorsuccinimid, N-Bromsuccinimid, N-Chloracetamid und dergleichen. Als Lösungsmittel verwendet man vorzugsweise einen halogenierten Kohlenwasserstoff, wie Methylenchlorid, Chloroform, Tetrachlorkohlenstoff und dergleichen. Die Reaktion kann in einem Temperaturbereich von etwa 0°C bis zur Siedetemperatur des Reaktionsgemisches durchgeführt werden. Vorzugsweise arbeitet man bei Raumtemperatur.

Durch Spaltung der Acetalgruppe in einer Verbindung der Formel VIIIe gemäss Verfahrensvariante r) können Verbindungen der Formel I hergestellt werden, worin Rd die Gruppe —CHO bedeutet. Die Spaltung wird vorzugsweise durch Umacetalisieren in Gegenwart einer Säure, wie p-Toluolsulfonsäure, und eines Ketons, wie Cyclohexanon, Aceton und dergleichen bewerkstelligt. Die Reaktion kann in einem Temperaturbereich von Raumtemperatur bis zur Siedetemperatur des Reaktionsgemisches durchgeführt werden.

Gemäss Verfahrensvariante s) können Verbindungen der Formel I hergestellt werden, worin $R^1$ eine Gruppe der Formel $—(A^1)_m—OCO—NR^3R^4$ bedeutet. Für den vorliegenden Zweck geeignete Lösungsmittel sind beispielsweise Aether, wie Tetrahydrofuran, Dioxan und Diäthyläther, N,N-Dimethylformamid und Dimethylsulfoxid. Die Reaktion wird zweckmässigerweise bei Raumtemperatur durchgeführt.

Gemäss Verfahrensvariante t) können Verbindungen der Formel I, welche einen oder mehrere basische Substituenten besitzen, in pharmazeutisch annehmbare Säureadditionssalze übergeführt werden. Derartige Säureadditionssalze können nach an sich bekannten und jedem Fachmann geläufigen Methoden hergestellt werden. Es kommen dabei sowohl Salze mit anorganischen als auch Salze mit organischen Säuren in Betracht, beispielsweise Hydrochloride, Hydrobromide, Sulfate, Nitrate, Citrate, Acetate, Maleate, Succinate, Methansulfonate, p-Toluolsulfonate und dergleichen.

Die als Ausgangsstoffe verwendeten Verbindungen der Formeln II (sofern Ra eine von Phenyl verschiedene Bedeutung hat, wenn Rb und Rc zusammen die Gruppe —CH=CH—CH=CH— und die gestrichtelte Linie eine zusätzliche Bindung bedeuten) VIIIa, VIIIb, VIIIc, VIIId, VIIIe und VIIIf sind neu und ebenfalls Gegenstand der vorliegenden Erfindung. Diese Stoffe können wie nachfolgend beschrieben hergestellt werden.

Die neuen Verbindungen der Formel II können beispielsweise dadurch hergestellt werden, dass man eine Verbindung der allgemeinen Formel

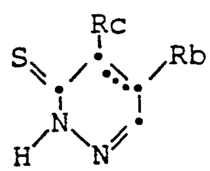

XXI

worin Rb, Rc und die gestrichelte Linie obige Bedeutung besitzen,
mit einer Verbindung der allgemeinen Formel

$$Ra-CHX^2-COX^1 \quad XXII$$

oder

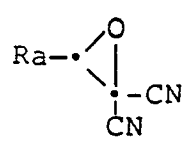

XXIII

worin $X^1$ und $X^2$ je Halogen bedeuten und $R^1$ obige Bedeutung besitzt,
umsetzt. Die Reaktion einer Verbindung der Formel XXI mit einer Verbindung der Formel XXII, worin $X^1$ vorzugsweise Chlor und $X^2$ vorzugsweise Brom bedeuten, wird vorzugsweise bei Raumtemperatur in einem halogenierten Kohlenwasserstoff, wie Chloroform, durchgeführt, worauf man mit einem basischen Amin, wie Triäthylamin, behandelt. Die Reaktion einer Verbindung der Formel XXI mit einer Verbindung der Formel XXIII wird vorzugsweise in einem inerten Lösungsmittel, wie Aceton, N,N-Dimethylformamid, Dimethylsulfoxid und dergleichen, bei Raumtemperatur durchgeführt.

Die Carbonsäuren der Formel VIIIa können durch Hydrolyse der entsprechenden niederen Alkylester der Formel I hergestellt werden. Diese Hydrolyse kann nach an sich bekannten Methoden, beispielsweise in Analogie zu Verfahrensvariante c), durchgeführt werden.

Die Isocyanate der Formel VIIIb können hergestellt werden, indem man eine Verbindung der Formel Ib, worin $Q^1$ die Gruppe der Formel —NH— bedeutet, in einem inerten Lösungsmittel mit Phosgen behandelt. Geeignete Lösungsmittel sind beispielsweise halogenierte Kohlenwasserstoffe, wie Chloroform und 1,2-Dichloräthan. Die Isocyanate der Formel VIIIb können aber auch hergestellt werden, indem man ein Carbonsäurehalogenid der Formel VIIIc in einem inerten organischen Lösungsmittel mit einem Azid, wie

Natriumazid oder Trimethylsilylazid, in das entsprechende Carbonsäureazid überführt und dieses durch Erwärmen zum entsprechenden Isocyanat umlagert. Geeignete Lösungsmittel sind beispielsweise Aether, wie Dioxan, Aethylenglykoldimethyläther, Diäthylenglykoldimethyläther und dergleichen, Ketone, wie Aethylmethylketon, und dergleichen. Die Umlagerung wird bei Temperaturen von 80°C und darüber bewerkstelligt.

Die Carbonsäurehalogenide der Formel VIIIc können hergestellt werden, indem man eine Carbonsäure der Formel Ia mit einem Halogenierungsmittel behandelt. Geeignete Halogenierungsmittel sind beispielsweise Thionylchlorid, Oxalylchlorid, Phosphorpentachlorid und dergleichen. In einer bevorzugten Ausführungsform verwendet man überschüssiges Thionylchlorid und arbeitet ohne zusätzliches Lösungsmittel bei Raumtemperatur.

Die Diazoketone der Formel VIIId können hergestellt werden, indem man ein Carbonsäurehalogenid der Formel VIIIc in einem inerten organischen Lösungsmittel mit Diazomethan umsetzt. Geeignete Lösungsmittel sind beispielsweise Aether, wie Tetrahydrofuran, Dioxan und Diäthyläther. Die Reaktion wird vorzugsweise in einem Temperaturbereich von etwa 0° bis etwa 10°C durchgeführt.

Die Verbindungen der Formel VIIIe können in Analogie zur Verfahrensvariante a) hergestellt werden, wobei man als Ausgangsstoff eine Verbindung der allgemeinen Formel $HC{\equiv}C{-}CH(OR^7)OR^8$ verwendet, worin $R^7$ und $R^8$ obige Bedeutung besitzen.

Die Verbindungen der Formel VIIIf können hergestellt werden, indem man eine Verbindung der Formel Ib' in einem inerten Lösungsmittel, beispielsweise in einem Aether, wie Dioxan, und in Gegenwart einer Base, beispielsweise eines basischen Amins, wie Pyridin, mit Chlorameisensäurephenylester umsetzt.

Die übrigen als Ausgangsstoffe verwendeten Verbindungen gehören an sich bekannten Stoffklassen an. Wie bereits eingangs erwähnt besitzen die Verbindungen der Formel I wertvolle pharmakologische Eigenschaften. Sie entfalten insbesondere ausgeprägte muskelrelaxierende, sedativ-hypnotische, antikonvulsive und/oder anxiolytische Eigenschaften und weisen eine nur geringe Toxizität auf. Diese Eigenschaften können beispielsweise im nachfolgend beschriebenen und für die Erfassung derartiger Eigenschaften allgemein anerkannten Antipentetrazoltest nachgewiesen werden.

In diesem Tierversuch verabreicht man Mäusen die zu testende Verbindung oral und 30 Minuten später 120 mg/kg Pentetrazol intraperitoneal, das in ungeschützten Versuchstieren 1—4 Minuten nach der Injektion Emprosthotonus und tonische Streckungen der vorderen und/oder hinteren Goer vorderen und/oder hinteren Gliedmassen bewirkt. Man verwendet 10 Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere vor den durch Pentetrazol bewirkten krampfartigen Anfällen schützt. In der nachfolgenden Tabelle werden die Resultate zusammengestellt, welche mit repräsentativen Vertretern der durch die allgemeine Formel I definierten Verbindungsklasse in dem vorstehend geschilderten Versuch erhalten worden sind. Die Tabelle enthält ausserdem Angaben über die akute Toxizität ($DL_{50}$) einiger dieser Verbindungen in mg/kg bei einmaliger oraler Verabreichung an Mäusen.

## Tabelle

| Verbindung | $ED_{50}$ in mg/kg p.o. | $DL_{50}$ in mg/kg p.o. |
|---|---|---|
| A | 1,1 | 1500 |
| F | 0,79 | 5000 |
| G | 0,08 | 5000 |
| H | 0,66 | >4000 |
| M | 0,83 | 5000 |

A = 3-Methoxy-1-[(4-oxo-3-phenyl-4H-pyrido[2,1-a]phthalazin-1-yl)carbonyl]azetidin

A = 3-Methoxy-1-[(4-oxo-3-phenyl-4H-pyrido[2,1-a]phthalazin-1-yl)carbonyl]azetidin
F = 1-[(10-Chlor-4-oxo-3-phenyl-4H-pyrido[2,1-a]phthalazin-1-yl)carbonyl]-3-methoxyazetidin
G = N,N-Dimethyl-10-chlor-4-oxo-3-phenyl-4H-pyrido[2,1-a]phthalazin-1-carboxamid
H = N,N-Diäthyl-10-chlor-4-oxo-3-phenyl-4H-pyrido[2,1-a]phthalazin-1-carboxamid
M = 3-Methoxy-1-[(7-oxo-8-phenyl-7H-pyrido[1,2-b]thieno[2,3-d]pyridazin-10-yl)carbonyl]azetidin.

Die Verbindungen der Formel I und die pharmazeutisch annehmbaren Säureadditionssalze von Verbindungen der Formel I mit einem basischen Substituenten können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Also solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten. Arzneimittel enthaltend ein erfindungsgemässes Produkt und einen therapeutisch inerten Träger, sowie ein Verfahren zu deren Herstellung, das dadurch gekennzeichnet ist, dass man ein erfindungsgemässes Produkt und gegebenenfalls einen oder mehrere andere therapeutisch wertvolle Stoffe in eine galenische Darreichungsform bringt, sind ebenfalls Gegenstand der vorliegenden Erfindung.

Die erfindungsgemässen Produkte können, wie bereits erwähnt, bei der Bekämpfung oder Verhütung von Krankheiten, insbesondere bei der Bekämpfung von Konvulsionen und Angstzuständen, sowie für die Herstellung von Arzneimitteln mit muskelrelaxierenden, sedativ-hypnotischen, antikonvulsiven und/oder anxiolytischen Eigenschaften verwendet werden. Die Dosierung kann dabei innerhalb weiter Grenzen variieren und ist natürlich in jedem einzelnen Fall den individuellen Gegebenheiten anzupassen. Bei oraler Verabreichung liegt die tägliche Dosis in einem Bereich von etwa 1 mg bis etwa 100 mg.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

I) 66.95 g Thieno[2,3-d]pyridazin-7(6H)-thion werden unter Argon in 3200 ml Methylenchlorid suspendiert, worauf man 102,7 g α-Bromphenylessigsäurechlorid dazutropft. Man rührt ca. 30 Minuten bei Raumtemperatur und tropft dann 121 ml Triäthylamin dazu, wobei auf 25—30° gekühlt wird. Es wird etwa 40 Minuten gerührt. Nach Abdampfen des Lösungsmittels im Vakuum wird der Rückstand in 1000 ml Wasser und 200 ml Aether aufgenommen, worauf man ca. 30 Minuten rührt. Die ausgefallenen Kristalle werden abgesaugt, mit Wasser und Aether gewaschen und über Nacht getrocknet. Die getrockneten Kristalle werden erneut mit 1000 ml Wasser verrührt. Nach dem Absaugen werden die rotvioletten Kristalle mit Wasser gewaschen und im Vakuum getrocknet. Man erhält 3-Hydroxy-2-phenylthiazolo[3,2-b]thieno[2,3-d]pyridazin-4-ium-hydroxid (inneres Salz) vom Smp. 260—264° (Zersetzung).

In analoger Weise erhält man:

II) Aus 7-Chlor-1(2H)-phthalazin-thion und α-Bromphenylessigsäurechlorid nach Umkristallisieren das 9-Chlor-3-hydroxy-2-phenylthiazolo[2,3-a]phthalazin-4-ium-hydroxid (inneres Salz) vom Smp. 296—298° (Chloroform).

a) 13,5 g 3-Hydroxy-2-phenylthiazolo[2,3-a]phthalazin-4-ium-hydroxid (inneres Salz) und 8,1 ml Propiolsäuremethylester werden in 200 ml Toluol unter Feuchtigkeitsausschluss während 24 Stunden unter Rückfluss erhitzt. Man lässt dann abkühlen und rührt während 1 Stunde im Eisbad. Die Kristalle werden abgesaugt, getrocknet und schliesslich aus Toluol umkristallisiert. Man erhält den 4-Oxo-3-phenyl-4H-pyrido[2,1-a]phthalazin-1-carbonsäuremethylester als gelbe Kristalle vom Smp. 174—175°.

In analoger Weise erhält man:

b) Aus 3-Hydroxy-2-phenylthiazolo[3,2-b]thieno[2,3-d]pyridazin-4-ium-hydroxid (inneres Salz) und Propiolsäuremethylester nach Umkristallisieren aus Acetonitril den 7-Oxo-8-phenyl-7H-pyrido[1,2-b]-thieno[2,3-d]pyridazin-10-carbonsäuremethylester als gelbe Kristalle vom Smp. 198—199° und

c) aus 9-Chlor-3-hydroxy-2-phenylthiazolo[2,3-a]phthalazin-4-ium-hydroxid (inneres Salz) und Propiolsäuremethylester nach Umkristallisieren aus Acetonitril den 10-Chlor-4-oxo-3-phenyl-4H-pyrido[2,1-a]-phthalazin-1-carbonsäuremethylester als gelbe Kristalle vom Smp. 234—236°.

Beispiel 2

a) 11 g 4-Oxo-3-phenyl-4H-pyrido[2,1-a]phthalazin-1-carbonsäuremethylester werden in 300 ml Aethanol aufgenommen, worauf man eine Lösung von 3,7 g Kaliumhydroxid in 30 ml Wasser hinzufügt und unter Rückfluss erhitzt, bis die Reaktion beendet ist. Dann wird das Reaktionsgemisch auf Zimmertemperatur abgekühlt und auf 2200 ml Wasser gegossen. Man stellt durch Zugabe von 1N wässeriger Salzsäure auf pH 7 und entfernt Verunreinigungen durch zweimalige Extraktion mit je 300 ml Methylenchlorid. Die Wasserphase wird mit 2N wässeriger Salzsäure auf pH 1 angesäuert, und die gebildeten Kristalle werden abgesaugt. Nach mehrmaligem Waschen mit Wasser und Trocknen im Vakuum erhält man 4-Oxo-3-phenyl-4H-pyrido[2,1-a]phthalazin-1-carbonsäure als gelbe Kristalle vom Smp. 236—237° (Zers.).

In analoger Weise erhält man:

b) Aus 7-Oxo-8-phenyl-7H-pyrido[1,2-b]thieno[2,3-d]phthalazin-10-carbonsäuremethylester nach Umkristallisieren aus Dimethylformamid die 7-Oxo-8-phenyl-7H-pyrido[1,2-b]thieno[2,3-d]pyridazin-10-carbonsäure als gelbe Kristalle vom Smp. 262—264° (Zersetzung) und

c) aus 10-Chlor-4-oxo-3-phenyl-4H-pyrido[2,1-a]phthalazin-1-carbonsäuremethylester nach Umkristallisieren aus Acetonitril/Dimethylformamid die 10-Chlor-4-oxo-3-phenyl-4H-pyrido[2,1-a]phthalazin-1-carbonsäure als gelbe Kristalle vom Smp. 242° (Zersetzung).

Beispiel 3

a) 3,68 g 4-Oxo-3-phenyl-4H-pyrido[2,1-a]phthalazin-1-carbonsäure werden unter Feuchtigkeitsausschluss in 80 ml Toluol suspendiert, worauf man 5,1 ml Thionylchlorid und 0,2 ml N,N-Dimethylformamid dazugibt und etwa während 2 Stunden bei Zimmertemperatur rührt. Die Reaktionsmischung wird im Vakuum eingedampft; der Rückstand wird in 50 ml Toluol aufgenommen, worauf man erneut im Vakuum eindampft. Das so erhaltene reine Carbonsäurechlorid wird in 90 ml Dioxan aufgenommen, worauf man nacheinander 6,52 ml Triäthylamin und 1,4 g 3-Hydroxyazetidin-hydrochlorid dazugibt. Es wird bis zur Beendigung der Reaktion bei Zimmertemperatur gerührt. Nach Entfernen des Lösungsmittels im Vakuum wird der Rückstand mit einem Gemisch aus 200 ml Wasser und 100 ml gesättigter wässeriger Kochsalzlösung versetzt, worauf man auf ca. 2° abkühlt und während 30 Minuten verrührt. Die Kristalle werden abgesaugt und zweimal mit 15 ml Wasser gewaschen. Die so erhaltenen Kristalle werden im Vakuum bei 70° getrocknet. Die Wasserphase wird dreimal mit Methylenchlorid extrahiert; die vereinigten organischen Phasen werden einmal mit 50 ml Wasser gewaschen, über Natriumsulfat getrocknet, filtriert und eingedampft. Die Kristalle werden mit dem oben erhaltenen Material vereinigt und in 150 ml Aether während 30 Minuten verrührt. Der Aether wird durch Filtration entfernt, und die gelben Kristalle werden getrocknet. Man erhält 3-Hydroxy-1-[(4-oxo-3-phenyl-4H-pyrido[2,1-a]phthalazin-1-yl)carbonyl]azetidin vom Smp. 260—264° (Zers.).

In analoger Weise erhält man aus 4-Oxo-3-phenyl-4H-pyrido[2,1-a]phthalazin-1-carbonsäure und:

b) Diäthylamin das N,N-Diäthyl-4-oxo-3-phenyl-4H-pyrido[2,1-a]phthalazin-1-carboxamid vom Smp. 167° (Essigester);

c) Morpholin das 4-[(4-Oxo-3-phenyl-4H-pyrido[2,1-a]phthalazin-1-yl)carbonyl]morpholin vom Smp. 246—248° (Acetonitril).

Beispiel 4

In Analogie zu Beispiel 3a) erhält man aus 7-Oxo-8-phenyl-7H-pyrido[1,2-b]thieno[2,3-d]pyridazin-10-carbonsäure und:

a) Diäthylamin das N,N-Diäthyl-7-oxo-8-phenyl-7H-pyrido[1,2-b]thieno[2,3-d]pyridazin-10-carboxamid vom Smp. 207—209° (Acetonitril);

b) 3-Methoxyazetidin das 3-Methoxy-1-[(7-oxo-8-phenyl-7H-pyrido[1,2-b]thieno[2,3-d]pyridazin-10-yl)-carbonyl]azetidin vom Smp. 209—210° (Aethanol);

c) cis-2,6-Dimethylmorpholin das cis-2,6-Dimethyl-4-[(7-oxo-8-phenyl-7H-pyrido[1,2-b]thieno[2,3-d]-pyridazin-10-yl)carbonyl]morpholin vom Smp. 250—252° (Aethanol);

d) (R)-Prolinol das (R)-1-[(7-Oxo-8-phenyl-7H-pyrido[1,2-b]thieno[2,3-d]pyridazin-10-yl)carbonyl]-2-pyrrolidinmethanol vom Smp. 252—254° (Acetonitril);

e) (S)-Prolinol das (S)-1-[(7-Oxo-8-phenyl-7H-pyrido[1,2-b]thieno[2,3-d]pyridazin-10-yl)carbonyl]-2-pyrrolidinmethanol vom Smp. 250—253° (Acetonitril);

f) (R)-2-(Methoxymethyl)pyrrolidin das (R)-2-(Methoxymethyl)-1-[(7-oxo-8-phenyl-7H-pyrido[1,2-b]-thieno[2,3-d]pyridazin-10-yl)carbonyl]pyrrolidin vom Smp. 195—201° (Aethanol);

g) (S)-2-(Methoxymethyl)pyrrolidin das (S)-2-(Methoxymethyl)-1-[(7-oxo-8-phenyl-7H-pyrido[1,2-b]-thieno[2,3-d]pyridazin-10-yl)carbonyl]pyrrolidin vom Smp. 194—197° (Aethanol);

h) rac-3-Methoxy-pyrrolidin das rac-3-Methoxy-1-[(7-oxo-8-phenyl-7H-pyrido[1,2-b]thieno[2,3-d]pyridazin-10-yl)carbonyl]pyrrolidin vom Smp. 231—232° (Aethanol);

i) 2-Methoxyäthylamin das N-(2-Methoxyäthyl)-7-oxo-8-phenyl-7H-pyrido[1,2-b]thieno[2,3-d]pyridazin-10-carboxamid vom Smp. 206—208° (Aethanol);

j) Morpholin das 4-[(7-Oxo-8-phenyl-7H-pyrido[1,2-b]thieno[2,3-d]pyridazin-10-yl)carbonyl]morpholin vom Smp. 246—247° (Aethanol);

k) N-Aethyl-N-(2-methoxyäthyl)amin das N-Aethyl-N-(2-methoxyäthyl)-7-oxo-8-phenyl-7H-pyrido[1,2-b]thieno[2,3-d]pyridazin-10-carboxamid vom Smp. 134—136° (Aethanol);

l) 3-Methoxypropylamin das N-(3-Methoxypropyl)-7-oxo-8-phenyl-7H-pyrido[1,2-b]thieno[2,3-d]pyridazin-10-carboxamid vom Smp. 226—228° (Aethanol);

m) Methylamin das N-Methyl-7-oxo-8-phenyl-7H-pyrido[1,2-b]thieno[2,3-d]pyridazin-10-carboxamid vom Smp. 304—308° (Zers.: Dimethylformamid);

n) N,N-Dimethylamin das N,N-Dimethyl-7-oxo-8-phenyl-7H-pyrido[1,2-b]thieno[2,3-d]pyridazin-10-carboxamid vom Smp. 215—217° (Aethanol).

Beispiel 5

In Analogie zu Beispiel 3a) erhält man aus 10-Chlor-4-oxo-3-phenyl-4H-pyrido[2,1-a]phthalazin-1-carbonsäure und:

a) Diäthylamin das N,N-Diäthyl-10-chlor-4-oxo-3-phenyl-4H-pyrido[2,1-a]phthalazin-1-carboxamid vom Smp. 212—215° (Acetonitril);

b) Morpholin das 4-[10-Chlor-4-oxo-3-phenyl-4H-pyrido[2,1-a]phthalazin-1-yl)carbonyl]morpholin vom Smp. 254—257° (Dimethylformamid);

c) rac--3-Methoxypyrrolidin das rac-3-Methoxy-1-[(10-chlor-4-oxo-3-phenyl-4H-pyrido[2,1-a]phthalazin-1-yl)carbonyl]pyrrolidin vom Smp. 203—205° (Acetonitril);

d) (R)-Prolinol das (R)-1-[(10-chlor-4-oxo-3-phenyl-4H-pyrido[2,1-a]phthalazin-1-yl)carbonyl]-2-pyrrolidinmethanol vom Smp. 261—264° (Acetonitril);

e) (R)-2-(Methoxymethyl)pyrrolidin das (R)-2-(Methoxymethyl)-1-[(10-chlor-4-oxo-3-phenyl-4H-pyrido-[2,1-a]phthalazin-1-yl)carbonyl]pyrrolidin vom Smp. 160—163° (Aether/Aceton/n-Hexan);

f) (S)-(2-Methoxymethyl)pyrrolidin das (S)-2-(Methoxymethyl)-1-(10-chlor-4-oxo-3-phenyl-4H-pyrido-[2,1-a]phthalazin-1-yl)carbonyl]pyrrolidin vom Smp. 107—110° (Aether/Aceton);

g) N-Aethyl-N-(2-methoxyäthyl)amin das N-Aethyl-N-(2-methoxyäthyl)-10-chlor-4-oxo-3-phenyl-4H-pyrido[2,1-a]phthalazin-1-carboxamid vom Smp. 167° (Essigester);

h) 3-Methoxyazetidin das 1-[(10-Chlor-4-oxo-3-phenyl-4H-pyrido[2,1-a]phthalazin-1-yl)carbonyl]-3-methoxyazetidin vom Smp. 238—240° (Acetonitril);

i) Dimethylamin das N,N-Dimethyl-10-chlor-4-oxo-3-phenyl-4H-pyrido[2,1-a]phthalazin-1-carboxamid vom Smp. 244—246° (Aethanol).

Beispiel 6

Das aus 2,58 g 7-Oxo-8-phenyl-7H-pyrido[1,2-b]thieno[2,3-d]pyridazin-10-carbonsäure gemäss Beispiel 3a) hergestellte Säurechlorid wird mit 0,8 g Cyclopropancarboxamidoxim in 50 ml Essigsäure bis zum vollständigen Umsatz unter Rückfluss erhitzt. Das Lösungsmittel wird im Vakuum entfernt, und der Rückstand wird an Kieselgel chromatographiert. Nach Umkristallisieren aus Essigsäureäthylester erhält man 10-(3-Cyclopropyl-1,2,4-oxadiazol-5-yl)-7-oxo-8-phenyl-7H-pyrido[1,2-b]thieno[2,3-d]pyridazin als gelbe Kristalle vom Smp. 201—202° und 7-Oxo-8-phenyl-7H-pyrido[1,2-b]thieno[2,3-d]pyridazin als gelbe Kristalle vom Smp. 242—244° (Acetonitril).

Beispiel 7

3,15 g 3-Hydroxy-1-[(4-oxo-3-phenyl-4H-pyrido[2,1-a]phthalazin-1-yl)carbonyl]azetidin werden unter Argon in 30 ml N,N-Dimethylformamid suspendiert, worauf man im Eisbad auf 1—3° abkühlt und 0,81 ml Methyljodid und 0,81 g pulverisiertes Kaliumhydroxid (Gehalt etwa 89%) dazugibt. Man rührt während 20 Minuten im Eisbad, gibt nochmals die gleichen Mengen an Methyljodid und Kaliumhydroxid dazu und rührt noch während 20 Minuten. Die Reaktionsmischung wird auf 150 ml Eiswasser gegossen und mit 2N wässeriger Salzsäure auf pH 5 gestellt. Nach mehrmaligem Ausschütteln mit Methylenchlorid trocknet man die organische Phase über Natriumsulfat, filtriert sie und dampft ein. Der Rückstand wird an Kieselgel mit Methylenchlorid/Methanol (9:1) chromatographiert. Das so erhaltene Rohprodukt wird aus Aethanol umkristallisiert. Man erhält 3-Methoxy-1-[(4-oxo-3-phenyl)-4H-pyrido[2,1-a]phthalazin-1-yl)carbonyl]azetidin als gelbe Kristalle vom Smp. 136—138°.

Beispiel A

Die Verbindung A (3-Methoxy-1-[(4-oxo-3-phenyl-4H-pyrido[2,1-a]phthalazin-1-yl)carbonyl]azetidin) kann in an sich bekannter Weise als Wirkstoff zur Herstellung von pharmazeutischen Präparaten nachfolgender Zusammensetzung verwendet werden:

a) *Tabletten*

| | mg/Tablette |
|---|---|
| Verbindung A | 5 |
| Milchzucker | 135 |
| Maisstärke | 51 |
| Polyvinylpyrrolidon | 8 |
| Magnesiumstearat | 1 |
| Tablettengewicht | 200 |

b) *Kapseln*

| | mg/Kapsel |
|---|---|
| Verbindung A | 10 |
| Milchzucker | 30 |
| Maisstärke | 8,5 |
| Talk | 1 |
| Magnesiumstearat | 0,5 |
| Kapselfüllgewicht | 50 |

Anstelle der Verbindung A kann auch eine der nachfolgend aufgeführten Verbindungen als Wirkstoff zur Herstellung von pharmazeutischen Präparaten obiger Zusammensetzung verwendet werden:

Verbindung B: N,N-Diäthyl-4-oxo-3-phenyl-4H-pyrido[2,1-a]phthalazin-1-carboxamid,

Verbindung C: 4-[(4-Oxo-3-phenyl-4H-pyrido[2,1-a]phthalazin-1-yl)carbonyl]morpholin,

Verbindung D: (S)-2-(Methoxymethyl)-1-[(10-chlor-4-oxo-3-phenyl-4H-pyrido[2,1-a]phthalazin-1-yl)carbonyl]pyrrolidin,

Verbindung E: N-Aethyl-N-(2-methoxyäthyl)-10-chlor-4-oxo-3-phenyl-4H-pyrido[2,1-a]phthalazin-1-carboxamid,

Verbindung F: 1-[(10-Chlor-4-oxo-3-phenyl-4H-pyrido[2,1-a]phthalazin-1-yl)carbonyl]-3-methoxyazetidin,

Verbindung G: N,N-Dimethyl-10-chlor-4-oxo-3-phenyl-4H-pyrido[2,1-a]phthalazin-1-carboxamid,

Verbindung H: N,N-Diäthyl-10-chlor-4-oxo-3-phenyl-4H-pyrido[2,1-a]phthalazin-1-carboxamid,

Verbindung I: 4-[(10-Chlor-4-oxo-3-phenyl-4H-pyrido[2,1-a]phthalazin-1-yl)carbonyl]morpholin,

Verbindung J: rac-3-Methoxy-1-[(10-chlor-4-oxo-3-phenyl-4H-pyrido[2,1-a]phthalazin-1-yl)carbonyl]-pyrrolidin,

Verbindung K: (R)-2-(Methoxymethyl)-1-[(10-chlor-4-oxo-3-phenyl-4H-pyrido[2,1-a]phthalazin-1-yl)carbonyl]pyrrolidin,

Verbindung L: N,N-Diäthyl-7-oxo-8-phenyl-7H-pyrido[1,2-b]thieno[2,3-d]pyridazin-10-carboxamid,

Verbindung M: 3-Methoxy-1-[(7-oxo-8-phenyl-7H-pyrido[1,2-b]thieno[2,3-d]pyridazin-10-yl)carbonyl]-azetidin,

Verbindung N: cis-2,6-Dimethyl-4-[(7-oxo-8-phenyl-7H-pyrido[1,2-b]thieno[2,3-d]pyridazin-10-yl)carbonyl]morpholin,

Verbindung O: (R)-2-(Methoxymethyl)-1-[(7-oxo-8-phenyl-7H-pyrido[1,2-b]thieno[2,3-d]pyridazin-10-yl)carbonyl]pyrrolidin,

Verbindung P: (S)-2-(Methoxymethyl)-1-[(7-oxo-8-phenyl-7H-pyrido[1,2-b]thieno[2,3-d]pyridazin-10-yl)carbonyl]pyrrolidin,

Verbindung Q: rac-3-Methoxy-1-[(7-oxo-8-phenyl-7H-pyrido[1,2-b]thieno[2,3-d]pyridazin-10-yl)carbonyl]pyrrolidin,

Verbindung R: N-(2-Methoxyäthyl)-7-oxo-8-phenyl-7H-pyrido[1,2-b]thieno[2,3-d]pyridazin-10-carboxamid,

Verbindung S: 4-[(7-Oxo-8-phenyl-7H-pyrido[1,2-b]thieno[2,3-d]pyridazin-10-yl)carbonyl]morpholin,

Verbindung T: N-Aethyl-N-(2-methoxyäthyl)-7-oxo-8-phenyl-7H-pyrido[1,2-b]thieno[2,3-d]pyridazin-10-carboxamid,

Verbindung U: N-(3-Methoxypropyl)-7-oxo-8-phenyl-7H-pyrido[1,2-b]thieno[2,3-d]pyridazin-10-carboxamid,

Verbindung V: N,N-Dimethyl-7-oxo-8-phenyl-7H-pyrido[1,2-b]thieno[2,3-d]pyridazin-10-carboxamid,

Verbindung W: N-Methyl-7-oxo-8-phenyl-7H-pyrido[1,2-b]thieno[2,3-d]pyridazin-10-carboxamid und

Verbindung X: 4-Oxo-3-phenyl-4H-pyrido[2,1-a]phthalazin-1-carbonsäuremethylester.

**Patentansprüche für die Vertragsstaaten: BE CH DE FR GB IT LI LU NL SE**

1. Verbindungen der allgemeinen Formel

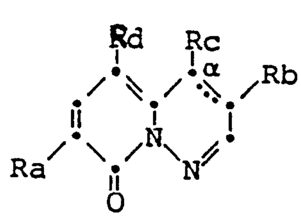

I

worin Ra eine gegebenenfalsl durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_\alpha—S—CH=CH—$ (a), $>C_\alpha—CH=CH—S—$ (b) oder $>C_\alpha—CH=CH—CH=CH—$ (c), die gestrichelte Linie eine zusätzliche Bindung, Rd die Gruppe der Formel $—(A^1)_m—(CO)_n—(Q^1A^2)_q—R^1$, m, n und q je die Zahl 0 oder 1, $A^1$ niederes Alkylen, $A^2$ niederes Alkylen, eine direkte Bidung oder die Gruppe —CO—, $Q^1$ ein Sauerstoffatom oder die Gruppe $—NR^2—$, $R^1$ Wasserstoff, Hydroxy, Cyano, Nitro, Halogen, niederes Alkoxy, niederes Alkyl, niederes Alkoxycarbonyl, Aryl, eine Gruppe der Formel $—NR^3R^4$ oder einen über ein Kohlenstoffatom gebundenen, 5-gliedrigen, gegebenen- falls durch eine oder zwei niedere Alkylgruppen und gegebenenfalls durch eine $(C_{3-6})$-Cycloalkyl, Hydroxy, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegenbenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N—R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Massgabe, dass n die Zahl 0 bedeutet, wenn q die Zahl 1 und $A^2$ die Gruppe —CO— bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die gruppe —CO— bedeuten, dass $R^1$ eine von Hydroxy, Cyano, Nitro, Halogen, niederes Alkoxycarbonyl, niederes Alkoxy und $—NR^3R^4$ verschiedene Bedeutung hat, wenn q die Zahl 1 und $A^2$ eine direkte Bindung bedeuten, dass Rd eine eine von Acetyl verschiedene Bedeutung hat, wenn Ra Phenyl und Rb und Rc zusammen die Gruppe —CH=CH—CH=CH— bedeuten, dass die mit nieder bezeichneten Reste höchstens sieben Kohlenstoffatome besitzen und dass Aryl eine gegebenenfalls durch Halogen, Trifluormethyl, niederes Alkyl, niederes Alkoxy, Nitro, Amino oder mono- oder di(niederes Alkyl)amino substituierte Phenylgruppe bedeutet, und von pharmazeutisch annehmbaren Säureadditionssalzen von Verbindungen der Formel I mit einem oder mehreren basischen Substituenten.

2. Verbindungen nach Anspruch 1, worin Ra Phenyl bedeutet.

3. Verbindungen nach Ansprüche 1 oder 2, worin Rb und Rc zusammen mit dem mit α bezeichneten Kohlenstoffatom eine gegebenenfalls durch Halogen substituierte Gruppe der Formel $>C_\alpha—S—CH=CH—$ oder $>C_\alpha—CH=CH—CH=CH—$ und die gestrichelte Linie eine zusätzliche Bindung bedeuten.

4. Verbindungen nach Anspruch 3, worin Rb und Rc zusamen mit dem mit α bezeichneten Kohlenstoffatom die Gruppe der Formel $>C_\alpha—S—CH=CH—$ oder $>C_\alpha—CH=CCl—CH=CH—$ bedeuten.

5. Verbindungen nach einem der Ansprüche 1—4, worin $Q^1$ ein Sauerstoffatom, $A^2$ die Gruppe —CO—, $R^1$ die Gruppe $—NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten, gesättigten N-Heterocyclus, der als Ringglied noch ein Sauerstoffatom enthalten kann, und entweder m und q die Zahl 0 und n die Zahl 1 oder m und q die Zahl 1 und n die Zahl 0 bedeuten.

6. Verbindungen nach einem der Anspruch 5, worin $A^1$ Methylen und $R^3$ niederes Alkoxyalkyl und $R^4$ Wasserstoff oder niederes Alkyl oder $R^3$ und $R^4$ zusammen mit dem Stickstoffatom eine gegebenfalls durch eine oder zwei niedere Alkylgruppen und gegenbenfalls durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4-Morpholinylgruppe bedeuten.

7. Verbindungen nach Anspruch 6, worin $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl, 2-(niederes Hydroxyalkyl)-1-pyrrolidinyl, 4-Hydroxy-1-piperidinyl, 4-(niederes Alkoxy)-1-piperidinyl, 4-Morpholinyl oder 2,6-Di(niederes Alkyl)-4-morpholinyl bedeuten.

8. Verbindungen nach einem der Ansprüche 1—7, worin m und q die Zahl 0 und n die Zahl 1 bedeuten.

9. Verbindungen nach einem der Ansprüche 1—4, worin m und q die Zahl 0, n die Zahl 1 und $R^1$ Hydroxy oder niederes Alkoxy bedeuten.

10. 3-Methoxy-1-[(4-oxo-3-phenyl-4H-pyrido[2,1-a]phthalazin-1-yl)carbonyl]azetidin.

11. 4-Oxo-3-phenyl-4H-pyrido[2,1-a]phthalazin-1-carbonsäuremethylester.
12. 4-Oxo-3-phenyl-4H-pyrido[2,1-a]phthalazin-1-carbonsäure.
13. 3-Hydroxy-1-[(4-oxo-3-phenyl-4H-pyrido[2,1-a]phthalazin-1-yl)carbonyl]azetidin.
14. Verbindungen der allgemeinen Formel

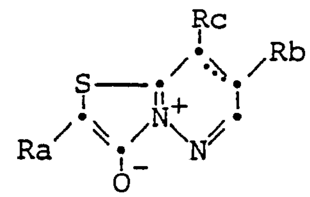

II

worin Ra, Rb, Rc und die gestrichelte Linie die in Anspruch 1 angegebene Bedeutung besitzen, mit der Massgabe, dass Ra eine von Phenyl verschiedene Bedeutung hat, wenn Rb und Rc zusammen die Gruppe —CH=CH—CH=CH— und die gestrichelte Linie eine zützliche Bindung bedeuten.

15. Verbindungen der allgemeinen Formel

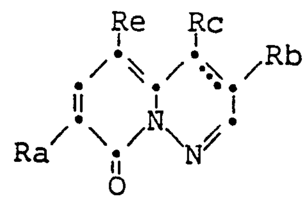

VIII

worin Re die Gruppe $—(A^1)_m—OOC—X^2$, $—(A^1)_m—(CO)_n—(Q^1A^{22})_q—NR^{31}R^{41}$, $—(A^1)_m—N{=}C{=}O$, $—(A^1)_m—CO—X^1$, $—(A')_m—(CO)—CH^-—N_2^+$ oder $—CH(OR^7)OR^8$ bedeutet, und $A^1$, $Q^1$, Ra, Rb, Rc, m, n, q und die gestrichelte Linie die in Anspruch 1 angegebene und A', $A^{22}$, $R^7$, $R^8$, $R^{31}$, $R^{41}$, $X^1$ und $X^2$ die in Anspruch 13 angegebene Bedeutung besitzen.

16. Verbindungen der allgemeinen Formel

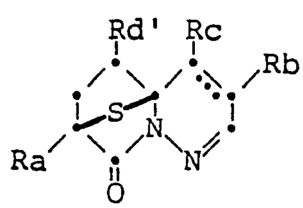

XVIII und

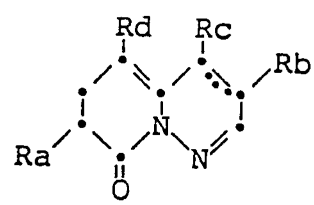

XIX

worin Ra, Rb, Rc, Rd und die gestrichelte Linie die in Anspruch 1 und Rd' die in Anspruch 13 angegebene Bedeutung besitzen, mit der Massgabe, dass Rd' eine von Acetyl und Aethoxycarbonyl verschiedene Bedeutung hat, wenn Ra Phenyl, Rb und Rc zusammen die Gruppe —CH=CH—CH=CH— und die gestrichelte Linie eine zusätzliche Bindung bedeuten.

17. Verbindungen nach einem der Ansprüche 1—13 zur Anwendung als therapeutische Wirkstoffe.

18. Verbindungen nach Anspruch 17 zur Anwendung als muskelrelaxierend, sedativ-hypnotisch, anxiolytisch und/oder antikonvulsiv wirksame Stoffe.

19. Verfahren zur Herstellung von Verbindungen nach einem der Ansprüche 1—13 und von pharmazeutisch annehmbaren Säureadditionssalzen davon, dadurch gekennzeichnet, dass man

a) eine Verbindung der allgemeinen Formel

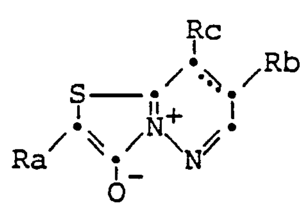

II

worin Ra, Rb, Rc und die gestrichelte Linie die in Anspruch 1 angegebene Bedeutung besitzen, bei erhöhter Temperatur mit einer Verbindung der allgemeinen Formel

$HC{\equiv}C—Rd'$ III oder $H_2C{=}CH—Rd'$ IV

worin Rd', Cyano, Nitro oder die Gruppe der Formel $—CO—(Q^1A^2)_q—R^1$ bedeutet, und q, $A^2$, $Q^1$ und $R^1$ die in Anspruchy 1 angegebene Bedeutung besitzen,

oder mit Phenylvinylsulfoxid umsetzt und das erhaltene Cycloadditionsprodukt gegebenenfalls mit einer starken Base behandelt, oder

b) eine Verbindung der allgemeinen Formel

$$ROOC—C(Ra)=CHR' \qquad V$$

worin R niederes Alkyl und R' Wasserstoff oder niederes Alkoxy bedeuten, und Ra die in Anspruch 1 angegebene Bedeutung besitzt,
wenn R' Wasserstoff bedeutet, bei erhöhter Temperatur oder, wenn R' niederes Alkoxy bedeutet, in Gegenwart einer starken Base mit einer Verbindung der allgemeinen Formel

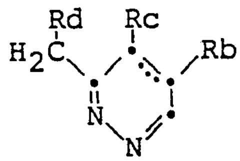

VI

worin Rb, Rc, Rd und die gestrichelte Linie die in Anspruch 1 angegebene Bedeutung besitzen, umsetzt und das erhaltene Cyclokondensationsprodukt, wenn R' Wasserstoff bedeutet, dehydriert, oder

c) eine Verbindung der Formel I, welche eine veresterte Carboxygruppe besitzt, hydrolysiert, oder

d) eine Carbonsäure der allgemeinen Formel

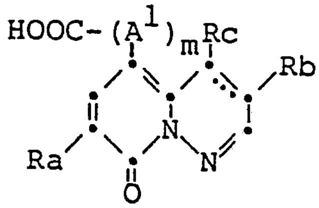

Ia

worin $A^1$, Ra, Rb, Rc, m und die gestrichelte Linie die in Anspruch 1 angebene Bedeutung besitzen, mit einem Alkohol der allgemeinen Formel

$$HO—A^{21}—R^1 \qquad VII$$

worin $A^{21}$ niederes Alkylen oder eine direkte Bindung bedeutet, und $R^1$ die in Anspruch 1 angegebene Bedeutung besitzt,
verestert, oder

e) eie Carbonsäure der obigen Formel Ia oder eine Carbonsäure der allgemeinen Formel

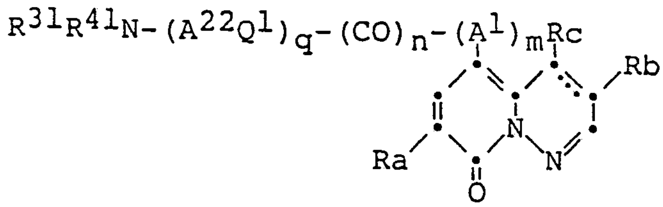

VIIIa

worin $A^{22}$ niederes Alkylen oder die Gruppe —CO— und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- der Schwefelatom oder die Gruppe >N—$R^5$ enthalten kann, und $A^1$, $Q^1$, Ra, Rb, Rc, $R^5$, m, n, q und die gestrichelte Linie die in Anspruch 1 angegebene Bedeutung besitzen,
oder ein reaktives Derivat dvon mit einem Amin der allgemeinen Formel

$$HNR^2—A^{21}—R^1 \text{ IX oder } HHR^3R^4 \qquad X$$

worin $R^1$, $R^2$, $R^3$ und $R^4$ die in Anspruch 1 angebene und $A^{21}$ obige Bedeutung besitzen,
bzw. mit Ammoniak oder einem mono- oder di(niederen Alkyl)amin in das entsprechende Amid überführt, oder

f) eine Verbindung der allgemeinen Formel

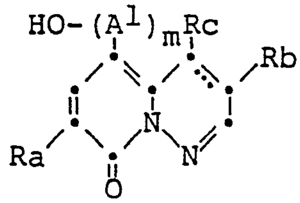

Ib'

worin $A^1$, Ra, Rb, Rc, m und die gestrichelte Linie die in Anspruch 1 angegebene Bedeutung besitzen, in Gegenwart einer Base mit einer Verbindung der allgemeinen Formel

$$X—A^{21}—R^1 \qquad XI$$

worin X eine Abgangsgruppe bedeutet, $A^{21}$ obige und $R^1$ die in Anspruch 1 angegebene Bedeutung besitzen,
bzw. eine Verbindung der Formel I, welche eine freie Hydroxygruppe besitzt, mit einer Verbindung der allgemeinen Formel

$$R—X \qquad XII$$

worin R niederes Alkyl bedeutet, und X obige Bedeutung besitzt,
umsetzt, oder

g) eine Verbindung der allgemeinen Formel

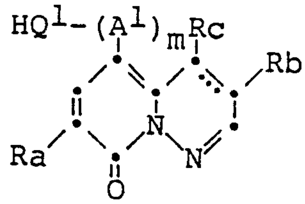

Ib

worin $A^1$, $Q^1$, Ra, Rb, Rc, m und die gestrichelte Linie die in Anspruch 1 angegebene Bedeutung besitzen,
in Gegenwart eines säurebindenden Mittels mit einem reaktiven Derivat einer Carbonsäure der allgemeinen Formel

$$R^1—COOH \qquad XIII$$

worin $R^1$ die in Anspruch 1 angegebene Bedeutung besitzt,
umsetzt, oder

h) eine Verbindung der allgemeinen Formel

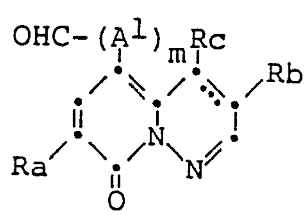

Ic

worin $A^1$, Ra, Rb, Rc, m und die gestrichelte Linie die in Anspruch 1 angegebene Bedeutung besitzen, in Gegenwart eines Reduktionsmittels mit einem Amin der obigen Formel IX oder X umsetzt, oder

i) eine Verbindung der allgemeinen Formel

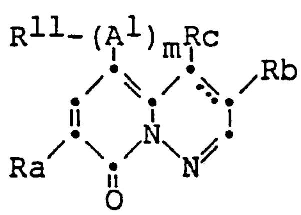

Id

worin $R^{11}$ Nitro, Cyano oder niederes Alkoxycarbonbyl bedeutet, und $A^1$, Ra, Rb, Rc, m und die gestrichelte Linie die in Anspruch 1 angegebene Bedeutung besitzen,

oder eine Verbindung der obigen Formel Ia oder ein reaktives Derivat davon reduziert, oder

j) einen Alkohol der obigen Formel Ib' oder einen Alkohol der allgemeinen Formel

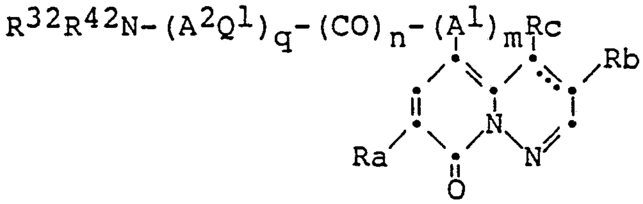

Ie

worin $A^1$, $A^2$, $Q^1$, Ra, Rb, Rc, m, n, q und die gestrichelte Linie die in Anspruch 1 angegebene Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigten N-Heterocyclus bedeuten, der als Ringglied noch ein Sauerstoff- der Schwefelatom oder die Gruppe >N—$R^5$ enthalten kann, und $R^5$ die in Anspruch 1 angegebene Bedeutung besitzt,
oxidiert, oder

k) ein Isocyanat der allgemeinen Formel

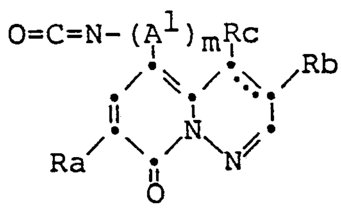

VIIIb oder $O{=}C{-}N{-}R^{33}$ XIV

worin $A^1$, Ra, Rb, Rc, m und die gestrichelte Linie die in Anspruch 1 angegebene Bedeutung besitzen, und $R^{33}$ Wasserstoff, niederes Alkyl oder $(C_{3-7})$-Cycloalkyl bedeutet,
mit einem niederen Alkohol oder einem Amin der obigen Formel X bzw. mit einer Verbindung der obigen Formel Ib umsetzt, oder

l) eine Verbindung der allgemeinen Formel

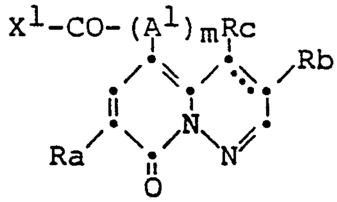

VIIIc

worin $X^1$ en Halogenatom bedeutet, und $A^1$, Ra, Rb, Rc, m und die gestrichelte Linie die in Anspruch 1 angegebene Bedeutung besitzen,
mit einem niederen Alkylmagnesiumhalogenid umsetzt, oder

m) eine Verbindung der allgemeinen Formel

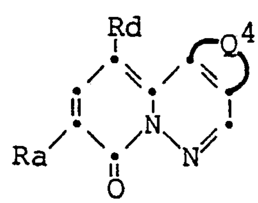

Ih

worin $Q^4$ die in Anspruch 1 definierte Gruppe (a) oder (b) bedeutet, und Ra und Rd die in Anspruch 1 angegebene Bedeutung besitzen,
am Thiophenring halogeniert, oder

n) eie Verbindung der obigen Formel VIIIc in Gegenwart einer Base mit einer Verbindung der allgemeinen Formel

$HYN{=}C(NH_2){—}R''$ XV,

$H_2N{—}CHR''{—}CHR'''{—}Y'H$ oder XVI

$H_2N{—}NH{—}C(R''){=}Y''$ XVII

worin Y ein Sauerstoffatom oder die Gruppe —NR'''—, Y' ein Sauerstoffatom oder die Gruppe —NH—,

Y'' ein Sauerstoff- oder Schwefelatom und R'' und R''' je Wasserstoff oder niederes Alkyl bedeuten, umsetzt und das erhaltene Produkt cyclisiert, oder

o) eine Verbindung der allgemeinen Formel

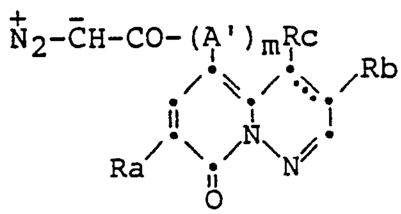

VIIId

worin A' $C_{1-6}$-Alkylen bedeutet, und Ra, Rb, Rc, die gestrichelte Linie und m die in Anspruch 1 angegebene Bedeutung besitzen, mit einem niederen Alkohol umsetzt, oder

p) eine Carbonsäure der Formel Ia, worin m die Zahl 0 bedeutet, decarboxyliert, oder

q) eine Verbindung der Formel I, worin Rd Wasserstoff bedeutet, am Pyridonring halogeniert, oder

r) in einer Verbindung der allgemeinen Formel

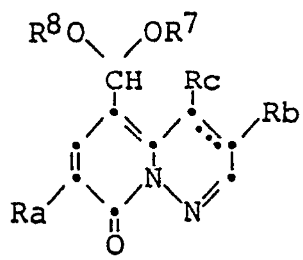

VIIIe

worin $R^7$ und $R^8$ je niederes Alkyl oder zusammen niederes Alkylen bedeuten, und Ra, Rb, Rc und die gestrichlete Linie die in Anspruch 1 angegebene Bedeutung besitzen, die Acetalgruppe spaltet, oder

s) eine Verbindung der allgemeinen Formel

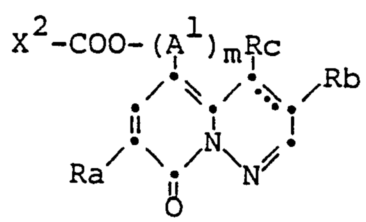

VIIIf

worin $X^2$ Phenoxy bedeutet, und $A^1$, Ra, Rb, Rc, die gestrichelte Linie und m die in Anspruch 1 angegebene Bedeutung besitzen, mit einem Amin der obigen Formel X umsetzt, und

t) erwünschtenfalls eine erhaltene Verbindung der Formel I mit einem basischen Substituenten in ein pharmazeutisch anehmbares Säureadditionssalz überführt.

20. Arzneimittel, enthaltend eine Verbindung nach einem der Ansprüche 1—13 und ein therapeutisch inertes Trägermaterial.

21. Muskelrelaxierend, sedativ-hypnotisch, anxiolytisch und/oder antikonvulsiv wirksame Mittel, enthaltend eine Verbindung nach einem der Ansprüche 1—13 und ein therapeutisch inertes Trägermaterial.

22. Verwendung von Verbindungen nach einem der Ansprüche 1—13 zur Herstellung von muskelrelaxierend, sedativ-hypnotisch, anxiolytisch und/oder antikonvulsiv wirksmen Mitteln.

23. Verwendung von Verbindungen nach einem der Ansprüche 14—16 zur Herstellung von therapeutischen Wirkstoffen.

24. Verwendung von Verbindungen nach einem der Ansprüche 14—16 zur Herstellung von Verbindungen nach einem der Ansprüche 1—13.

**Patentansprüche für den Vertragsstaat: GR**

1. Verfahren zur Herstellung von Verbindungen der allgemeinen Formel

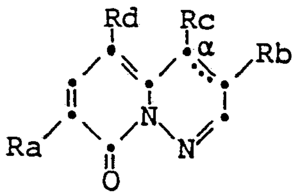

I

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_α—S—CH=CH—$ (a), $>C_α—CH=CH—S—$ (b) oder $>C_α—CH=CH—CH=CH—$ (c), die gestrichelte Linie eine zusätzliche Bindung, Rd die Gruppe der Formel $—(A^1)_m—(CO)_n—(Q^1A^2)_q—R^1$, m, n und q je die Zahl 0 oder 1, $A^1$ niederes Alkylen, $A^2$ niederes Alkylen, eine direkte Bindung oder die Gruppe —CO—, $Q^1$ ein Sauerstoffatom oder die Gruppe $—NR^2—$, $R^1$ Wasserstoff, Hydroxy, Cyano, Nitro, Halogen, niederes Alkoxy, niederes Alkyl, niederes Alkoxycarbonyl, Aryl, eine Gruppe der Formel $—NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegenbenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N—R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Massgabe, dass n die Zahl 0 bedeutet, wenn q die Zahl 1 und $A^2$ die Gruppe —CO— bedeuten, dass $R^1$ eine von Cyano, Nitro, Halogen oder niederes Alkoxycarbonyl verschiedene Bedeutung hat, wenn q die Zahl 0 und n die Zahl 1 oder wenn q die Zahl 1 und $A^2$ die gruppe —CO— bedeuten, dass $R^1$ eine von Hydroxy, Cyano, Nitgro, Halogen, niederes Alkoxycarbonyl, niederes Alkoxy und $—NR^3R^4$ verschiedene Bedeutung hat, wenn q die Zahl 1 und $A^2$ eine direkte Bindung bedeuten, dass Rd ene eine von Acetyl verschiedene Bedeutung hat, wenn Ra Phenyl und Rb und Rc zusammen die Gruppe —CH=CH—CH=CH— bedeuten, dass die mit nieder bezeichneten Reste höchstens sieben Kohlenstoffatome besitzen und dass Aryl eine gegebenenfalls durch Halogen, Trifluormethyl, niederes Alkyl, niederes Alkoxy, Nitro, Amino oder mono- oder di(niederes Alkyl)amino substituierte Phenylgruppe bedeutet, und von pharmazeutisch annehmbare Säureadditionssalze von Verbindungen der Formel I mit einem oder mehreren basischen Substituenten, dadurch gekennzeichnet, dass man

a) eine Verbindung der allgemeinen Formel

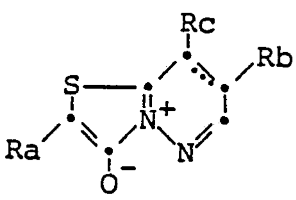

II

worin Ra, Rb, Rc und die gestrichelte Linie obige Bedeutung besitzen,
bei erhöhter Temperatur mit einer Verbindung der allgemeinen Formel

$$HCX{\equiv}C—Rd'\ \text{III oder}\ H_2C=CH—Rd' \qquad \text{IV}$$

worin Rd' Cyano, Nitro oder die Gruppe der Formel —CO—$(Q^1A^2)_q$—$R^1$ bedeutet, und q, $A^2$, $Q^1$ und $R^1$ obige Bedeutung besitzen,
oder mit Phenylvinylsulfoxid umsetzt und das erhaltene Cycloadditionsprodukt gegebenenfalls mit einer starken Base behandelt, oder

b) eine Verbindung der allgemeinen Formel

$$ROOC—C(Ra)=CHR' \qquad V$$

worin R niederes Alkyl und R' Wasserstoff oder niederes Alkoxy bedeuten, und Ra obige Bedeutung besitzt,
wenn R' Wasserstoff bedeutet, bei erhöhter Temperatur oder, wenn R' niederes Alkoxy bedeutet, in Gegenwart einer starken Base mit einer Verbindung der allgemeinen Formel

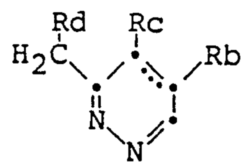

VI

worin Rb, Rc, Rd und die gestrichelte Linie obige Bedeutung besitzen,
umsetzt und das erhaltene Cyclokondensationsprodukt, wenn R' Wasserstoff bedeutet, dehydriert, oder

c) eie Verbindung der Formel I, welche eine veresterte Carboxygruppe besitzt, hydrolyiert, oder

d) eine Carbonsäure der allgemeinen Formel

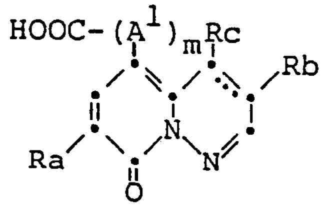

Ia

worin $A^1$, Ra, Rb, Rc, m und die gestrichelte Linie obige Bedeutung besitzen,
mit einem Alkohol der allgemeinen Formel

$$HO—A^{21}—R^1 \qquad VII$$

worin $A^{21}$ niederes Alkylen oder eine direkte Bindung bedeutet, und $R^1$ obige Bedeutung besitzt, verestert, oder

e) eine Carbonsäure der obigen Formel Ia oder eine Carbonsäure der allgemeinen Formel

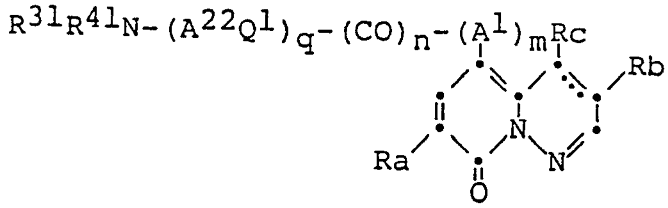

VIIIa

worin $A^{22}$ niederes Alkylen oder die Gruppe —CO— und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- der Schwefelatom oder die Gruppe >N—$R^5$ enthalten kann, und $A^1$, $Q^1$, Ra, Rb, Rc, $R^5$, m, n, q und die gestrichelte Linie die in obige Bedeutung besitzen,
oder ein reaktives Derivat davon mit einem Amin der allgemeinen Formel

$$HNR^2—A^{21}—R^1 \text{ IX oder } HNR^3R^4 \qquad X$$

worin $A^{21}$, $R^1$, $R^2$, $R^3$ und $R^4$ obige Bedeutung besitzen,
bzw. mit Ammoniak oder einem mono- oder di(niederen Alkyl)amin in das entsprechende Amid überführt, oder

f) eine Verbindung der allgemeinen Formel

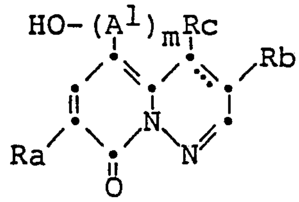

Ib'

worin $A_1$, Ra, Rb, Rc, m und die gestrichelte Linie obige Bedeutung besitzen,
in Gegenwart einer Base mit einer Verbindung der allgemeinen Formel

$$X—A^{21}—R^1 \qquad XI$$

worin X eine Abgangsgruppe bedeutet, $A^{21}$ obige und $R^1$ obige Bedeutung besitzen,
bzw. eine Verbindung der Formel I, welche eine freie Hydroxygruppe besitzt, mit einer Verbindung der allgemeinen Formel

$$R—X \qquad XII$$

worin R niederes Alkyl bedeutet, und X obige Bedeutung besitzt,
umsetzt, oder

g) eine Verbindung der allgemeinen Formel

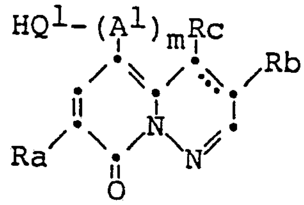

Ib

worin $A^1$, $Q^1$, Ra, Rb, Rc, m und die gestrichelte Linie obige Bedeutung besitzen,
in Gegenwart eines säurebindenden Mittels mit einem reaktiven Derivat einer Carbonsäure der allgemeinen Formel

$$R^1—COOH \qquad XIII$$

worin $R^1$ obige Bedeutung besitzt,
umsetzt, oder

h) eine Verbindung der allgemeinen Formel

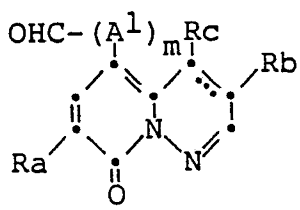

Ic

worin $A^1$, Ra, Rb, Rc, m und die gestrichelte Linie obige Bedeutung Bedeutung besitzen,
in Gegenwart eines Reduktionsmittels mit einem Amin der obigen Formel IX oder X umsetzt, oder

i) eine Verbindung der allgemeinen Formel

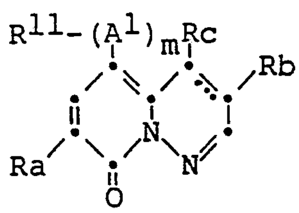

Id

worin $R^{11}$ nitro, Cyano oder niederes Alkoxycarbonyl bedeutet, und $A^1$, Ra, Rb, Rc, m und die gestrichelte Linie die obige angegebene Bedeutung besitzen,
oder eine Verbindung der obigen Formel Ia oder ein reaktives Derivat davon reduziert, oder

j) einen Alkohol der obigen Formel Ib' oder einen Alkohol der allgemeinen Formel

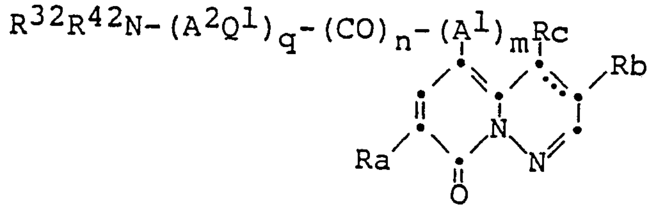

Ie

worin $A^1$, $A^2$, $Q^1$, Ra, Rb, Rc, m, n, q und die gestrichelte Linie obige Bedeutung besitzen, und $R^{32}$ und $R^{42}$ zusammen mit dem Stickstoffatom einen 3- bis 7-gliedrigen, gegebenenfalls durch eine oder zwei niedere Alkylgruppen und durch eine Hydroxygruppe substituierten, gesättigten N-Heterocyclus bedeuten, der als Ringglied noch ein Sauerstoff- der Schwefelatom oder die Gruppe $>N—R^5$ enthalten kann, und $R^5$ obige Bedeutung besitzt,
oxidiert, oder

k) ein Isocyanat der allgemeinen Formel

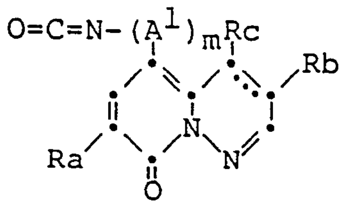

VIIIb oder $O=C-N-R^{33}$ XIV

worin $A^1$, Ra, Rb, Rc, m und die gestrichelte Linie obige Bedeutung besitzen, und $R^{33}$ Wasserstoff, niederes Alkyl oder $(C_{3-7})$-Cycloalkyl bedeutet,
mit einem niederen Alkohol oder einem Amin der obigen Formel X bzw. mit einer Verbindung der obigen Formel Ib umsetzt, oder

l) eine Verbindung der allgemeinen Formel

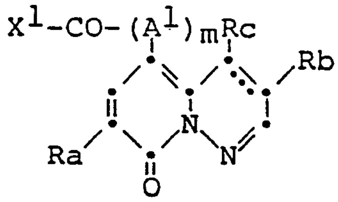

VIIIc

worin $X^1$ en Halogenatom bedeutet, und $A^1$, Ra, Rb, Rc, m und die gestrichelte Linie obige Bedeutung besitzen,
mit einem niederen Alkylmagnesiumhalogenid umsetzt, oder

m) eine Verbindung der allgemeinen Formel

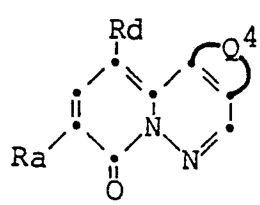

Ih

worin $Q^4$ die obige Gruppe (a) oder (b) bedeutet, und Ra und Rd obige Bedeutung besitzen,
am Thiophenring halogeniert, oder

n) eine Verbindung der obigen Formel VIIIc in Gegenwart einer Base mit einer Verbindung der allgemeinen Formel

$$HYN=C(NH_2)—R'' \quad XV,$$

$$H_2N—CHR''—CHR'''—Y'H \text{ oder} \quad XVI$$

$$H_2N—NH—C(R'')=Y'' \quad XVII$$

worin Y ein Sauerstoffatom oder die Gruppe —NR'''—, Y' ein Sauerstoffatom oder die Gruppe —NH—,

Y'' ein Sauerstoff- oder Schwefelatom und R'' und R''' je Wasserstoff oder niederes Alkyl bedeuten, umsetzt und das erhaltene Produkt cyclisiert, oder

o) eine Verbindung der allgemeinen Formel

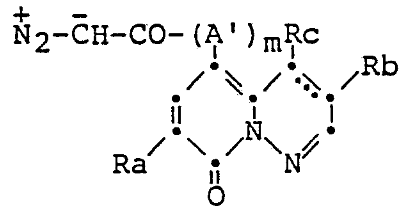

VIIId

worin A' $C_{1-6}$-Alkylen bedeutet, und Ra, Rb, Rc, die gestrichelte Linie und m obige Bedeutung besitzen, mit einem niederen Alkohol umsetzt, oder

p) eine Carbonsäure der Formel Ia, worin m die Zahl 0 bedeutet, decarboxyliert, oder

q) eine Verbindung der Formel I, worin Rd Wasserstoff bedeutet, am Pyridonring halogeniert, oder

r) in einer Verbindung der allgemeinen Formel

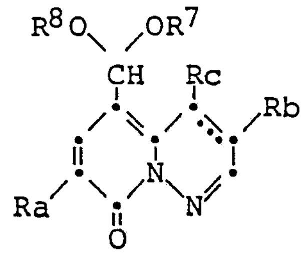

VIIIe

worin $R^7$ und $R^8$ je niederes Alkyl oder zusammen niederes Alkylen bedeuten, und Ra, Rb, Rc und die gestrichlete Linie obige Bedeutung besitzen,
die Acetalgruppe spaltet, oder

s) eine Verbindung der allgemeinen Formel

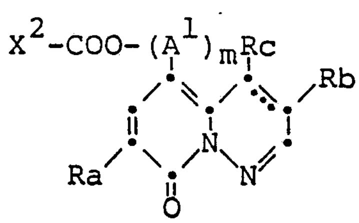

VIIIf

worin $X^2$ Phenoxy bedeutet, und $A^1$, Ra, Rb, Rc, die gestrichelte Linie und m obige Bedeutung besitzen, mit einem Amin der obigen Formel X umsetzt, und

t) erwünschtenfalls eine erhaltene Verbindung der Formel I mit einem basischen Substituenten in ein pharmazeutisch anehmbares Säureadditionssalz überführt.

2. Verfahren nach Anspruch 1 worin Ra Phenyl bedeutet.

3. Verfahren nach Ansprüche 1 oder 2, worin Rb und Rc zusammen mit dem mit α bezeichneten Kohlenstoffatom eine gegebenenfalls durch Halogen substituierte Gruppe der Formel $>C_\alpha 3$—S—CH=CH— oder $>C_\alpha$—CH=CH—CH=CH— und die gestrichelte Linie eine zusätzliche Bindung bedeuten.

4. Verfahren nach Anspruch 3, worin Rb und Rc zusamen mit dem mit α bezeichneten Kohlenstofatom die Gruppe der Formel $>C_\alpha$—S—CH=CH— oder $>C_\alpha$—CH=CCl—CH=CH— bedeuten.

5. Verfahren nach einem der Ansprüche 1—4, worin $Q^1$ ein Sauerstoffatom, $A^2$ die Gruppe —CO—, $R^1$ die Gruppe —$NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niederee Alkoxyalkylgruppe substituierten, gesättigten H-Heterocyclus, der als Ringglied noch ein Sauerstoffatom enthalten kann, und entweder m und q die Zahl 0 und n die Zahl 1 oder m und q die Zahl 1 und n die Zahl 0 bedeuten.

6. Verfahren nach einem der Anspruch 5, worin $A^1$ Methylen und $R^3$ niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4-Morpholinylgruppe bedeuten.

7. Verfahren nach Anspruch 6, worin $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes Alkoxy)-1-pyrrolidinyl, 2-(niederes Alkoxyalkyl)-1-pyrrolidinyl, 2-(niederes Hydroxyalkyl)-1-pyrrolidinyl, 4-Hydroxy-1-piperidinyl, 4-(niederes Alkoxy)-1-piperidinyl, 4-Morpholinyl oder 2,6-Di(niederes Alkyl)-4-morpholinyl bedeuten.

8. Verfahren nach einem der Ansprüche 1—7, worin m und q die Zahl 0 und n die Zahl 1 bedeuten.

9. Verfahren nach einem der Ansprüche 1—4, worin m und q die Zahl 0, n die Zahl 1 und $R^1$ Hydroxy oder niederes Alkoxy bedeuten.

10. Verfahren nach Anspruch 1, dadurch gekennzeichnet, dass man 3-Methoxy-1-[(4-oxo-3-phenyl-4H-pyrido[2,1-a)phthalazin-1-yl)carbonyl]azetidin herstellt.

11. Verfahren zur Herstellung von Arznemitteln, insbesondere von muskelrelaxierend, sedativ-hypnotisch, anxiolytisch und/oder antikonvulsiv wirksamen Mitteln, dadurch gekennzeichnet, dass man eine Verbindung der in Anspruch 1 definierten Formel I oder ein pharmazeutisch annehmbarers Säureadditionssalz einer Verbindung der Formel I mit einem oder mehreren basischen Substituenten und gegebenenfalls einem anderen therapeutisch wirksam Stoff zusammen mit einem therapeutisch inerten Trägrematerial n eine galenische Darreichungsform bringt.

12. Verwendung von Verbindungen der in Anspruch 1 definierten Formel I zur Herstellung von muskelrelaxierend, sedativ-hypnotisch, anxiolytisch und/oder antikonvulsiv wirksamen Mitteln.

13. Verbindungen der allgemeinen Formel

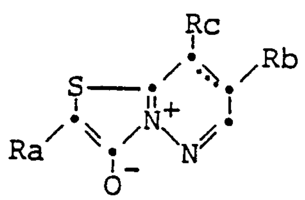

II

worin Ra, Rb, Rc und die gestrichelte Linie die in Anspruch 1 angegebene Bedeutung besitzen, mit der Massgabe, dass Ra eine von Phenyl verschiedene Bedeutung hat, wenn Rb und Rc zusammen die Gruppe —CH=CH—CH=CH— und die gestrichelte Linie eine zusätzliche Bindung bedeuten.

14. Verbindungen der allgemeinen Formel

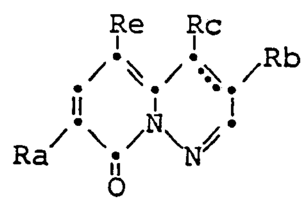

VIII

worin Re die Gruppe $-(A^1)_m-OOC-X^2$, $-(A^1)_m-(CO)_n-(Q^1A^{22})_q-NR^{31}R^{41}$, $-(A^1)_m-N=C=O$, $-(A^1)_m-CO-X^1$, $-(A')_m-(CO)-CH^{-}-N_2^{+}$ oder $-CH(OR^7)OR^8$ bedeutet, und $A^1$, $Q^1$, Ra, Rb, Rc, m, n, q und die gestrichelte Linie sowie A', $A^{22}$, $R^7$, $R^8$, $R^{31}$, $R^{41}$, $X^1$ und $X^2$ die in Anspruch 13 angegebene Bedeutung besitzen.

15. Verbindungen der allgemeinen Formel

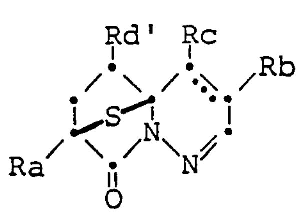

XVIII

und

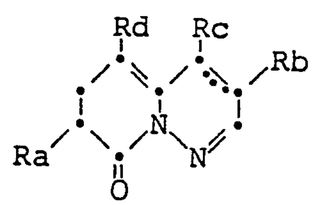

XIX

worin Ra, Rb, Rc, Rd und die gestrichelte Linie sowie Rd' die in Anspruch 13 angegebene Bedeutung besitzen, mit der Massgabe, dass Rd' eine von Acetyl und Aethoxycarbonyl verschiedene Bedeutung hat, wenn Ra Phenyl, Rb und Rc zusammen die Gruppe —CH=CH—CH=CH— und die gestrichelte Linie eine zusätzliche Bindung bedeuten.

**Patentansprüche für die Vertragsstaaten: AT ES**

1. Verfahren zur Herstellung von Verbindungen der allgemeinen Formel

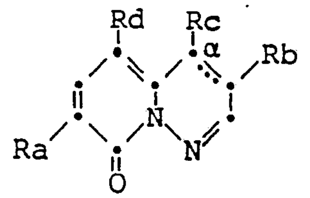

I

worin Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel $>C_α—S—CH=CH—$ (a), $>C_α—CH=CH—S—$ (b) oder $>C_α—CH=CH—CH=CH—$ (c), die gestrichelte Linie eine zusätzliche Bindung, Rd die Gruppe der Formel $—(A^1)_m—(CO)^1—(Q_1A^2)_q—R^1$, m, n und q je die Zahl 0 oder 1, $A^1$ niederes Alkylen, $A^2$ niederes Alkylen, eine direkte Bindung oder die Gruppe —CO—, $Q^1$ ein Sauerstoffatom oder die Gruppe $—NR^2—$, $R^1$ Wasserstoff, Hydroxy, Cyano, Nitro, Halogen, niederes Alkoxy, niederes Alkyl, niederes Alkoxycarbonyl, Aryl, eine Gruppe der Formel $—NR^3R^4$ oder einen über ein Kohlenstoffatom gebundenen, 5-gliedrigen, gegebenenfalls durch eine oder zwei niedere Alkylgruppen und gegebenenfalls durch eine $(C_{3-6})$-Cycloalkyl-, Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, $R^2$ Wasserstoff, niederes Alkyl oder Aryl, $R^3$ und $R^4$ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte $(C_{3-7})$-Cycloalkylgruppe oder $R^3$ und $R^4$ zusammen mit dem Stickstoffatom einen 3- bis 7-gliedrigen, gegebenenfalls durch eine oder zwei niedere Alkylgruppen und gegenbenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe $>N—R^5$ enthalten kann, und $R^5$ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)carbamoyl bedeuten, mit der Massgabe, dass n die Zahl 0 bedeutet, wenn q die Zahl 1 und $A^2$ die Gruppe —CO— bedeuten, dass $R^1$ eine von Cyano, Nitro, Halogen oder niederes Alkoxycarbonyl verschiedene Bedeutung hat, wenn q die Zhl 0 und n die Zahl 1 oder wenn q die Zahl 1 und $A^2$ die gruppe —CO— bedeuten, dass $R^1$ eine von Hydroxy, Cyano, Nitro, Halogen, niederes Alkoxycarbonyl, niederes Alkoxy und $—NR^3R^4$ verschiedene Bedeutung hat, wenn q die Zahl 1 und $A^2$ eine direkte Bindung bedeuten, dass Rd eine von Acetyl verschiedene Bedeutung hat, wenn Ra Phenyl und Rb und Rc zusammen die Gruppe —CH=CH—CH=CH— bedeuten, dass die mit nieder bezeichneten Reste höchstens sieben Kohlenstoffatome besitzen und dass Aryl eine gegebenenfalls durch Halogen, Trifluormethyl, niederes Alkyl, niederes Alkoxy, Nitro, Amino oder mono- oder di(niederes Alkyl) amino substituierte Phenylgruppe bedeutet, und pharmazeutisch annehmbare Säureadditionssalzen von Verbindungen der Formel I mit einem oder mehreren basischen Substituenten, dadurch gekennzeichnet, dass man

a) eine Verbindung der allgemeinen Formel

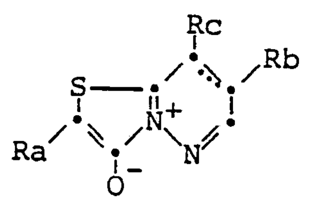

II

worin Ra, Rb, Rc und die gestrichelte Linie obige Bedeutung besitzen, bei erhöhter Temperatur mit einer Verbindung der allgemeinen Formel

$HC{\equiv}C{-}Rd'$ III oder $H_2C{=}CH{-}Rd'$ IV

worin Rd' Cyano, Nitro oder die Gruppe der Formel $-CO-(Q^1A^2)_q-R^1$ bedeutet, und q, $A^2$, $Q^1$ und $R^1$ obige Bedeutung besitzen, oder mit Phenylvinylsulfoxid umsetzt und das erhaltene Cycloadditionsprodukt gegebenenfalls mit einer starken Base behandelt, oder

b) eine Verbindung der allgemeinen Formel

$ROOC{-}C(Ra){=}CHR'$ V

worin R niederes Alkyl und R' Wasserstoff oder niederes Alkoxy bedeuten, und Ra obige Bedeutung besitzt, wenn R' Wasserstoff bedeutet, bei erhöhter Temperatur oder, wenn R' niederes Alkoxy bedeutet, in Gegenwart einer starken Base mit einer Verbindung der allgemeinen Formel

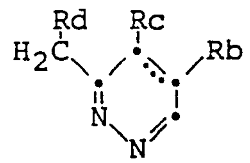

VI

worin Rb, Rc, Rd und die gestrichelte Linie obige Bedeutung besitzen, umsetzt und das erhaltene Cyclokondensationsprodukt, wenn R' Wasserstoff bedeutet, dehydriert, oder

c) eie Verbindung der Formel I, welche eine veresterte Carboxygruppe besitzt, hydrolysiert, oder

d) eine Carbonsäure der allgemeinen Formel

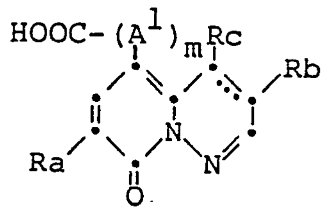

Ia

worin $A^1$, Ra, Rb, Rc, m und die gestrichelte Linie obige Bedeutung besitzen, mit einem Alkohol der allgemeinen Formel

$HO{-}A^{21}{-}R^1$ VII

worin $A^{21}$ niederes Alkylen oder eine direkte Bindung bedeutet, und $R^1$ obige Bedeutung besitzt, verestert, oder

e) eine Carbonsäure der obigen Formel Ia oder eine Carbonsäure der allgemeinen Formel

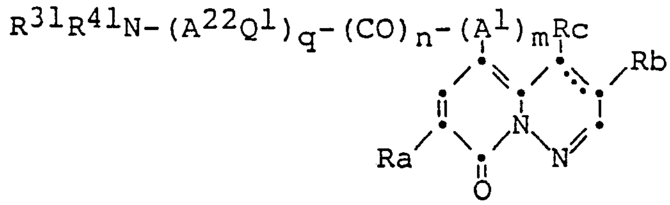

VIIIa

worin $A^{22}$ niederes Alkylen oder die Gruppe —CO— und $R^{31}$ und $R^{41}$ zusammen mit dem Stickstoffatom einen 3- bis 7-gliedrigen, gegebenenfalls durch eine oder zwei niedere Alkylgruppen und durch eine Carboxygruppe substituierten, gesättigten N-Heterocyclus bedeuten, der als Ringglied noch ein Sauerstoff- der Schwefelatom oder die Gruppe $>N{-}R^5$ enthalten kann, und $A^1$, $Q^1$, Ra, Rb, Rc, $R^5$, m, n, q und die gestrichelte Linie obige Bedeutung besitzen, oder ein reaktives Derivat dvon mit einem Amin der allgemeinen Formel

$HNR^2{-}A^{21}{-}R^1$ IX oder $HNR^3R^4$ X

worin $A^{21}$, $R^1$, $R^2$, $R^3$ und $R^4$ obige Bedeutung besitzen, bzw. mit Ammoniak oder einem mono- oder di(niederen Alkyl)amin in das entsprechende Amid überführt, oder

f) eine Verbindung der allgemeinen Formel

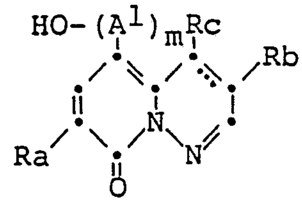

Ib'

worin $A_1$, Ra, Rb, Rc, m und die gestrichelte Linie obige Bedeutung besitzen, in Gegenwart einer Base mit einer Verbindung der allgemeinen Formel

$$X—A^{21}—R^1 \qquad XI$$

worin X eine Abgangsgruppe bedeutet, $A^{21}$ obige und $R^1$ obige Bedeutung besitzen, bzw. eine Verbindung der Formel I, welche eine freie Hydroxygruppe besitzt, mit einer Verbindung der allgemeinen Formel

$$R—X \qquad XII$$

worin R niederes Alkyl bedeutet, und X obige Bedeutung besitzt, umsetzt, oder

g) eine Verbindung der allgemeinen Formel

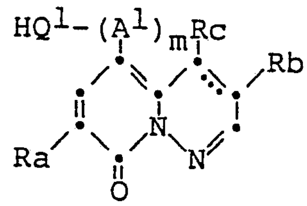

Ib

worin $A^1$, $Q^1$, Ra, Rb, Rc, m und die gestrichelte Linie obige Bedeutung besitzen, in Gegenwart eines säurebindenden Mittels mit einem reaktiven Derivat einer Carbonsäure der allgemeinen Formel

$$R^1—COOH \qquad XIII$$

worin $R^1$ obige Bedeutung besitzt, umsetzt, oder

h) eine Verbindung der allgemeinen Formel

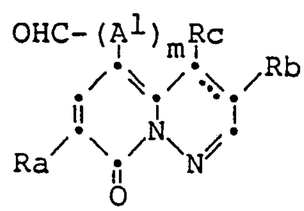

Ic

worin $A^1$, Ra, Rb, Rc, m und die gestrichelte Linie obige Bedeutung besitzen, in Gegenwart eines Reduktionsmittels mit einem Amin der obigen Formel IX oder X umsetzt, oder

i) eine Verbindung der allgemeinen Formel

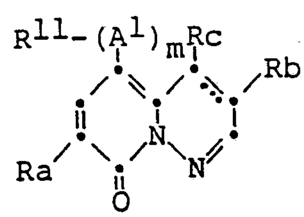

Id

worin $A^{11}$ nitro, Cyano oder niederes Alkoxycarbonbyl bedeutet, und $A^1$, Ra, Rb, Rc, m und die gestrichelte Linie die obige angegebene Bedeutung besitzen, oder eine Verbindung der obigen Formel Ia oder ein reaktives Derivat davon reduziert, oder

j) einen Alkohol der obigen Formel Ib' oder einen Alkohol der allgemeinen Formel

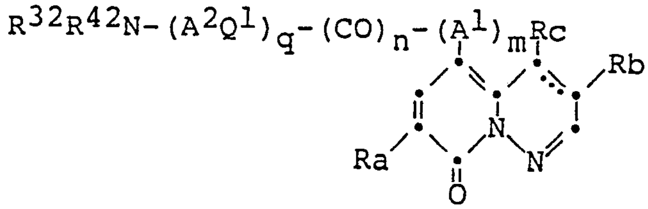

Ie

worin $A^1$, $A^2$, $Q^1$, Ra, Rb, Rc, m, n, q und die gestrichelte Linie obige Bedeutung besitzen, und $R^{32}$ und $R^{42}$ zusammen mit dem Stickstoffatom einen 3- bis 7-gliederigen, gegebenenfalls durch eine oder zwei niedere Alkylgruppen und durch eine Hydroxygruppe substituierten, gesättigten N-Heterocyclus bedeuten, der als Ringglied noch ein Sauerstoff- der Schwefelatom oder die Gruppe >N—$R^5$ enthalten kann, und $R^5$ obige Bedeutung besitzt,
oxidiert, oder

k) ein Isocyanat der allgemeinen Formel

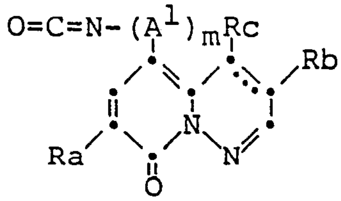

VIIIb oder $O=C-N-R^{33}$ XIV

worin $A^1$, Ra, Rb, Rc, m und die gestrichelte Linie obige Bedeutung besitzen, und $R^{33}$ Wasserstoff, niederes Alkyl oder $(C_{3-7})$-Cycloalkyl bedeutet,
mit einem niederen Alkohol oder einem Amin der obigen Formel X bzw. mit einer Verbindung der obigen Formel Ib umsetzt, oder

l) eine Verbindung der allgemeinen Formel

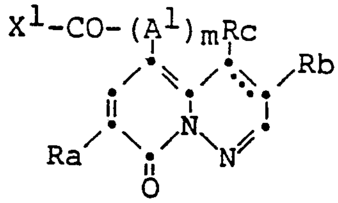

VIIIc

worin $X^1$ en Halogenatom bedeutet, und $A^1$, Ra, Rb, Rc, m und die gestrichelte Linie obige Bedeutung besitzen,
mit einem niederen Alkylmagnesiumhalogenid umsetzt, oder

m) eine Verbindung der allgemeinen Formel

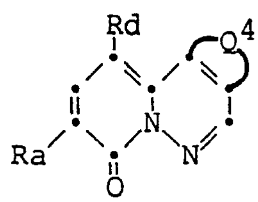

Ih

worin $Q^4$ die obige Gruppe (a) oder (b) bedeutet, und Ra und Rd obige Bedeutung besitzen,
am Thiophenring halogeniert, oder

n) eine Verbindung der obigen Formel VIIIc in Gegenwart einer Base mit einer Verbindung der allgemeinen Formel

$HYN=C(NH_2)—R''$ XV,

$H_2N—CHR''—CHR'''—Y'H$ oder XVI

$H_2N—NH—C(R'')=Y''$ XVII

worin Y ein Sauerstoffatom oder die Gruppe —NR'''—, Y' ein Sauerstoffatom oder die Gruppe —NH—, Y'' ein Sauerstoff- oder Schwefelatom und R'' und R''' je Wasserstoff oder niederes Alkyl bedeuten,

umsetzt und das erhaltene Produkt cyclisiert, oder

o) eine Verbindung der allgemeinen Formel

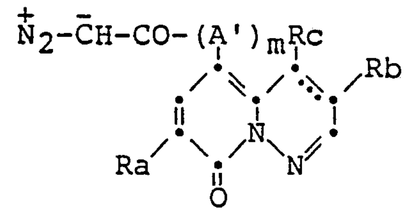

VIIId

worin A' $C_{1-6}$-Alkylen bedeutet, und Ra, Rb, Rc, die gestrichelte Linie und m obige Bedeutung besitzen, mit einem niederen Alkohol umsetzt, oder

p) eine Carbonsäure der Formel Ia, worin m die Zahl 0 bedeutet, decarboxyliert, oder

q) eine Verbindung der Formel I, worin Rd Wasserstoff bedeutet, am Pyridonring halogeniert, oder

r) in einer Verbindung der allgemeinen Formel

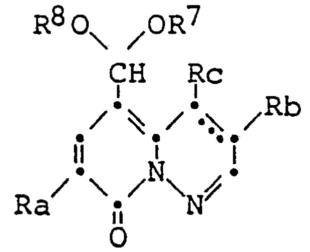

VIIIe

worin $R^7$ und $R^8$ je niederes Alkyl oder zusammen niederes Alkylen bedeuten, und Ra, Rb, Rc und die gestrichlete Linie obige Bedeutung besitzen, die Acetalgruppe spaltet, oder

s) eine Verbindung der allgemeinen Formel

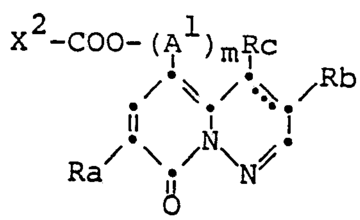

VIIIf

worin $X^2$ Phenoxy bedeutet, und $A^1$, Ra, Rb, Rc, die gestrichelte Linie und m obige Bedeutung besitzen, mit einem Amin der obigen Formel X umsetzt, und

t) erwünschtenfalls eine erhaltene Verbindung der Formel I mit einem basischen Substituenten in ein pharmazeutisch anehmbares Säureadditionssalz überführt.

2. Verfahren nach Anspruch 1, worin Ra Phenyl bedeutet.

3. Verfahren nach Ansprüche 1 oder 2, worin Rb und Rc zusammen mit dem mit α bezeichneten Kohlenstoffatom eine gegebenenfalls durch Halogen substituierte Gruppe der Formel $>C_\alpha—S—CH=CH—$ oder $>C_\alpha—CH=CH—CH=CH—$ und die gestrichelte Linie eine zusätzliche Bindung bedeuten.

4. Verfahren nach Anspruch 3, worin Rb und Rc zusammen mit dem mit α bezeichneten Kohlenstoffatom die Gruppe der Formel $>C_\alpha—S—CH=CH—$ oder $>C_\alpha—CH=CCl—CH=CH—$ bedeuten.

5. Verfahren nach einem der Ansprüche 1—4, worin $Q^1$ ein Sauerstoffatom, $A^2$ die Gruppe —CO—, $R^1$ die Gruppe $—NR^3R^4$, $R^3$ niederes Alkoxyalkyl und $R^4$ Wasserstoff oder niederes Alkyl oder $R^3$ und $R^4$ zusammen mit dem Stickstoffatom einen 4-, 5- oder 6-gliedrigen, gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierten, gesättigten H-Heterocyclus, der als Ringglied noch ein Sauerstoffatom enthalten kann, und entweder m und q die Zahl 0 und n die Zahl 1 oder m und q die Zahl 1 und n die Zahl 0 bedeuten.

6. Verfahren nach einem der Anspruch 5, worin $A^1$ Methylen und $R^3$ niederes Alkoxyalkyl und $R^4$ Wasserstoff oder niederes Alkyl oder $R^3$ und $R^4$ zusammen mit dem Stickstoffatom eine gegebenenfalls durch eine oder zwei niedere Alkylgruppen und gegebenenfalls durch eine Hydroxy-, niedere Alkoxy-, niedere Hydroxyalkyl- oder niedere Alkoxyalkylgruppe substituierte 1-Azetidinyl-, 1-Pyrrolidinyl-, 1-Piperidinyl- oder 4-Morpholinylgruppe bedeuten.

7. Verfahren nach Anspruch 6, worin $R^3$ 2-(niederes Alkoxy)äthyl und $R^4$ Wasserstoff oder niederes Alkyl oder $R^3$ und $R^4$ zusammen mit dem Stickstoffatom 3-(niederes Alkoxy)-1-azetidinyl, 3-(niederes

Alkoxy)-1-pyrrolidinyl, 2-(niederes Alkoxyalkyl)-1-pyrrolidinyl, 2-(niederes Hydroxyalkyl)-1-pyrrolidinyl, 4-Hydroxy-1-piperidinyl, 4-(niederes Alkoxy)-1-piperidinyl, 4-Morpholinyl oder 2,6-Di(niederes Alkyl)-4-morpholinyl bedeuten.

8. Verfahren nach einem der Ansprüche 1—7, worin m und q die Zahl 0 und n die Zahl 1 bedeuten.

9. Verfahren nach einem der Ansprüche 1—4, worin m und q die Zahl 0, n die Zahl 1 und $R^1$ Hydroxy oder niederes Alkoxy bedeuten.

10. Verfahren nach Anspruch 1, dadurch gekennzeichnet, dass man 3-Methoxy-1-[(4-oxo-3-phenyl-4H-pyrido[2,1-a)phthalazin-1-yl)carbonyl]azetidin herstellt.

11. Verfahren zur Herstellung von Arzneimitteln, insbesondere von muskelrelaxierend, sedativ-hypnotisch, anxiolytisch und/oder antikonvulsiv wirksamen Mitteln, dadurch gekennzeichnet, dass man eine Verbindung der in Anspruch 1 definierten Formel I oder ein pharmazeutisch annehmbarers Säureadditionssalz einer Verbindung der Formel I mit einem oder mehreren basischen Substituenten und gegebenenfalls einem andere therapeutisch wirksamen Stoff zusammen mit einem therapeutisch inerten Trägermaterial in eine galenische Darreichungsform bringt.

12. Verwendung von Verbindungen der in Anspruch 1 definierten Formel I zur Herstellung von muskelrelaxierend, sedativ-hypnotisch, anxiolytisch und/oder antikonvulsiv wirksamen Mitteln.

**Revendications pour les Etats contractants: BE CH DE FR GB IT LI LN NL SE**

1. Composés de formule générale

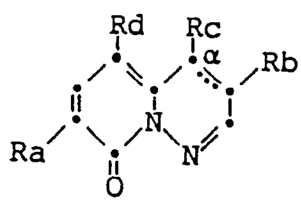

I

dans laquelle Ra désigne un groupe phényle, pyridyle ou thiényle, substitué le cas échéant par un halogène, un groupe trifluorométhyle, nitro, alkyle inférieur ou alcoxy inférieur, Rb et Rc forment avec l'atome de carbone désigné par α un groupe répondant à la formule $>C_\alpha—S—CH=CH—$ (a), $>C_\alpha—CH=CH—S—$ (b) ou $>C_\alpha—CH=CH—CH=CH—$ (c), substitué le cas échéant par un halogène, un groupe trifluorométhyle, alkyle inférieur, alcoxy inférieur, nitro, amino ou mono- ou di(alkyle inférieur)-amino, la ligne pointillée une liaison supplémentaire, Rd le groupe répondant à la formule $—(A^1)_m—(CO)_n—(Q^1A^2)_q—R^1$, m, n et q chacun le nombre 0 ou 1, $A^1$ un groupe alkylène inférieur, $A^2$ un groupe alkylène inférieur, une liaison directe ou le groupe —CO—, $Q^1$ un atome d'oxygène ou le groupe $—NR^2—$, $R^1$ l'hydrogène, un groupe hydroxy, cyano, nitro, un halogène, un groupe alcoxy inférieur, alkyle inférieur, (alcoxy inférieur)carbonyle, aryle, un groupe répondant à la formule $—NR^3R^4$ ou un hétérocycle saturé, partiellement insaturé ou aromatique, lié par un atome de carbone, à cinq maillons, substitué le cas échéant par un ou deux groupes alkyle inférieur et le cas échéant par un groupe cycloalkyle en $C_3$ à $C_6$, hydroxy, alcoxy inférieur, alcanoyleoxy inférieur, hydroxyalkyle inférieur, alcoxyalkyle inférieur, alcanoyloxyalkyle inférieur, (alcoxy inférieur)carbonyle, alcanoyle inférieur, carbamoyle, mono- ou di(alkyle inférieur)carbamoyle, oxo- ou alkylènedioxy, $R^2$ l'hydrogène, un groupe alkyle inférieur ou aryle, $R^3$ et $R^4$ chacun l'hydrogène, un groupe alkyle inférieur, alcoxyalkyle inférieur, dialcoxyalkyle inférieur, alkylène dioxyalkyle inférieur, cyanoalkyle inférieur, halogénoalkyle inférieur, hydroxyalkyle inférieur, dihydroxyalkyle inférieur, alcanoyle inférieur, (alcoxy inférieur)carbonyle ou un groupe cycloalkyle en $C_3$ à $C_7$ substitué le cas échéant par un groupe hydroxy, alcoxy inférieur, alcanoyloxy inférieur, hydroxyalkyle inférieur, alcoxyalkyle inférieur, alcanoyloxyalkyle inférieur, oxo, carbamoyle, mono- ou di(alkyle inférieur)carbamoyle ou par un groupe alkylènedioxy inférieur, ou bien $R^3$ et $R^4$ forment avec l'atome d'azote un hétérocycle azoté saturé, à trois à sept maillons, substitué le cas échéant par un ou deux groupes alkyle inférieur, et le cas échéant par un ou deux groupes hydroxy, alcoxy inférieur, alcanoyloxy inférieur, hydroxyalkyle inférieur, alcoxyalkyle inférieur, alcanoyloxyalkyle inférieur, (alcoxy inférieur)carbonyle, alcanoyle inférieur, carbamoyle, mono- ou di(alkyle inférieur)carbamoyle, oxo ou alkylènedioxy inférieur, qui peut encore contenir comme maillon du cycle un atome d'oxygène ou un atome de soufre, ou le groupe $>N—R^5$, et $R^5$ l'hydrogène, un groupe alkyle inférieur, hydroxyalkyle inférieur, alcoxyalkyle inférieur, alcanoyloxyalkyle inférieur, alcanoyle inférieur, (alcoxy inférieur)carbonyle, carbamoyle ou mono- ou di(alkyle inférieur)carbamoyle, sous réserve que n désigne le nombre 0 lorsque q désigne le nombre 1 et $A^2$ le groupe —CO—, que $R^1$ ait une signification différente de cyano, nitro, halogène ou (alcoxy inférieur)-carbonyle, lorsque q désigne le nombre 0 et n le nombre 1 ou lorsque q désigne le nombre 1 et $A^2$ le groupe —CO—, que $R^1$ ait une signification différente d'hydroxy, cyano, nitro, halogène, (alcoxy inférieur)-carbonyle, alcoxy inférieur et $—NR^3R^4$ lorsque q désigne le nombre 1 et $A^2$ représente une liaison directe, que Rd ait une signification différente d'acétyle lorsque Ra représente un phényle et Rb et Rc ensemble le groupe $—CH=CH—CH=CH—$ que les radicaux désignés comme inférieurs possèdant au plus 7 atomes de carbone, et qu'aryle désigne un groupe phényle substitué le cas échéant par un halogène, un groupe

trifluorométhyle, alkyle inférieur, alcoxy inférieur, nitro, amino ou mono- ou di(alkyle inférieur)amino, et les sels d'addition d'acides pharmaceutiquement acceptables des composés de formule I avec un ou plusieurs substituants basiques.

2. Composés selon la revendication 1 où représente un phényle.

3. Composés selon les revendications 1 ou 2, où Rb et Rc représentent avec l'atome de carbone désigné par α un groupe de formule $>C_\alpha—S—CH=CH—$ ou $>C_\alpha—CH=CH—CH=CH—$ substitué le cas échéant par un halogène et la ligne pointillée représente une liaison supplémentaire.

4. Composés selon les revendication 3, où Rb et Rc représentent avec l'atome de carbone désigné par le groupe de formule $>C_\alpha—S—CH=CH—$ ou $>C_\alpha—CH=CCl—CH=CH—$.

5. Composés selon l'une des revendications 1—4, où $Q^1$ représente un atome d'oxygène, $A^2$ le groupe —CO—, $R^1$ le groupe $—NR^3R^4$, $R^3$ un alcoxyalkyle inférieur et $R^4$ un hydrogène ou un alkyle inférieur, ou $R^3$ et $R^4$ représentent avec l'atome d'azote un hétérocycle azoté saturé à 4, 5 ou 6 maillons substitué le cas échéant par un ou deux groupes alkyle inférieur et le cas échéant par un groupe hydroxy, alcoxy inférieur, hydroxyalkyle inférieur ou alcoxyalkyle inférieur, qui peut encore contenir comme maillon un atome d'oxygène, et ou bien m et q représentent le nombre 0 et n le nombre 1, ou bien m et q représentent le nombre 1 et n le nombre 0.

6. Composés selon les revendication 5, où $A^1$ représente un méthylène et $R^3$ un alcoxyalkyle inférieur et $R^4$ représente un hydrogène ou un alkyle inférieur, ou $R^3$ et $R^4$ représentent avec l'atome d'azote un groupe 1-azétidinyle, 1-pyrrolidinyle, 1-pipéridinyle ou 4-morpholinyle substitué le cas échéant par un ou deux groupes alkyle et le cas échéant par un groupe hydroxy, alcoxy inférieur, hydroxyalkyle inférieur ou alcoxyalkyle inférieur.

7. Composés selon la revendication 6, où $R^3$ représente un 1-(alcoxy inférieur)éthyle et $R^4$ un hydrogène ou un alkyle inférieur, ou $R^3$ et $R^4$ représentent avec l'atome d'azote un 3-(alcoxy inférieur)-1-azétidinyle, 3-(alcoxy inférieur)-1-pyrrolidinyle, 2-(alcoxyalkyle inférieur)-1-pyrrolidinyle, 2-(hydroxyalkyle inférieur)-1-pyrrolidinyle, 4-hydroxy-1-pipéridinyle, 4-(alcoxy inférieur)-1-pipéridinyle, 4-morpholinyle ou 2,6-di(alkyle inférieur)-4-morpholinyle.

8. Composés selon l'une des revendications 1—7, où m et q représentent le nombre 0 et n le nombre 1.

9. Composés selon l'une des revendications 1—4, où m et q représentent le nombre 0, n le nombre 1 et $R^1$ un hydroxy ou un alcoxy inférieur.

10. 3-méthoxy-1-[(4-oxo-3-phényl-4H-pyrido[2,1-a]phtalazin-1-yl)carbonyl]azétidine.

11. Ester méthylique de l'acide 4-oxo-3-phényl-4H-pyrido[2,1-a]phtalazine-1-carboxylique.

12. Acide 4-oxo-3-phényl-4H-pyrido[2,1-a]phtalazine-1-carboxylique.

13. 3-hydroxy-1-[(4-oxo-3-phényl-4H-pyrido[2,1-a]phtalazin-1-yl)carbonyl]azétidine.

14. Composés de formule générale

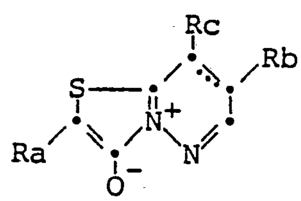

II

où Ra, Rb, Rc et la ligne pointillée ont la signification donnée dans la revendication 1, sous réserve que Ra a une signification différente de phényle lorsque Rb et Rc ensemble représentent le groupe —CH=CH—CH=CH— et la ligne pointillée représente une liaison supplémentaire.

15. Composés de formule générale

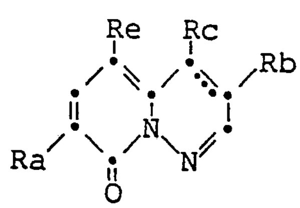

VIII

où Re représente le groupe

$$—(A^1)_m—OOC—X^2,\ —(A^1)_m—(CO)_n—(Q^1A^{22})_q—NR^{31}R^{41},\ —(A^1)_m—N=C=O,$$

$$—(A^1)_m—CO—X^1,\ —(A')_m—(CO)—CH^-—N_2^+ \text{ ou } —CH(OR^7)OR^8$$

et $A^1$, $Q^1$, Ra, Rb, Rc, m, n, q et la ligne pointillée ont la signification donnée dans la revendication 1 et A', $A^{22}$, $R^7$, $R^8$, $R^{31}$, $R^{41}$, $X^1$ et $X^2$ ont la signification donnée dans la revendication 19.

16. Composés de formules générales

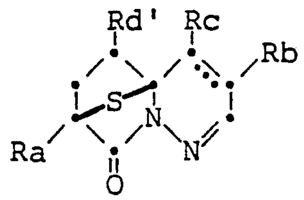

XVIII und

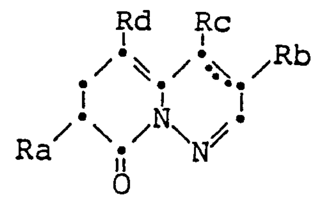

XIX

où Ra, Rb, Rc, Rd et la ligne pointillée ont la signification donnée dans la revendication 1 et Rd' a la signification donnée dans la revendication 19 sous réserve que Rd' ait une signification différente d'acétyle et d'éthoxycarbonyle lorsque Ra représente un phényle, Rb et Rc ensemble le groupe —CH=CH—CH=CH— et la ligne pointillée représente une liaison supplémentaire.

17. Composés selon l'une des revendications 1—13 aux fins d'application comme substances actives thérapeutiques.

18. Composés selon les revendication 17 aux fins d'application comme substances à action de relaxation musculaire, sédative-hypnotique, anxiolytique et/ou anticonvulsive.

19. Procédé de préparation de composés selon l'une des revendications 1—13 et de leurs sels d'addition d'acides pharmaceutiquement acceptables, caractérisé en ce que

a) on fait réagir un composé répondant à la formule générale

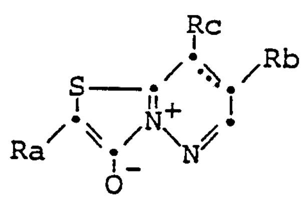

II

dans laquelle Ra, Rb, Rc et la ligne pointillée possèdent la signification donnée dans la revendication 1, à chaud, avec un composé répondant à la formule générale

$HC{\equiv}C—Rd'$ III ou $H_2C{=}CH—Rd'$ IV

dans laquelle Rd' désigne un groupe cyano, nitro ou le groupe répondant à la formule $—CO—(Q^1A^2)_q—R^1$, et q, $A^2$, $Q^1$ et $R^1$ possèdent la signification donnée dans la revendication 1, ou avec du sulfoxyde de phénylvinyle, et on traite le cas échéant le produit de cycloaddition obtenu avec une base forte, ou

b) on fait réagir un composé répondant à la formule générale

ROOC—C(Ra)=CHR'

dans laquelle R désigne un groupe alkyle inférieur et R' l'hydrogène ou un groupe alcoxy inférieur, et Ra possède la signification donnée dans la revendication 1, lorsque $R^1$ désigne l'hydrogène, à chaud ou lorsque $R^1$ désigne un groupe alcoxy inférieur, en présence d'une base forte, avec un composé répondant à la formule générale

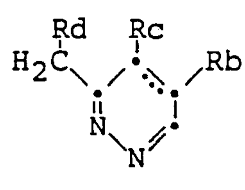

VI

dans laquelle Rb, Rc, Rd et la ligne pointillée possèdent la signification donnée dans la revendication 1, et on déshydrogène le produit de cyclocondensation obtenu, lorsque R' désigne l'hydrogène, ou

c) on hydrolyse un composé répondant à la formule I, qui possède un groupe carboxy estérifié, ou

d) on estérifie un acide carboxylique répondant à la formule générale

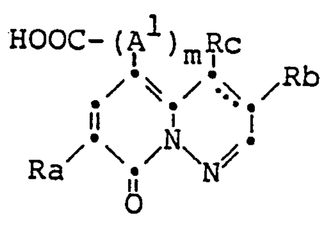

Ia

dans laquelle $A^1$, Ra, Rb, Rc, m et la ligne pointillée possèdent la signification donnée dans la revendication 1,
avec un alcool répondant à la formule générale

HO—$A^{21}$—$R^1$ VII

dans laquelle $A^{21}$ désigne un groupe alkylène inférieur ou une liaison directe, et $R^1$ possède la signification donnée dans la revendication 1, ou

e) on transforme un acide carboxylique répondant à la formule Ia précédente ou un acide carboxylique répondant à la formule générale

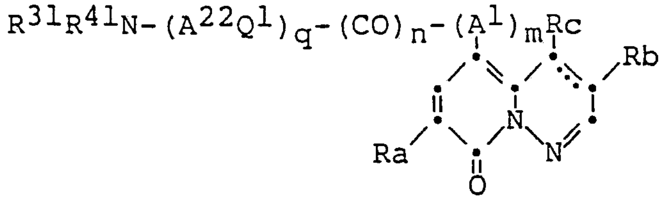

VIIIa

dans laquelle $A^{22}$ désigne un groupe alkylène inférieur ou le groupe —CO— et $R^{31}$ et $R^{41}$ forment avec l'atome d'azote un hétérocycle azoté saturé à 3 à 7 maillons, substitué le cas échéant par un ou deux groupes alkyle inférieur et par un groupe carboxy, qui peut encore contenir comme maillon du cycle un atome d'oxygène ou de soufre, ou le groupe >N—$R^5$, et $A^1$, $Q^1$, Ra, Rb, Rc, $R^5$, m, n, q et la ligne pointillée possèdent la signification donnée dans la revendication 1,

ou un de ses dérivés réactifs, avec une amine répondant à la formule générale

$HNR^2$—$A^{21}$—$R^1$ IX

ou $HNR^3R^4$ X

dans laquelle $R^1$, $R^2$, $R^3$ et R possèdent la signification donnée dans la revendication 1 et $A^{21}$ la signification précédente,
ou avec l'ammoniac ou une mono- ou di(alkyle inférieur)amine en l'amide correspondant ou

f) on fait réagir un composé répondant à la formule générale

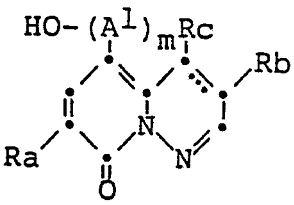

Ib'

dans laquelle $A^1$, Ra, Rb, Rc, m et la ligne pointillée possèdent la signification donnée dans la revendication 1,
en présence d'une base, avec un composé répondant à la formule générale

X—$A^{21}$—$R^1$ XI

dans laquelle X désigne un groupe partant, $A^{21}$ possède la signification précédente et $R^1$ la signification donnée dans la revendication 1,
ou un composé répondant à la formule I, qui possède un groupe hydroxy libre, avec un composé répondant à la formule générale

R—X XII

dans laquelle R désigne un groupe alkyle inférieur, et X possède la signification précédente, ou

g) on fait réagir un composé répondant à la formule générale

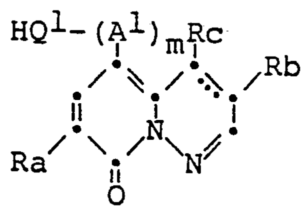

Ib

dans laquelle $A^1$, $Q^1$, Ra, Rb, Rc, m et la ligne pointillée possèdent la signification donnée dans la revendication 1,

en présence d'un agent fixant les acides, avec un dérivé réactif d'un acide carboxylique répondant à la formule générale

$$R^1—COOH \qquad XIII$$

dans laquelle $R^1$ possède la signification donnée dans la revendication 1, ou

h) on fait réagir un composé répondant à la formule générale

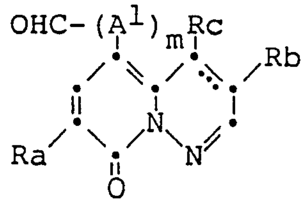

Ic

dans laquelle A', Ra, Rb, Rc, m et la ligne pointillée possèdent la significations donnée dans la revendication 1,

en présence d'un agent réducteur, avec une amine répondant à la formule IX ou X précédente, ou

i) on réduit un composé répondant à la formule générale

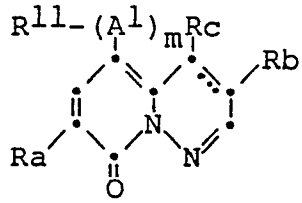

Id

dans laquelle $R^{11}$ désigne un groupe nitro, cyano ou (alcoxy inférieur)carbonyle et $A^1$, Ra, Rb, Rc, m et la ligne pointillée possèdent la signification donnée dans la revendication 1,

ou un composé répondant à la formule la précédente ou un de ces dérivés réactifs, ou

j) on oxyde un alcool répondant à la formule Ib' qui précède ou un alcool répondant à la formule générale

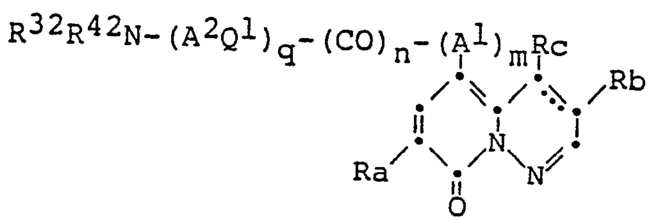

Ie

dans laquelle $A^1$, $A^2$, $Q^1$, Ra, Rb, Rc, m, n, q et la ligne pointillée possèdent la signification donnée dans la revendication 1, et $R^{32}$ et $R^{42}$ forment avec l'atome d'azote un hétérocycle azoté saturé à trois à sept maillons, substitué le cas échéant par un ou deux groupes alkyle inférieur et par un groupe hydroxy, qui peut encore contenir comme maillon du cycle un atome d'oxygène ou de soufre, ou le groupe >N—$R^5$ et $R^5$ possède la signification donnée dans la revendication 1, ou

k) on fait réagir un isocyanate répondant à la formule générale

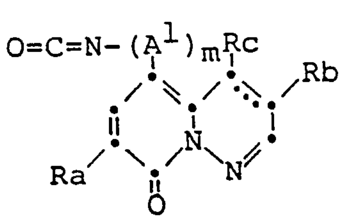

VIIIb ou $O=C-N-R^{33}$ XIV

dans laquelle $A^1$, Ra, Rb, Rc, m et la ligne pointillée possède la signification donnée dans la revendication 1, et $R^{33}$ désigne l'hydrogène, un groupe alkyle inférieur ou cycloalkyle en $C_3$ à $C_7$,

avec un alcool inférieur ou une amine répondant à la formule X précédente, ou avec un composé répondant à la formule Ib précédente, ou

l) on fait réagir un composé répondant à la formule générale

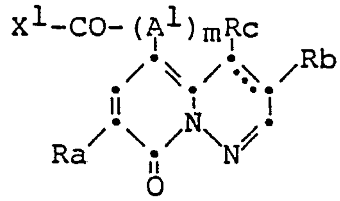

VIIIc

dans laquelle $X^1$ désigne un atome d'halogène, et $A^1$, Ra, Rb, Rc, m et la ligne pointillée possèdent la signification donnée dans la revendication 1, avec un halogénure d'alkylmagnésium inférieur, ou

m) on halogéne un composé répondant à la formule générale

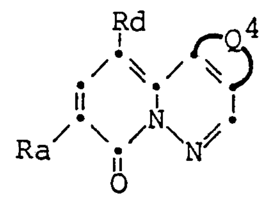

Ih

dans laquelle $Q^4$ désigne le groupe (a) ou (b) définie dans la revendication 1 et Ra et Rd ont la signification donnée dans la revendication 1, sur le cycle thiophène, ou

n) on fait réagir un composé répondant à la formule VIIIc précédente en présence d'une base, avec un composé répondant à la formule générale

$HYN{=}C(NH_2){-}R''$ XV

$H_2N{-}CHR''{-}CHR'''{-}Y'H$ XVI

ou $H_2N{-}NH{-}C(R''){=}Y''$ XVII

dans laquelle Y désigne un atome d'oxygène ou le groupe —NR'''—, Y' un atome d'oxygène ou le groupe —NH—, Y'' un atome d'oxygène ou de soufre et R'' et R''' chacun l'hydrogène ou un groupe alkyle inférieur,

et on cyclise le produit obtenu, ou

o) on fait réagir un composé répondant à la formule générale

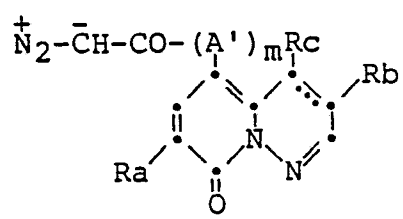

VIIId

dans laquelle A' désigne un groupe alkylène en $C_1$ à $C_6$, et Ra, Rb, Rc, la ligne pointillée et m possèdent la signification donnée dans la revendication 1,

avec un alcool inférieur, ou

p) on décarboxyle un acide carboxylique répondant à la formule Ia, dans laquelle m désigne le nombre 0, ou

q) on halogène un composé répondant à la formula I, dans lequel Rd désigne l'hydrogène, sur le cycle pyridone, ou

r) dans un composé répondant à la formule générale

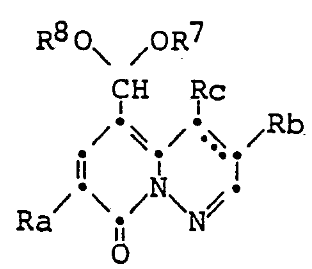

VIIIe

dans laquelle $R^7$ et $R^8$ désignent chacun un groupe alkyle inférieur ou ensemble un groupe alkylène inférieur, et Ra, Rb, Rc et la ligne pointillée possèdent la signification donnée dans la revendication 1, on coupe le groupe acetal, ou

s) on fait réagir un composé répondant à la formule générale

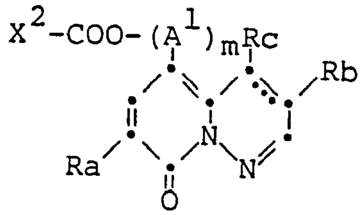

VIIIf

dans laquelle $X^2$ désigne un groupe phénoxy, et $A^1$, Ra, Rb, Rc, la ligne pointillée et m possèdent la signification donnée dans la revendication 1,
avec une amine répondant à la formule X précédente, et

t) si on le désire, on transforme un composé répondant à la formule I obtenue, portant un substituant basique, en un sel d'addition d'acide pharmaceutiquement acceptable.

20. Médicament contenant un composé selon l'une des revendications 1—13 et un support thérapeutiquement inerte.

21. Agent à action de relaxation musculaire, sédative-hypnotique, anxiolytique et/ou anticonvulsive contenant un composé selon l'une des revendications 1—13 et un support thérapeutiquement inerte.

22. Application de composés selon l'une des revendications 1—13 à la préparation d'agents à action de relaxation musculaire, sédative-hypnotique, anxiolytique et/ou anticonvulsive.

23. Application de composés selon l'une des revendications 14—16 à la préparation de substances actives thérapeutiques.

24. Application de composés selon l'une des revendications 14—16 à la préparation de composés selon l'une des revendications 1—13.

**Revendications pour l'Etat contractant: GR**

1. Procédé de préparation de composés répondant à la formule générale

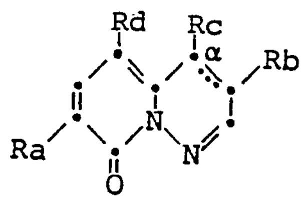

I

dans laquelle Ra désigne un groupe phényle, pyridyle ou thiényle, substitué le cas échéant par un halogène, un groupe trifluorométhyle, nitro, alkyle inférieur ou alcoxy inférieur, Rb et Rc forment avec l'atome de carbone désigné par α un groupe répondant à la formule $>C_\alpha—S—CH=CH—$ (a), $>C_\alpha—CH=CH—S—$ (b) $>C_\alpha—CH=CH—CH=CH—$ (c), substitué le cas échéant par un halogène, un groupe trifluorométhyle, alkyle inférieur, alcoxy inférieur, nitro, amino ou mono- ou di(alkyle inférieur)amino, la ligne pointillée une liaison supplémentaire, Rd le groupe répondant à la formule $—(A^1)_m—(CO)_n—(Q^1A^2)_q—R^1$, m, n et q chacun le nombre 0 ou 1, $A^1$ un groupe alkylène inférieur, $A^2$ un groupe alkylène inférieur, une liaison directe ou le groupe —CO—, $Q^1$ un atome d'oxygène ou le groupe $—NR^2—$, $R^1$ l'hydrogène, un groupe hydroxy, cyano, nitro, un halogène, un groupe alcoxy inférieur, alkyle inférieur, (alcoxy inférieur)carbonyle, aryle, un groupe répondant à la formule $—NR^3R^4$ ou un hétérocycle saturé, partiellement insaturé ou aromatique, lié par un atome de carbone, à cinq maillons, substitué le cas échéant par un ou deux groupes alkyle inférieur et le cas échéant par un groupe cycloalkyle en $C_3$ à $C_6$, hydroxy, alcoxy inférieur, alcanoyloxy inférieur, hydroxyalkyle inférieur, alcoxyalkyle inférieur, alcanoyloxyalkyle inférieur, (alcoxy inférieur)carbonyle, alcanoyle inférieur, carbamoyle inférieur, mono- ou di-(alkyle inférieur)carbamoyle, oxo- ou alkylenedioxy, $R^2$ l'hydrogène, un groupe alkyle inférieur ou aryle, $R^3$ et $R^4$ chacun l'hydrogène, un groupe alkyle inférieur, alcoxyalkyle inférieur, dialcoxyalkyle inférieur, alkylène dioxyalkyle inférieur, cyanoalkyle inférieur, halogénoalkyle inférieur, hydroxyalkyle inférieur, dihydroxyalkyle inférieur, alcanoyle inférieur, (alcoxy inférieur)carbonyle ou un groupe cycloalkyle en $C_3$ à $C_7$ substitué le cas échéant par un groupe hydroxy, alcoxy inférieur, alcanoyloxy inférieur, hydroxyalkyle inférieur, alcoxyalkyle inférieur, alcanoyloxyalkyle inférieur, oxo, carbamoyle, mono- ou di(alkyle inférieur)carbamoyle ou par un groupe alkylènedioxy inférieur, ou bien $R^3$ et $R^4$ forment avec l'atome d'azote un hétérocycle azoté saturé, à trois à sept maillons, substitué le cas échéant par un ou deux groupes alkyle inférieur, et le cas échéant par un ou deux groupes hydroxy, alcoxy inférieur, alcanoyloxy inférieur, hydroxyalkyle inférieur, alcoxyalkyle inférieur, alcanoyloxyalkyle inférieur, (alcoxy inférieur)carbonyle,

alcanoyle inférieur, carbamoyle, mono- ou di(alkyle inférieur)carbamoyle, oxo ou alkylènedioxy inférieur, qui peut encore contenir comme maillon du cycle un atome d'oxygène ou un atome de soufre, ou le groupe >N—$R^5$, et $R^5$ l'hydrogène, un groupe alkyle inférieur, hydroxyalkyle inférieur, alcoxyalkyle inférieur, alcanoyloxyalkyle inférieur, alcanoyle inférieur, (alcoxy inférieur)carbonyle, carbamoyle ou mono- ou di(alkyle inférieur)carbamoyle, sous réserve que n désigne le nombre 0 lorsque q désigne le nombre 1 et $A^2$ le groupe —CO—, que $R^1$ ait une signification différente de cyano, nitro, halogène ou (alcoxy inférieur)-carbonyle, lorsque q désigne le nombre 0 et n le nombre 1 ou lorsque q désigne le nombre 1 et $A^2$ le groupe —CO—, que $R^1$ ait une signification différente d'hydroxy, cyano, nitro, halogène, (alcoxy inférieur)-carbonyle, alcoxy inférieur et —$NR^3R^4$ lorsque q désigne le nombre 1 et $A^2$ représente une liaison directe, que Rd ait une signification différente d'acétyle lorsque Ra représente un phényle et Rb et Rc ensemble le groupe —CH=CH—CH=CH— que les radicaux désignés comme inférieurs possèdant au plus 7 atomes de carbone, et qu'aryle désigne un groupe phényle substitué le cas échéant par un halogène, un groupe trifluorométhyle, alkyle inférieur, alcoxy inférieur, nitro, amino ou mono- ou di(alkyle inférieur)amino, et de sels d'addition d'acides pharmaceutiquement acceptables des composés répondant à la formule I ayant un ou plusieurs substituants basiques, caractérisé en ce que

a) on fait réagir un composé répondant à la formule générale

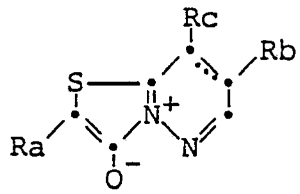

II

dans laquelle Ra, Rb, Rc et la ligne pointillée possèdent la signification précédente, à chaud, avec un composé répondant à la formule générale

$HC{\equiv}C$—Rd' III ou $H_2C{=}CH$—Rd' IV

dans laquelle Rd' désigne un groupe cyano, nitro ou le groupe répondant à la formule —CO—$(Q^1A^2)_q$—$R^1$, et q, $A^2$, $Q^1$ et $R^1$ possèdent la signification précédente, ou avec du sulfoxyde de phénylvinyle, et on traite le cas échéant le produit de cycloaddition obtenu avec une base forte, ou

b) on fait réagir un composé répondant à la formule générale

ROOC—C(Ra)=CHR' V

dans laquelle R désigne un groupe alkyle inférieur et R' l'hydrogène ou un groupe alcoxy inférieur, et Ra possède la signification ci-dessus, lorsque $R^1$ désigne l'hydrogène, à chaud ou lorsque R' désigne un groupe alcoxy inférieur, en présence d'une base forte, avec un composé répondant à la formule générale

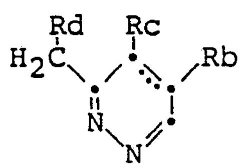

VI

dans laquelle Rb, Rc, Rd et la ligne pointillée possèdent la signification précédente, et on déshydrogène le produit de cyclocondensation obtenu, lorsque R' désigne l'hydrogène, ou

c) on hydrolyse un composé répondant à la formule I, qui possède un groupe carboxy estérifié, ou

d) on estérifie un acide carboxylique répondant à la formule générale

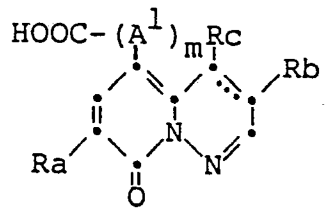

Ia

dans laquelle $A^1$, Ra, Rb, Rc, m et la ligne pointillée possèdant la signification précédente, avec un alcool répondant à la formule générale

$HO—A^{21}—R^1$ VII

dans laquelle $A^{21}$ désigne un groupe alkylène inférieur ou une liaison directe, et $R^1$ possède la signification qui précède, ou

e) on transforme un acide carboxylique répondant à la formule Ia précédente ou un acide carboxylique répondant à la formule générale

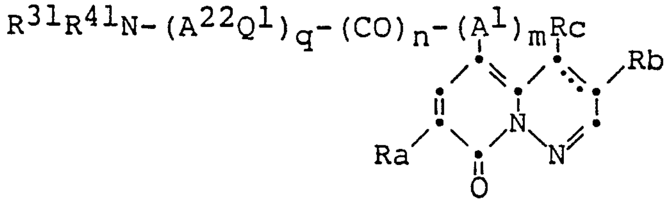

VIIIa

dans laquelle $A^{22}$ désigne un groupe alkylène inférieur ou le groupe —CO— et $R^{31}$ et $R^{41}$ forment avec l'atome d'azote un hétérocycle azoté saturé à 3 à 7 maillons, substitué le cas échéant par un ou deux groupes alkyle inférieur et par un groupe carboxy, qui peut encore contenir comme maillon du cycle un atome d'oxygène ou de soufre, ou le groupe $>N—R^5$, et $A^1$, $Q^1$, Ra, Rb, Rc, $R^5$, m, n, q et la ligne pointillée possèdent la signification précédente,

ou un de ses dérivés réactifs, avec une amine répondant à la formule générale

$HNR^2—A^{21}—R^1$ IX

ou $HNR^3R^4$ X

dans laquelle $A^{21}$, $R^1$, $R^2$, $R^3$ et $R^4$ possèdent la signification précédente,
ou avec l'ammoniac ou une mono- ou di(alkyle inférieur)amine en l'amide correspondant ou

f) on fait réagir un composé répondant à la formule générale

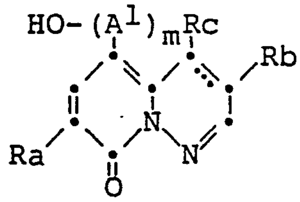

Ib'

dans laquelle $A^1$, Ra, Rb, Rc, m et la ligne pointillée possèdent la signification précédente,
en présence d'une base, avec un composé répondant à la formule générale

$X—A^{21}—R^1$ XI

dans laquelle X désigne un groupe partant, $A^{21}$ et $R^1$ possèdent la signification précédente,
ou un composé répondant à la formule I, qui possède un groupe hydroxy libre, avec un composé répondant à la formule générale

$R—X$ XII

dans laquelle R désigne un groupe alkyle inférieur, et X possède la signification précédente, ou

g) on fait réagir un composé répondant à la formule générale

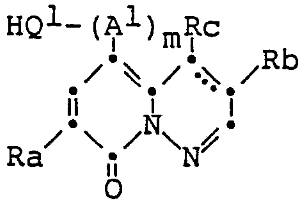

Ib

dans laquelle $A^1$, $Q^1$, Ra, Rb, Rc, m et la ligne pointillée possèdent la signification précédente,

en présence d'un agent fixant les acides, avec un dérivé réactif d'un acide carboxylique répondant à la formule générale

$R^1—COOH$ XIII

dans laquelle $R^1$ possède la signification précédente, ou

h) on fait réagir un composé répondant à la formule générale

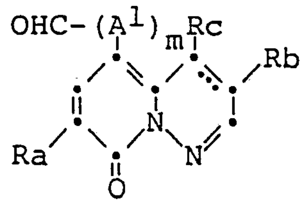

Ic

dans laquelle A', Ra, Rb, Rc, m et la ligne pointillée possèdent la signification précédente, en présence d'un agent réducteur, avec une amine répondant à la formule IX ou X précédente, ou

i) on réduit un composé répondant à la formule générale

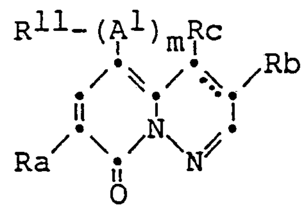

Id

dans laquelle $R^{11}$ désigne un groupe nitro, cyano ou (alcoxy inférieur)carbonyle et $A^1$, Ra, Rb, Rc, m et la ligne pointillée possèdent la signification précédemment indiquée, ou un composé répondant à la formule Ia précédente ou un de ces dérivés réactifs, ou

j) on oxyde un alcool répondant à la formule Ib' qui précède ou un alcool répondant à la formule générale

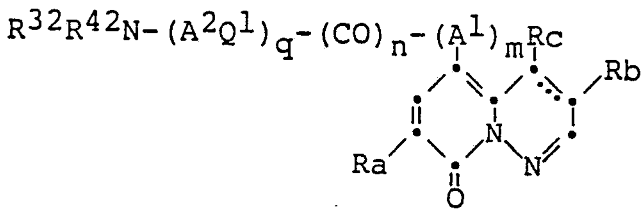

Ie

dans laquelle $A^1$, $A^2$, $Q^1$, Ra, Rb, Rc, m, n, q et la ligne pointillée possèdent la signification précédente, et $R^{32}$ et $R^{42}$ forment avec l'atome d'azote un hétérocycle azoté saturé à trois à sept maillons, substitué le cas échéant par un ou deux groupes alkyle inférieur et par un groupe hydroxy, qui peut encore contenir comme maillon du cycle un atome d'oxygène ou de soufre, ou le groupe >N—$R^5$ et $R^5$ possède la signification précédente ou

k) on fait réagir un isocyanate répondant à la formule générale

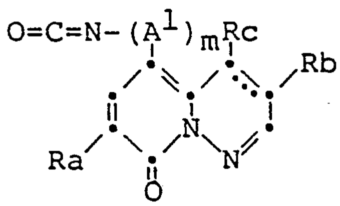

VIIIb ou $O{=}C{-}N{-}R^{33}$ XIV

dans laquelle $A^1$, Ra, Rb, Rc, m et la ligne pointillée possèdent la signification précédente, et $R^{33}$ désigne l'hydrogène, un groupe alkyle inférieur ou cycloalkyle en $C_3$ à $C_7$, avec un alcool inférieur ou une amine répondant à la formule X précédente, ou avec un composé répondant à la formule Ib précédente, ou

l) on fait réagir un composé répondant à la formule générale

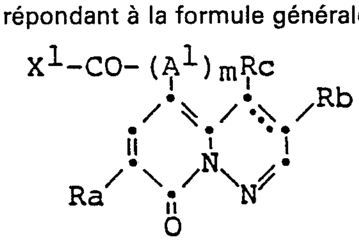

VIIIc

dans laquelle $X^1$ désigne un atome d'halogène, et $A^1$, Ra, Rb, Rc, m et la ligne pointillée possèdent la signification précédente, avec un halogénure d'alkylmagnésium inférieur, ou

m) on halogéne un composé répondant à la formule générale

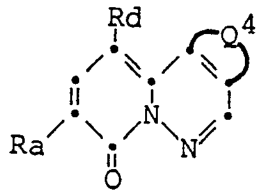

Ih

dans laquelle $Q^4$ désigne le groupe (a) ou (b) ci-dessus et Ra et Rd ont la signification précédente sur le cycle thiophène, ou

n) on fait réagir un composé répondant à la formule VIIIc précédente en présence d'une base, avec un composé répondant à la formule générale

$$HYN{=}C(NH_2){-}R'' \quad XV$$

$$H_2N{-}CHR''{-}CHR'''{-}Y'H \quad XVI$$

$$\text{ou } H_2N{-}NH{-}C(R''){=}Y'' \quad XVII$$

dans laquelle Y désigne un atome d'oxygène ou le groupe —NR'''—, Y' un atome d'oxygène ou le groupe —NH—, Y'' un atome d'oxygène ou de soufre et R'' et R''' chacun l'hydrogène ou un groupe alkyle inférieur,
et on cyclise le produit obtenu, ou

o) on fait réagir un composé répondant à la formule générale

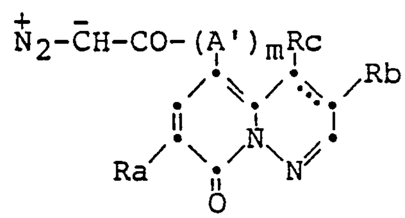

VIIId

dans laquelle A' désigne un groupe alkylène en $C_1$ à $C_6$, et Ra, Rb, Rc, la ligne pointillée et m possèdent la signification précédente,
avec un alcool inférieur, ou

p) on décarboxyle un acide carboxylique répondant à la formule Ia, dans laquelle m désigne le nombre 0, ou

q) on halogène un composé répondant à la formula I, dans lequel Rd désigne l'hydrogène, sur le cycle pyridone, ou

r) dans un composé répondant à la formule générale

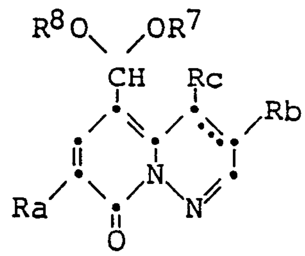

VIIIe

dans laquelle $R^7$ et $R^8$ désignent chacun un groupe alkyle inférieur ou ensemble un groupe alkylène inférieur, et Ra, Rb, Rc et la ligne pointillée possèdent la signification précédente,
on coupe le groupe acetal, ou

s) on fait réagir un composé répondant à la formule générale

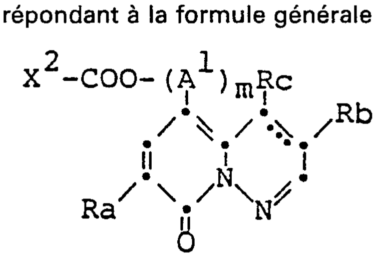

VIIIf

dans laquelle $X^2$ désigne un groupe phénoxy, et $A^1$, Ra, Rb, Rc, la ligne pointillée et m possèdent la signification précédente,
avec une amine répondant à la formule X précédente, et

t) si on le désire, on transforme un composé répondant à la formule I obtenue, portant un substituant basique, en un sel d'addition d'acide pharmaceutiquement acceptable.

2. Procédé selon la revendication 1 dans lequel Ra représente un phényle.

3. Procédé selon les revendications 1 ou 2, dans lequel Rb et Rc représentent avec l'atome de carbone désigné par α un groupe de formule $>C_\alpha—S—CH=CH—$ ou $>C_\alpha—CH=CH—CH=CH—$ substitué le cas échéant par un halogène et la ligne pointillée représente une liaison supplémentaire.

4. Procédé selon la revendication 3, dans lequel Rb et Rc représentent avec l'atome de carbone désigné par le groupe de formule $>C_\alpha—S—CH=CH—$ ou $>C_\alpha—CH=CCl—CH=CH—$.

5. Procédé selon l'une des revendications 1—4, dans lequel $Q^1$ représente un atome d'oxygène, $A^2$ le groupe —CO—, $R^1$ le groupe $—NR^3R^4$, $R^3$ un alcoxyalkyle inférieur et $R^4$ un hydrogène ou un alkyle inférieur, ou $R^3$ et $R^4$ représentent avec l'atome d'azote un hétérocycle azoté saturé à 4, 5 ou 6 maillons substitué le cas échéant par un ou deux groupes alkyle inférieur et le cas échéant par un groupe hydroxy, alcoxy inférieur, hydroxyalkyle inférieur ou alcoxyalkyle inférieur, qui peut encore contenir comme maillon un atome d'oxygène, et ou bien m et q représentent le nombre 0 et n le nombre 1, ou bien m et q représentent le nombre 1 et n le nombre 0.

6. Procédé selon la revendication 5, dans lequel $A^1$ représente un méthylène et $R^3$ un alcoxyalkyle inférieur et $R^4$ représente un hydrogène ou un alkyle inférieur, ou $R^3$ et $R^4$ représentent avec l'atome d'azote un groupe 1-azétidinyle, 1-pyrrolidinyle, 1-pipéridinyle ou 4-morpholinyle substitué le cas échéant par un ou deux groupes alkyle et le cas échéant par un groupe hydroxy, alcoxy inférieur, hydroxyalkyle inférieur ou alcoxyalkyle inférieur.

7. Procédé selon la revendication 6, dans lequel $R^3$ représente un 2-(alcoxy inférieur)éthyle et $R^4$ un hydrogène ou un alkyle inférieur, ou $R^3$ et $R^4$ représentent avec l'atome d'azote un 3-(alcoxy inférieur)-1-azétidinyle, 3-(alcoxy inférieur)-1-pyrrolidinyle, 2-(alcoxyalkyle inférieur)-1-pyrrolidinyle, 2-(hydroxyalkyle inférieur)-1-pyrrolidinyle, 4-hydroxy-1-pipéridinyle, 4-(alcoxy inférieur)-1-pipéridinyle, 4-morpholinyle ou 2,6-di(alkyle inférieur)-4-morpholinyle.

8. Procédé selon l'une des revendications 1—7, dans lequel m et q représentent le nombre 0 et n le nombre 1.

9. Procédé selon l'une des revendications 1—4, dans lequel m et q représentent le nombre 0, n le nombre 1 et $R^1$ un hydroxy ou un alcoxy inférieur.

10. Procédé selon la revendication 1, caractérisé en ce qu'on prépare la 3-méthoxy-1-[(4-oxo-3-phényl-4H-pyrido[2,1-a]phtalazin-1-yl)carbonyl]azétidine.

11. Procédé de préparation de médicaments, en particulier de relaxants musculaires, d'agents à action sédative-hypnotique, anxiolytique et/ou anti-convulsive, caractérisé en ce qu'on met sous une forme d'administration galénique un composé de formule I définie dans la revendication 1 ou un sel d'addition d'acide pharmaceutiquement acceptable d'un composé de formule I avec un ou plusieurs substituants basiques et la cas échéant un autre corps thérapeutiquement efficace avec un support thérapeutiquement inerte.

12. Application de composés de formule I définie dans la revendication 1 à la préparation d'agents à action de relaxation musculaire, sédative-hypnotique, anxiolytique et/ou anticonvulsive.

13. Composés de formule générale

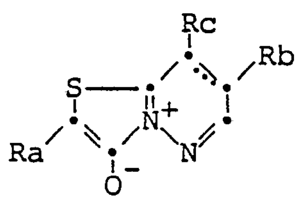

II

où Ra, Rb, Rc et la ligne pointillée ont la signification donnée dans la revendication 1, sous réserve que Ra a une signification différente de phényle lorsque Rb et Rc ensemble représentent le groupe —CH=CH—CH=CH— et la ligne pointillée représente une liaison supplémentaire.

14. Composés de formule générale

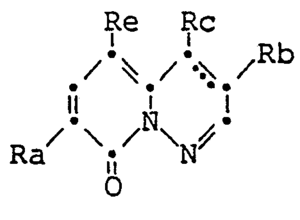

VIII

où Re représente le groupe

$$-(A^1)_m-OOC-X^2,\ -(A^1)_m-(CO)_n-(Q^1A^{22})_q-NR^{31}R^{41},\ -(A^1)_m-N{=}C{=}O,$$

$$-(A^1)_m-CO-X^1,\ -(A')_m-(CO)-CH^{-}-N_2^{+}\ \text{ou}\ -CH(OR^7)OR^8$$

et $A^1$, $Q^1$, Ra, Rb, Rc, m, n, q et la ligne pointillée ainsi que A', $A^{22}$, $R^7$, $R^8$, $R^{31}$, $R^{41}$, $X^1$ et $X^2$ ont la signification donnée dans la revendication 1.

15. Composés de formules générales

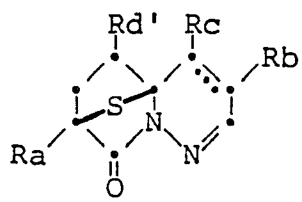
XVIII et

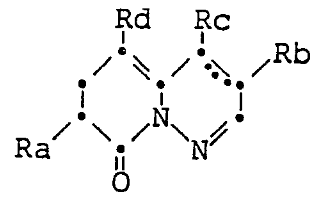
XIX

où Ra, Rb, Rc, Rd et la ligne pointillée ainsi que Rd' ont la signification donnée dans la revendication 1, sous réserve que Rd' ait une signification différente d'acétyle et d'éthoxycarbonyle lorsque Ra représente un phényle, Rb et Rc ensemble le groupe —CH=CH—CH=CH— et la ligne pointillée une liaison supplémentaire.

**Revendications pour les Etats contractants: AT ES**

1. Procédé de préparation de composés répondant à la formule générale

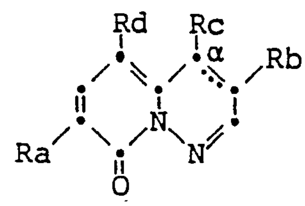
I

dans laquelle Ra désigne un groupe phényle, pyridyle ou thiényle, substitué le cas échéant par un halogène, un groupe trifluorométhyle, nitro, alkyle inférieur ou alcoxy inférieur, Rb et Rc forment avec l'atome de carbone désigné par α un groupe répondant à la formule $>C_\alpha-S-CH{=}CH-$ (a), $>C_\alpha-CH{=}CH-S-$ (b) ou $>C_\alpha-CH{=}CH-CH{=}CH-$ (c), substitué le cas échéant par un halogène, un groupe trifluorométhyle, alkyle inférieur, alcoxy inférieur, nitro, amino ou mono- ou di(alkyle inférieur)-amino, la ligne pointillée une liaison supplémentaire, Rd le groupe répondant à la formule $-(A^1)_m-(CO)_n-(Q^1A^2)_q-R^1$, m, n et q chacun le nombre 0 ou 1, $A^1$ un groupe alkylène inférieur, $A^2$ un groupe alkylène inférieur, une liaison directe ou le groupe —CO—, $Q^1$ un atome d'oxygène ou le groupe $-NR^2-$, $R^1$ l'hydrogène, un groupe hydroxy, cyano, nitro, un halogène, un groupe alcoxy inférieur, alkyle inférieur, (alcoxy inférieur)carbonyle, aryle, un groupe répondant à la formule $-NR^3R^4$ ou un hétérocycle saturé, partiellement insaturé ou aromatique, lié par un atome de carbone, à cinq maillons, substitué le cas échéant par un ou deux groupes alkyle inférieur et le cas échéant par un groupe cycloalkyle en $C_3$ à $C_6$, hydroxy, alcoxy inférieur, alcanoyloxy inférieur, hydroxyalkyle inférieur, alcoxyalkyle inférieur, alcanoyloxyalkyle inférieur, (alcoxy inférieur)carbonyle, alcanoyle inférieur, carbamoyle inférieur, mono- ou di-(alkyle inférieur)carbamoyle, oxo- ou alkylènedioxy, $R^2$ l'hydrogène, un groupe alkyle inférieur ou aryle, $R^3$ et $R^4$ chacun l'hydrogène, un groupe alkyle inférieur, alcoxyalkyle inférieur, dialcoxyalkyle inférieur, alkylène dioxyalkyle inférieur, cyanoalkyle inférieur, halogénoalkyle inférieur, hydroxyalkyle inférieur, dihydroxyalkyle inférieur, alcanoyle inférieur, (alcoxy inférieur)carbonyle ou un groupe cycloalkyle en $C_3$ à $C_7$ substitué le cas échéant par un groupe hydroxy, alcoxy inférieur, alcanoyloxy inférieur, hydroxyalkyle inférieur, alcoxyalkyle inférieur, alcanoyloxyalkyle inférieur, oxo, carbamoyle, mono- ou di(alkyle inférieur)carbamoyle ou par un groupe alkylènedioxy inférieur, ou bien $R^3$ et $R^4$ forment avec l'atome d'azote un hétérocycle azoté saturé, à trois à sept maillons, substitué le cas échéant par un ou deux groupes alkyle inférieur, et le cas échéant par un ou deux groupes hydroxy, alcoxy inférieur, alcanoyloxy inférieur, hydroxyalkyle inférieur, alcoxyalkyle inférieur, alcanoyloxyalkyle inférieur, (alcoxy inférieur)carbonyle, alcanoyle inférieur, carbamoyle, mono- ou di(alkyle inférieur)carbamoyle, oxo ou alkylènedioxy inférieur, qui peut encore contenir comme maillon du cycle un atome d'oxygène ou un atome de soufre, ou le groupe $>N-R^5$, et $R^5$ l'hydrogène, un groupe alkyle inférieur, hydroxyalkyle inférieur, alcoxyalkyle inférieur, alcanoyloxyalkyle inférieur, alcanoyle inférieur, (alcoxy inférieur)carbonyle, carbamoyle ou mono- ou di(alkyle inférieur)carbamoyle, sous réserve que n désigne le nombre 0 lorsque q désigne le nombre 1 et $A^2$ le groupe —CO—, que $R^1$ ait une signification différente de cyano, nitro, halogène ou (alcoxy inférieur)-carbonyle, lorsque q désigne le nombre 0 et n le nombre 1 ou lorsque q désigne le nombre 1 et $A^2$ le groupe —CO—, que $R^1$ ait une signification différente d'hydroxy, cyano, nitro, halogène, (alcoxy inférieur)-carbonyle, alcoxy inférieur et $—NR^3R^4$ lorsque q désigne le nombre 1 et $A^2$ représente une liaison directe, que Rd ait une signification différente d'acétyle lorsque Ra représente un phényle et Rb et Rc ensemble le groupe —CH=CH—CH=CH— que les radicaux désignés comme inférieurs possèdent au plus 7 atomes de carbone, et qu'aryle désigne un groupe phényle substitué le cas échéant par un halogène, un groupe trifluorométhyle, alkyle inférieur, alcoxy inférieur, nitro, amino ou mono- ou di(alkyle inférieur)amino, et de sels d'addition d'acide pharmaceutiquement acceptables des composés répondant à la formule I ayant un ou plusieurs substituants basiques, caractérisé en ce que

a) on fait réagir un composé répondant à la formule générale

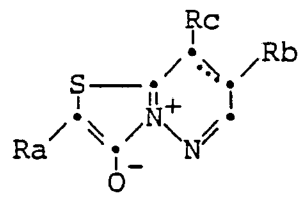

II

dans laquelle Ra, Rb, Rc et la ligne pointillée possèdent la signification précédente, à chaud, avec un composé répondant à la formule générale

$HC \equiv C—Rd'$ III ou $H_2C=CH—Rd'$ IV

dans laquelle Rd' désigne un groupe cyano, nitro ou le groupe répondant à la formule $—CO—(Q^1A^2)_q—R^1$, et q, $A^2$, $Q^1$ et $R^1$ possèdent la signification précédente,
ou avec du sulfoxyde de phénylvinyle, et on traite le cas échéant le produit de cycloaddition obtenu avec une base forte, ou

b) on fait réagir un composé répondant à la formule générale

ROOC—C(Ra)=CHR' V

dans laquelle R désigne un groupe alkyle inférieur et R' l'hydrogène ou un groupe alcoxy inférieur, et Ra possède la signification ci-dessus, lorsque R' désigne l'hydrogène, à chaud ou lorsque R' désigne un groupe alcoxy inférieur, en présence d'une base forte, avec un composé répondant à la formule générale

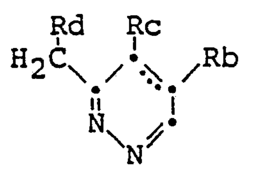

VI

dans laquelle Rb, Rc, Rd et la ligne pointillée possèdent la signification précédente,
et on déshydrogène le produit de cyclocondensation obtenu, lorsque R' désigne l'hydrogène, ou

c) on hydrolyse un composé répondant à la formule I, qui possède un groupe carboxy estérifié, ou

d) on estérifie un acide carboxylique répondant à la formule générale

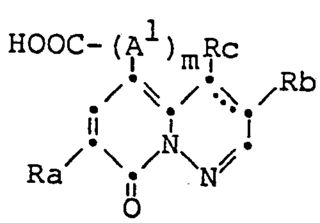

Ia

dans laquelle $A^1$, Ra, Rb, Rc, m et la ligne pointillée possèdant la signification précédente, avec un alcool répondant à la formule générale

$HO—A^{21}—R^1$ VII

dans laquelle $A^{21}$ désigne un groupe alkylène inférieur ou une liaison directe, et $R^1$ possède la signification qui précède, ou

e) on transforme un acide carboxylique répondant à la formule Ia précédente ou un acide carboxylique répondant à la formule générale

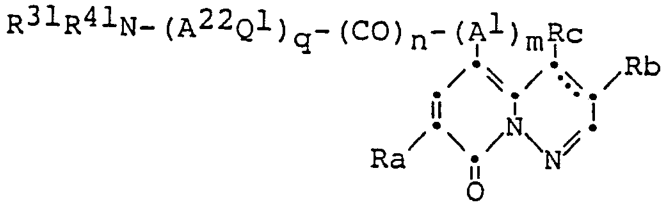

VIIIa

dans laquelle $A^{22}$ désigne un groupe alkylène inférieur ou le groupe —CO— et $R^{31}$ et $R^{41}$ forment avec l'atome d'azote un hétérocycle azoté saturé à 3 à 7 maillons, substitué le cas échéant par un ou deux groupes alkyle inférieur et par un groupe carboxy, qui peut encore contenir comme maillon du cycle un atome d'oxygène ou de soufre, ou le groupe >N—$R^5$, et $A^1$, $Q^1$, Ra, Rb, Rc, $R^5$, m, n, q et la ligne pointillée possèdent la signification précédente,

ou un de ses dérivés réactifs, avec une amine répondant à la formule générale

$$HNR^2—A^{21}—R^1 \qquad IX$$

$$\text{ou } HNR^3R^4 \qquad X$$

dans laquelle $A^{21}$, $R^1$, $R^2$, $R^3$ et $R^4$ possèdent la signification précédente,
ou avec l'ammoniac ou une mono- ou di(alkyle inférieur)amine en l'amide correspondant ou

f) on fait réagir un composé répondant à la formule générale

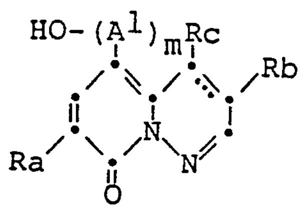

Ib'

dans laquelle $A^1$, Ra, Rb, Rc, m et la ligne pointillée possèdent la signification précédente,
en présence d'une base, avec un composé répondant à la formule générale

$$X—A^{21}—R^1 \qquad XI$$

dans laquelle X désigne un groupe partant, $A^{21}$ et $R^1$ possèdent la signification précédente,
ou un composé répondant à la formule I, qui possède un groupe hydroxy libre, avec un composé répondant à la formule générale

$$R—X \qquad XII$$

dans laquelle R désigne un groupe alkyle inférieur, et X possède la signification précédente, ou

g) on fait réagir un composé répondant à la formule générale

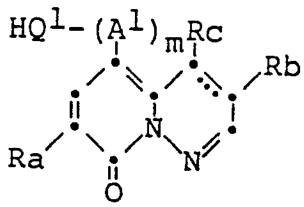

Ib

dans laquelle $A^1$, $Q^1$, Ra, Rb, Rc, m et la ligne pointillée possèdent la signification précédente,

en présence d'un agent fixant les acides, avec un dérivé réactif d'un acide carboxylique répondant à la formule générale

$$R^1—COOH \qquad XIII$$

dans laquelle $R^1$ possède la signification précédente, ou

h) on fait réagir un composé répondant à la formule générale

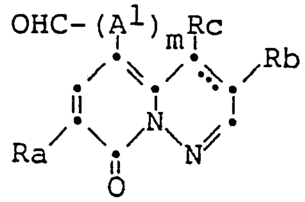

Ic

dans laquelle $A^1$, Ra, Rb, Rc, m et la ligne pointillée possèdent la signification précédente, en présence d'un agent réducteur, avec une amine répondant à la formule IX ou X précédente, ou

i) on réduit un composé répondant à la formule générale

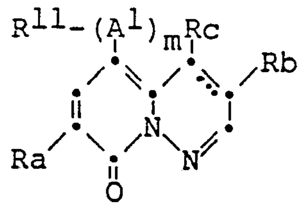

Id

dans laquelle $R^{11}$ désigne un groupe nitro, cyano ou (alcoxy inférieur)carbonyle et $A^1$, Ra, Rb, Rc, m et la ligne pointillée possèdent la signification précédemment indiquée, ou un composé répondant à la formule Ia précédente ou un de ces dérivés réactifs, ou

j) on oxyde un alcool répondant à la formule Ib' qui précéde ou un alcool répondant à la formule générale

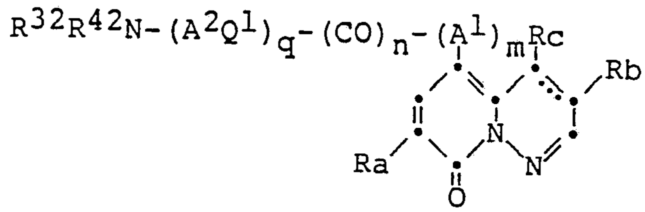

Ie

dans laquelle $A^1$, $A^2$, $Q^1$, Ra, Rb, Rc, m, n, q et la ligne pointillée possèdent la signification précédente, et $R^{32}$ et $R^{42}$ forment avec l'atome d'azote un hétérocycle azoté saturé à trois à sept maillons, substitué le cas échéant par un ou deux groupes alkyle inférieur et par un groupe hydroxy, qui peut encore contenir comme maillon du cycle un atome d'oxygène ou de soufre, ou le groupe >N—$R^5$ et $R^5$ possède la signification précédente ou

k) on fait réagir un isocyanate répondant à la formule générale

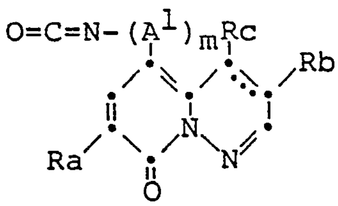

VIIIb ou $O=C-N-R^{33}$ XIV

dans laquelle $A^1$, Ra, Rb, Rc, m et la ligne pointillée possèdent la signification précédente, et $R^{33}$ désigne l'hydrogène, un groupe alkyle inférieur ou cycloalkyle en $C_3$ à $C_7$, avec un alcool inférieur ou une amine répondant à la formule X précédente, ou avec un composé répondant à la formule Ib précédente, ou

l) on fait réagir un composé répondant à la formule générale

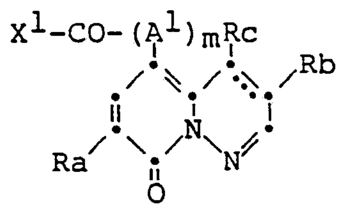

VIIIc

dans laquelle $X^1$ désigne un atome d'halogène, et $A^1$, Ra, Rb, Rc, m et la ligne pointillée possèdent la signification précédente, avec un halogénure d'alkylmagnésium inférieur, ou

m) on halogéne un composé répondant à la formule générale

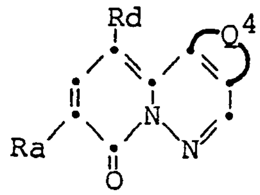

Ih

dans laquelle $Q^4$ désigne le groupe (a) ou (b) ci-dessus et Ra et Rd ont la signification précédente sur le cycle thiophène, ou

n) on fait réagir un composé répondant à la formule VIIIc précédente en présence d'une base, avec un composé répondant à la formule générale

$$HYN{=}C(NH_2){-}R'' \quad XV$$

$$H_2N{-}CHR''{-}CHR'''{-}Y'H \quad XVI$$

$$\text{ou } H_2N{-}NH{-}C(R''){=}Y'' \quad XVII$$

dans laquelle Y désigne un atome d'oxygène ou le groupe —NR'''—, Y' un atome d'oxygène ou le groupe —NH—, Y'' un atome d'oxygène ou de soufre et R'' et R''' chacun l'hydrogène ou un groupe alkyle inférieur,

et on cyclise le produit obtenu, ou

o) on fait réagir un composé répondant à la formule générale

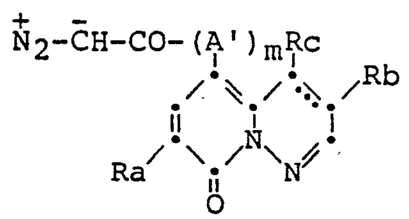

VIIId

dans laquelle A' désigne un groupe alkylène en $C_1$ à $C_6$, et Ra, Rb, Rc, la ligne pointillée et m possèdent la signification précédente,

avec un alcool inférieur, ou

p) on décarboxyle un acide carboxylique répondant à la formule Ia, dans laquelle m désigne le nombre 0, ou

q) on halogène un composé répondant à la formula I, dans lequel Rd désigne l'hydrogène, sur le cycle pyridone, ou

r) dans un composé répondant à la formule générale

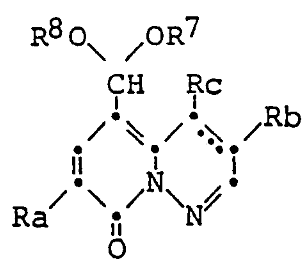

VIIIe

dans laquelle $R^7$ et $R^8$ désignent chacun un groupe alkyle inférieur ou ensemble un groupe alkylène inférieur, et Ra, Rb, Rc et la ligne pointillée possèdent la signification précédente,

on coupe le groupe acetal, ou

s) on fait réagir un composé répondant à la formule générale

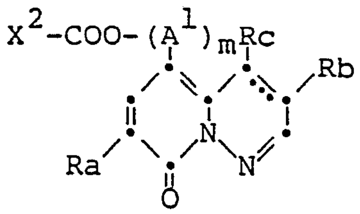

VIIIf

dans laquelle $X^2$ désigne un groupe phénoxy, et $A^1$, Ra, Rb, Rc, la ligne pointillée et m possèdent la signification précédente,
avec une amine répondant à la formule X précédente, et

t) si on le désire, on transforme un composé répondant à la formule I obtenue, portant un substituant basique, en un sel d'addition d'acide pharmaceutiquement acceptable.

2. Procédé selon la revendication 1 dans lequel Ra représente un phényle.

3. Procédé selon les revendications 1 ou 2, dans lequel Rb et Rc représentent avec l'atome de carbone désigné par α un groupe de formule $>C_\alpha$—S—CH=CH— ou $>C_\alpha$—CH=CH—CH=CH— substitué le cas échéant par un halogène et la ligne pointillée représente une liaison supplémentaire.

4. Procédé selon la revendication 3, dans lequel Rb et Rc représentent avec l'atome de carbone désigné par le groupe de formule $>C_\alpha$—S—CH=CH— ou $>C_\alpha$—CH=CCl—CH=CH—.

5. Procédé selon l'une des revendications 1—4, dans lequel $Q^1$ représente un atome d'oxygène, $A^2$ le groupe —CO—, $R^1$ le groupe —$NR^3R^4$, $R^3$ un alcoxyalkyle inférieur et $R^4$ un hydrogène ou un alkyle inférieur, ou $R^3$ et $R^4$ représentent avec l'atome d'azote un hétérocycle azoté saturé à 4, 5 ou 6 maillons substitué le cas échéant par un ou deux groupes alkyle inférieur et le cas échéant par un groupe hydroxy, alcoxy inférieur, hydroxyalkyle inférieur ou alcoxyalkyle inférieur, qui peut encore contenir comme maillon un atome d'oxygène, et ou bien m et q représentent le nombre 0 et n le nombre 1, ou bien m et q représentent le nombre 1 et n le nombre 0.

6. Procédé selon la revendication 5, dans lequel $A^1$ représente un méthylène et $R^3$ un alcoxyalkyle inférieur et $R^4$ représente un hydrogène ou un alkyle inférieur, ou $R^3$ et $R^4$ représentent avec l'atome d'azote un groupe 1-azétidinyle, 1-pyrrolidinyle, 1-pipéridinyle ou 4-morpholinyle substitué le cas échéant par un ou deux groupes alkyle et le cas échéant par un groupe hydroxy, alcoxy inférieur, hydroxyalkyle inférieur ou alcoxyalkyle inférieur.

7. Procédé selon la revendication 6, dans lequel $R^3$ représente un 2-(alcoxy inférieur)éthyle et $R^4$ un hydrogène ou un alkyle inférieur, ou $R^3$ et $R^4$ représentent avec l'atome d'azote un 3-(alcoxy inférieur)-1-azétidinyle, 3-(alcoxy inférieur)-1-pyrrolidinyle, 2-(alcoxyalkyle inférieur)-1-pyrrolidinyle, 2-(hydroxyalkyle inférieur)-1-pyrrolidinyle, 4-hydroxy-1-pipéridinyle, 4-(alcoxy inférieur)-1-pipéridinyle, 4-morpholinyle ou 2,6-di(alkyle inférieur)-4-morpholinyle.

8. Procédé selon l'une des revendications 1—7, dans lequel m et q représentent le nombre 0 et n le nombre 1.

9. Procédé selon l'une des revendications 1—4, dans lequel m et q représentent le nombre 0, n le nombre 1 et $R^1$ un hydroxy ou un alcoxy inférieur.

10. Procédé selon la revendication 1, caractérisé en ce qu'on prépare la 3-méthoxy-1-[(4-oxo-3-phényl-4H-pyrido[2,1-a]phtalazin-1-yl)carbonyl]azétidine.

11. Procédé de préparation de médicaments, en particulier de relaxants musculaires, d'agents à action sédative-hypnotique, anxiolytique et/ou anti-convulsive, caractérisé en ce qu'on met sous une forme d'administration galénique un composé de formule I définie dans la revendication 1 ou un sel d'addition d'acide pharmaceutiquement acceptable d'un composé de formule I avec un ou plusieurs substituants basiques et la cas échéant un autre corps thérapeutiquement efficace avec un support thérapeutiquement inerte.

12. Application de composés de formule I définie dans la revendication 1 à la préparation d'agents à action de relaxation musculaire, sédative-hypnotique, anxiolytique et/ou anticonvulsive.

**Claims for the Contracting States: BE CH DE FR GB IT LI LU NL SE**

1. Compounds of the general formula

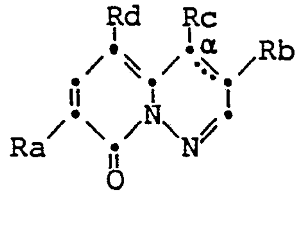

I

wherein Ra signifies a phenyl, pyridyl or thienyl group optionally substituted by halogen, trifluoromethyl, nitro, lower alkyl or lower alkoxy, Rb and Rc together with the carbon atom denoted by α signify a group of the formula $>C_α—S—CH=CH—$ (a), $>C_α—CH=CH—S—$ (b) or $>C_α—CH=CH—CH=CH—$ (c) which is optionally substituted by halogen, trifluoromethyl, lower alkyl, lower alkoxy, nitro, amino or mono- or di(lower alkyl)amino, the dotted line signifies an additional bond, Rd signifies the group of the formula $—(A^1)_m—(CO)_n—(Q^1A^2)_q—R^1$, m, n and q each signify the number 0 or 1, $A^1$ signifies lower alkylene, $A^2$ signifies lower alkylene, a direct bond or the group —CO—, $Q^1$ signifies an oxygen atom or the group $—NR^2—$, $R^1$ signifies hydrogen, hydroxy, cyano, nitro, halogen, lower alkoxy, lower alkyl, lower alkoxycarbonyl, aryl, a group of the formula $—NR^3R^4$ or a 5-membered saturated, partially unsaturated or aromatic heterocycle which is attached via a carbon atom and which is optionally substituted by one or two lower alkyl groups and optionally substituted by a $(C_{3-6})$-cycloalkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or alkylenedioxy group, $R^2$ signifies hydrogen, lower alkyl or aryl, $R^3$ and $R^4$ each signify hydrogen, lower alkyl, lower alkoxyalkyl, lower dialkoxyaryl, lower alkylenedioxyalkyl, lower cyanoalkyl, lower haloalkyl, lower hydroxyalkyl, lower dihydroxyalkyl, lower alkanoyl, lower alkoxycarbonyl or a $(C_{3-7})$-cycloalkyl group which is optionally substituted by hydroxy, lower alkoxy, lower alkanoyloxy, lower hydroxyalkyl, lower alkoxyalkyl, lower alkanoyloxyalkyl, oxo, carbamoyl, mono- or di(lower alkyl)carbamoyl or by lower alkylenedioxy or $R^3$ and $R^4$ together with the nitrogen atom signify a 3- to 7-membered saturated N-heterocycle which is optionally substituted by one or two lower alkyl groups and optionally substituted by one or two hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or lower alkylenedioxy groups and which can contain as a ring member an oxygen or sulphur atom or the group $>N—R^5$, and $R^5$ signifies hydrogen, lower alkyl, lower hydroxyalkyl, lower alkoxyalkyl, lower alkanoyloxyalkyl, lower alkanoyl, lower alkoxycarbonyl, carbamoyl or mono- or di(lower alkyl)carbamoyl, with the proviso that n signifies the number 0 when q signifies the number 1 and $A^2$ signifies the group —CO—, that $R^1$ has a significance different from cyano, nitro, halogen or lower alkoxycarbonyl when q signifies the number 0 and n signifies the number 1 or when q signifies the number 1 and $A^2$ signies the group —CO—, that $R^1$ has a significance different from hydroxy, cyano, nitro, halogen, lower alkoxycarbonyl, lower alkoxy and $—NR^3R^4$ when q signifies the number 1 and $A^2$ signifies a direct bond, that Rd has a significance different from acetyl when Ra signifies phenyl and Rb and Rc together signify the group —CH=CH—CH=CH—, that the residues denoted as lower have a maximum of seven carbon atoms and that aryl signifies a phenyl group which is optionally substituted by halogen, trifluoromethyl, lower alkyl, lower alkoxy, nitro, amino or mono- or di(lower alkyl)amino,

and pharmaceutically acceptable acid addition salts of compounds of formula I which have one or more basic substituents.

2. Compounds according to claim 1, wherein Ra signifies phenyl.

3. Compounds according to claim 1 or 2, wherein Rb and Rc together with the carbon atom denoted by α signify a group of the formula $>C_α—S—CH=CH—$ or $>C_α—CH=CH—CH=CH—$ which is optionally substituted by halogen and the dotted line signifies an additional bond.

4. Compounds according to claim 3, wherein Rb and Rc together with the carbon atom denoted by α signify the group of the formula $>C_α—S—CH=CH—$ or $>C_α—CH=CCl—CH=CH—$.

5. Compounds according to any one of claims 1—4, wherein $Q^1$ signifies an oxygen atom, $A^2$ signifies the group —CO—, $R^1$ signifies the group $—NR^3R^4$, $R^3$ signifies lower alkoxyalkyl and $R^4$ signifies hydrogen or lower alkyl or $R^3$ and $R^4$ together with the nitrogen atom signify a 4-, 5- or 6-membered saturated N-heterocycle which is optionally substituted by one or two lower alkyl groups and optionally substituted by a hydroxy, lower alkoxy, lower hydroxyalkyl or lower alkoxyalkyl group and which can contain as a ring member an oxygen atom and either m and q signify the number 0 and n signifies the number 1 or m and q signify the number 1 and n signifies the number 0.

6. Compounds according to claim 5, wherein $A^1$ signifies methylene and $R^3$ signifies lower alkoxyalkyl and $R^4$ signifies hydrogen or lower alkyl or $R^3$ and $R^4$ together with the nitrogen atom signify a 1-azetidinyl, 1-pyrrolidinyl, 1-piperidinyl or 4-morpholinyl group which is optionally substituted by one or two lower alkyl groups and optionally substituted by a hydroxy, lower alkoxy, lower hydroxyalkyl or lower alkoxyalkyl group.

7. Compounds according to claim 6, wherein $R^3$ signifies 2-(lower alkoxy)ethyl and $R^4$ signifies hydrogen or lower alkyl or $R^3$ and $R^4$ together with the nitrogen atom signify 3-(lower alkoxy)-1-azetidinyl, 3-(lower alkoxy)-1-pyrrolidinyl, 2-(lower alkoxyalkyl)-1-pyrrolidinyl, 2-(lower hydroxyalkyl)-1-pyrrolidinyl, 4-hydroxy-1-piperidinyl, 4-(lower alkoxy)-1-piperidinyl, 4-morpholinyl, or 2,6-di(lower alkyl)-4-morpholinyl.

8. Compounds according to any one of claims 1—7, wherein m and q signify the number 0 and n signifies the number 1.

9. Compounds according to any one of claims 1—4, wherein m and q signify the number 0, n signifies the number 1 and $R^1$ signifies hydroxy or lower alkoxy.

10. 3-Methoxy-1 [(4-oxo-3-phenyl-4H-pyrido[2,1-a]phthalazin-1-yl)carbonyl]azetidine.

11. Methyl 4-oxo-3-phenyl-4H-pyrido[2,1-a]phthalazine-1-carboxylate.

12. 4-Oxo-3-phenyl-4H-pyrido[2,1-a]phthalazine-1-carboxylic acid.

13. 3-Hydroxy-1-[(4-oxo-3-phenyl-4H-pyrido[2,1-a]phthalazin-1-yl)carbonyl]azetidine.

14. Compounds of the general formula

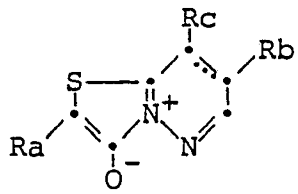

II

wherein Ra, Rb, Rc and the dotted line have the significance given in claim 1, with the proviso that Ra has a significance different from phenyl when Rb and Rc together signify the group —CH=CH—CH=CH— and the dotted line signifies an additional bond.

15. Compounds of the general formula

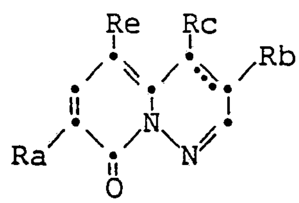

VIII

wherein Re signifies the group

$$—(A^1)_m—OOC—X^2,\ —(A^1)_m—(CO)_n—(Q^1A^{22})_q—NR^{31}R^{41},\ —(A^1)_m—N=C=O,\ —(A^1)_m—CO—X^1,$$

$$—(A')_m—(CO)—CH^-N_2^+ \text{ or } —CH(OR^7)OR^8, \text{ and}$$

$A^1$, $Q^1$, Ra, Rb, Rc, m, n, q and the dotted line have the significance given in claim 1 and A', $A^{22}$, $R^7$, $R^8$, $R^{31}$, $R^{41}$, $X^1$ and $X^2$ have the significance given in claim 19.

16. Compounds of the general formulae

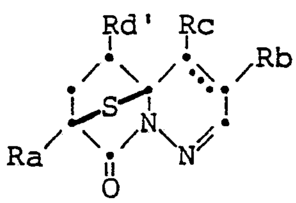

XVIII and

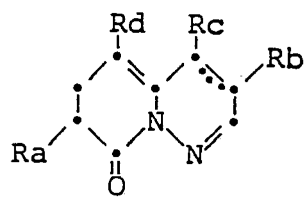

XIX

wherein Ra, Rb, Rc, Rd and the dotted line have the significance given in claim 1 and Rd' has the significance given in claim 19, with the proviso that Rd' has a significance different from acetyl and ethoxycarbonyl when Ra signifies phenyl, Rb and Rc together signify the group —CH=CH—CH=CH— and the dotted line signifies an additional bond.

17. Compounds according to any one of claims 1—13 for use as therapeutically active substances.

18. Compounds according to claim 17 for use as substances having muscle relaxant, sedative-hypnotic, anxiolytic and/or anticonvulsive activity.

19. A process for the manufacture of compounds according to any one of claims 1—13 and of pharmaceutically acceptable acid addition salts thereof, characterized by

a) reacting a compound of the general formula

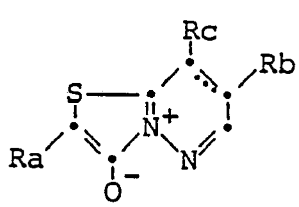

II

wherein Ra, Rb, Rc and the dotted line have the significance given in claim 1,
at an elevated temperature with a compound of the general formula

$$HC≡C—Rd' \text{ or} \qquad III$$

$$H_2C{=}CH{-}Rd' \qquad IV$$

wherein Rd' signifies cyano, nitro or the group of the formula $-CO-(Q^1A^2)_q-R^1$ and q, $A^2$, $Q^1$ and $R^1$ have the significance given in claim 1,
or with phenylvinyl sulphoxide and, if necessary, treating the cycloaddition product obtained with a strong base, or

b) reacting a compound of the general formula

$$ROOC{-}C(Ra){=}CHR' \qquad V$$

wherein R signifies lower alkyl, R' signifies hydrogen or lower alkoxy and Ra has the significance given in claim 1,
at an elevated temperature when R' signifies hydrogen or in the presence of a strong base when R' signifies lower alkoxy with a compound of the general formula

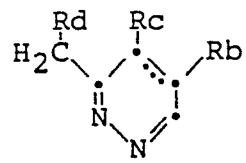

VI

wherein Rb, Rc, and Rd and the dotted line have the significance given in claim 1,
and dehydrogenating the cyclocondensation product obtained when R' signifies hydrogen, or

c) hydrolzying a compound of formula I which contains an esterified carboxy group, or

d) esterifying a carboxylic acid of the general formula

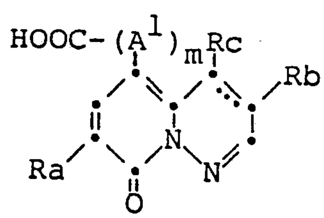

Ia

wherein $A^1$, Ra, Rb, Rc, m and the dotted line having the significance given in claim 1,
with an alcohol of the general formula

$$HO{-}A^{21}{-}R^1 \qquad VII$$

wherein $A^{21}$ signifies lower alkylene or a direct bond and $R^1$ has the significance given in claim 1,
or

e) converting a carboxylic acid of formula Ia above or a carboxylic acid of the general formula

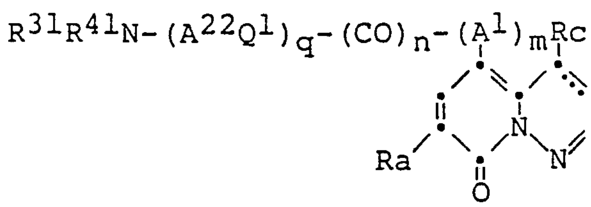

VIIIa

wherein $A^{22}$ signifies lower alkylene or the group —CO— and $R^{31}$ and $R^{41}$ together with the nitrogen atom signify a 3- to 7-membered saturated N-heterocycle which is optionally substituted by one or two lower alkyl groups and which is substituted by a carboxy group and which can contain as a ring member an oxygen or sulphur atom or the group $>N-R^5$, and $A^1$, $Q^1$, Ra, Rb, Rc, $R^5$, m, n, q and the dotted line have the significance give in claim 1,
or a reactive derivative thereof into the corresponding amide with, respectively, an amine of the general formula

$$HNR^2{-}A^{21}{-}R^1 \text{ or} \qquad IX$$

$$HNR^3R^4 \qquad X$$

wherein $R^1$, $R^2$, $R^3$ and $R^4$ have the significance given in claim 1 and $A^{21}$ has the above significance,
or with ammonia or a mono- or di(lower alkyl)amine, or

f) reacting a compound of the general formula

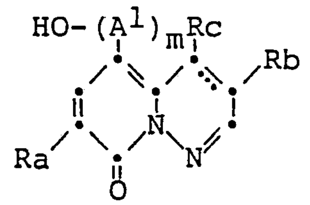

Ib'

wherein $A^1$, Ra, Rb, Rc, m and the dotted line have the significance given in claim 1, in the presence of a base with a compound of the general formula

$$X—A^{21}—R^1 \quad XI$$

wherein X signifies a leaving group, $A^{21}$ has the above significance and $R^1$ has the significance given in claim 1,
or reacting a compound of formula I which contains a free hydroxy group with a compound of the general formula

$$R—X \quad XII$$

wherein R signifies lower alkyl and X has the above significance,
or
g) reacting a compound of the general formula

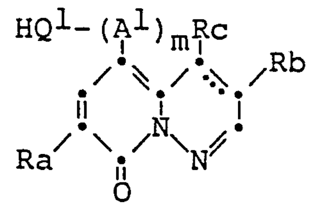

Ib

wherein $A^1$, $Q^1$, Ra, Rb, Rc, m and the dotted line have the significance given in claim 1, in the presence of an acid-binding agent with a reactive derivative of a carboxylic acid of the general formula

$$R^1—COOH \quad XIII$$

wherein $R^1$ has the significance given in claim 1,
or
h) reacting a compound of the general formula

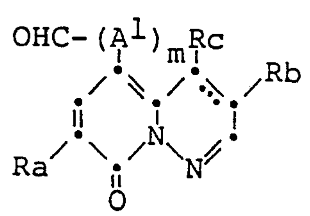

Ic

wherein $A^1$, Ra, Rb, Rc, m and the dotted line have the significance given in claim 1, in the presence of a reducing agent with an amine of formula IX or X above, or
i) reducing a compound of the general formula

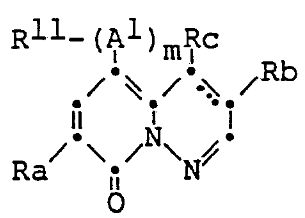

Id

wherein $R^{11}$ signifies nitro, cyano or lower alkoxycarbonyl and $A^1$, Ra, Rb, Rc, m and the dotted line have the significance given in claim 1,

or a compound of formula Ia above or a reactive derivative thereof, or

j) oxidizing an alcohol of formula Ib′ above or an alcohol of the general formula

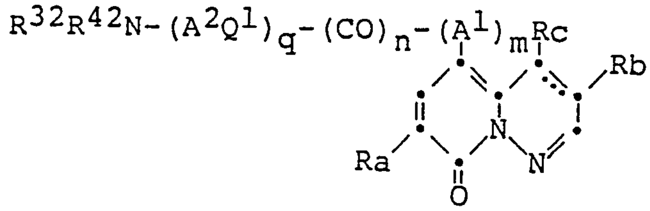

Ie

wherein $A^1$, $A^2$, $Q^1$, Ra, Rb, Rc, m, n, q and the dotted line have the significance given in claim 1 and $R^{32}$ and $R^{42}$ together with the nitrogen atom signify a 3- to 7-membered saturated N-heterocycle which is optionally substituted by one or two lower alkyl groups and which is substituted by a hydroxy group and which can contain as a ring member an oxygen or sulphur atom or the group $>N—R^5$, and $R^5$ has the significance given in claim 1,

or

k) reacting an isocyanate of the general formula

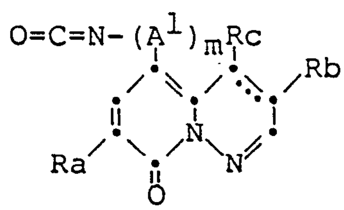

VIIIb or $O=C-N-R^{33}$ XIV

wherein $A^1$, Ra, Rb, Rc, m and the dotted line have the significance given in claim 1 and $R^{33}$ signifies hydrogen, lower alkyl or $(C_{3-7})$-cycloalkyl,

with, respectively, a lower alcohol or an amine of formula X above or with a compound of formula Ib above, or

l) reacting a compound of the general formula

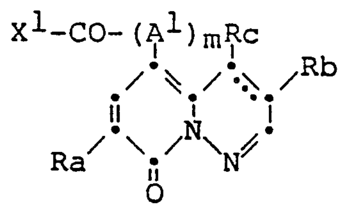

VIIIc

wherein $X^1$ signifies a halogen atom and $A^1$, Ra, Rb, Rc, m and the dotted line have the significance given in claim 1,

with a lower alkylmagnesium halide, or

m) halogenating a compound of the general formula

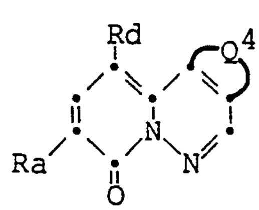

Ih

wherein $Q^4$ signifies the group (a) or (b) defined in claim 1 and Ra and Rd have the significance given in claim 1,

on the thiophene ring, or

n) reacting a compound of formula VIIIc above in the presence of a base with a compound of the general formula

$$HYN=C(NH_2)—R'', \qquad XV$$

$$H_2N—CHR''—CHR'''—Y'H \text{ or} \qquad XVI$$

$$H_2N—NH—C(R'')=Y'' \qquad XVII$$

wherein Y signifies an oxygen atom or the group $—NR'''—$, Y′ signifies an oxygen atom or the group

—NH—, Y'' signifies an oxygen or sulphur atom and R'' and R''' each signify hydrogen or lower alkyl, and cyclizing the product obtained, or

o) reacting a compound of the general formula

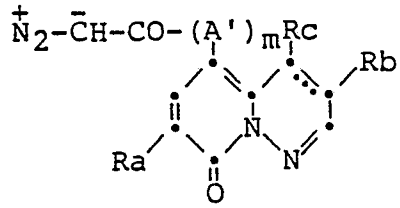

VIIId

wherein A' signifies $C_{1-6}$-alkylene and Ra, Rb, Rc, the dotted line and m have the significance given in claim 1,

with a lower alcohol, or

p) decarboxylating a carboxylic acid of formula Ia in which m signfies the number 0, or

q) halogenating a compound of formula I in which Rd signifies hydrogen on the pyridone ring, or

r) cleaving the acetal group in a compound of the general formula

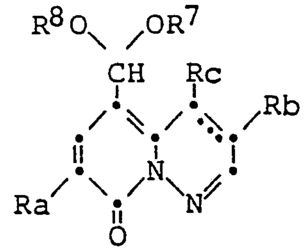

VIIIe

wherein $R^7$ and $R^8$ each signify lower alkyl or together signify lower alkylene and Ra, Rb, Rc and the dotted have the significance given in claim 1,

s) reacting a compound of the general formula

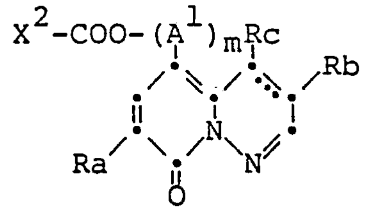

VIIIf

wherein $X^2$ signifies phenoxy and $A^1$, Ra, Rb, Rc, the dotted line and m have the significance given in claim 1,

with an amine of formula X above, and

t) if desired, converting a compound of formula I obtained which has a basic substituent into a pharmaceutically acceptable acid addition salt.

20. A medicament containing a compound according to any one of claims 1—13 and a therapeutically inert carrier material.

21. A medicament having muscle relaxant, sedative-hypnotic, anxiolytic and/or anticonvulsive activity, containing a compuond according to any one of claims 1—13 and a therapeutically inert carrier material.

22. The use of compounds according to any one of claims 1—13 for the manufacture of medicaments having muscle relaxant, sedative-hypnotic, anxiolytic and/or anticonvulsive activity.

23. The use of compounds according to any one of claims 14—16 for the manufacture of therapeutically active substances.

24. The use of compounds according to any one of claims 14—16 for the manufacture of compounds according to any one of claims 1—13.

**Claims for the Contracting State: GR**

1. A process for the manufacture of compounds of the general formula

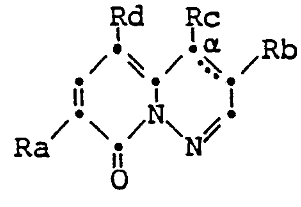

I

wherein Ra signifies a phenyl, pyridyl or thienyl group optionally substituted by halogen, trifluoromethyl, nitro, lower alkyl or lower alkoxy, Rb and Rc together with the carbon atom denoted by α signify a group of the formula $>C_\alpha$—S—CH=CH— (a), $>C_\alpha$—CH=CH—S— (b) or $>C_\alpha$—CH=CH—CH=CH— (c) which is optionally substituted by halogen, trifluoromethyl, lower alkyl, lower alkoxy, nitro, amino or mono- or di(lower alkyl)amino, the dotted line signifies an additional bond, Rd signifies the group of the formula $—(A^1)_m—(CO)_n—(Q^1A^2)_q—R^1$, m, n and q each signify the number 0 or 1, $A^1$ signifies lower alkylene, $A^2$ signifies lower alkylene, a direct bond or the group —CO—, $Q^1$ signifies an oxygen atom or the group $—NR^2—$, $R^1$ signifies hydrogen, hydroxy, cyano, nitro, halogen, lower alkoxy, lower alkyl, lower alkoxycarbonyl, aryl, a group of the formula $—NR^3R^4$ or a 5-membered saturated, partially unsaturated or aromatic heterocycle which is attached via a carbon atom and which is optionally substituted by one or two lower alkyl groups and optionally substituted by a $(C_{3-6})$-cycloalkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or alkylenedioxy group, $R^2$ signifies hydrogen, lower alkyl or aryl, $R^3$ and $R^4$ each signify hydrogen, lower alkyl, lower alkoxyalkyl, lower dialkoxyaryl, lower alkylenedioxyalkyl, lower cyanoalkyl, lower haloalkyl, lower hydroxyalkyl, lower dihydroxyalkyl, lower alkanoyl, lower alkoxycarbonyl or a $(C_{3-7})$-cycloalkyl group which is optionally substituted by hydroxy, lower alkoxy, loweralkanoyloxy, lower hydroxyalkyl, lower alkoxyalkyl, lower alkanoyloxyalkyl, oxo, carbamoyl, mono- or di(lower alkyl)carbamoyl or by lower alkylenedioxy or $R^3$ and $R^4$ together with the nitrogen atom signify a 3- to 7-membered saturated N-heterocycle which is optionally substituted by one or two lower alkyl groups and optionally substituted by one or two hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or lower alkylenedioxy groups and which can contain as a ring member an oxygen or sulphur atom or the group $>N—R^5$, and $R^5$ signifies hydrogen, lower alkyl, lower hydroxyalkyl, lower alkoxyalkyl, lower alkanoyloxyalkyl, lower alkanoyl, lower alkoxycarbonyl, carbamoyl or mono- or di(lower alkyl)carbamoyl, with the proviso that n signifies the number 0 when q signifies the number 1 and $A^2$ signifies the group —CO—, that $R^1$ has a significance different from cyano, nitro, halogen or lower alkoxycarbonyl when q signifies the number 0 and n signifies the number 1 or when q signifies the number 1 and $A^2$ signifies the group —CO—, that $R^1$ has a significance different from hydroxy, cyano, nitro, halogen, lower alkoxycarbonyl, lower alkoxy and $—NR^3R^4$ when q signifies the number 1 and $A^2$ signifies a direct bond, that Rd has a significance different from acetyl when Ra signifies phenyl and Rb and Rc together signify the group —CH=CH—CH=CH—, that the residues denoted as lower have a maximum of seven carbon atoms and that aryl signifies a phenyl group which is optionally substituted by halogen, trifluoromethyl, lower alkyl, lower alkoxy, nitro, amino or mono- or di(lower alkyl)amino,

and of pharmaceutically acceptable acid addition salts of compounds of formula I which have one or more basic substituents characterized by

a) reacting a compound of the general formula

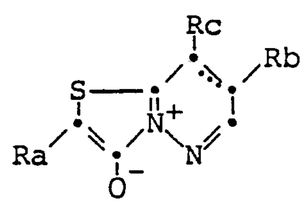

II

wherein Ra, Rb, Rc and the dotted line have the above significance,

at an elevated temperature with a compound of the general formula

HC≡C—Rd′ or III

$H_2C$=CH—Rd′ IV

wherein Rd′ signifies cyano, nitro or the group of the formula $—CO—(Q^1A^2)_q—R^1$ and q, $A^2$, $Q^1$ and $R^1$ have the above significance,

or with phenylvinyl sulphoxide and, if necessary, treating the cycloaddition product obtained with a strong base, or

b) reacting a compound of the general formula

ROOC—C(Ra)=CHR′ V

wherein R signifies lower alkyl, R′ signifies hydrogen or lower alkoxy and Ra has the above significance, at an elevated temperature when R′ signifies hydrogen or in the presence of a strong base when R′ signifies lower alkoxy with a compound of the general formula

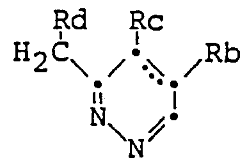

VI

wherein Rb, Rc, and Rd and the dotted line have the above significance, and dehydrogenating the cyclocondensation product obtained when R′ signifies hydrogen, or

c) hydrolzying a compound of formula I which contains an esterified carboxy group, or

d) esterifying a carboxylic acid of the general formula

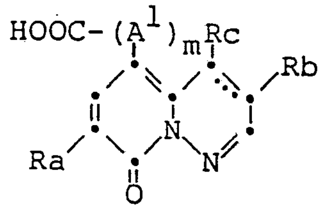

Ia

wherein $A^1$, Ra, Rb, Rc, m and the dotted line having the above significance, with an alcohol of the general formula

$$HO—A^{21}—R^1 \qquad VII$$

wherein $A^{21}$ signifies lower alkylene or a direct bond and $R^1$ has the above significance, or

e) converting a carboxylic acid of formula Ia above or a carboxylic acid of the general formula

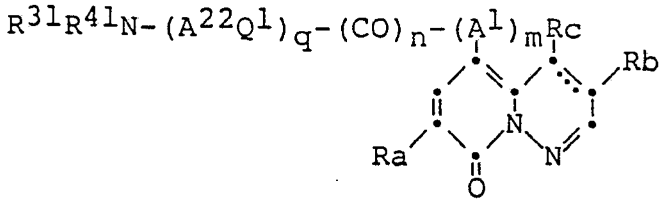

VIIIa

wherein $A^{22}$ signifies lower alkylene or the group —CO— and $R^{31}$ and $R^{41}$ together with the nitrogen atom signify a 3- to 7-membered saturated N-heterocycle which is optionally substituted by one or two lower alkyl groups and which is substituted by a carboxy group and which can contain as a ring member an oxygen or sulphur atom or the group >N—$R^5$, and $A^1$, $Q^1$, Ra, Rb, Rc, $R^5$, m, n, q and the dotted line have the above significance, or a reactive derivative thereof into the corresponding amide with, respectively, an amine of the general formula

$$HNR^2—A^{21}—R^1 \text{ or} \qquad IX$$

$$HNR^3R^4 \qquad X$$

wherein $A^{21}$, $R^1$, $R^2$, $R^3$ and $R^4$ have the above significance, or with ammonia or a mono- or di(lower alkyl)amine, or

f) reacting a compound of the general formula

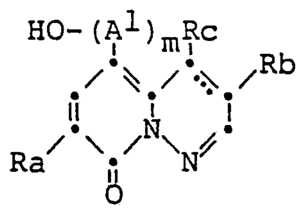

Ib'

wherein $A^1$, Ra, Rb, Rc, m and the dotted line have the above significance, in the presence of a base with a compound of the general formula

$$X—A^{21}—R^1 \qquad XI$$

wherein X signifies a leaving group, $A^{21}$ has the above significance and $R^1$ has the above significance,

or reacting a compound of formula I which contains a free hydroxy group with a compound of the general formula

R—X XII

wherein R signifies lower alkyl and X has the above significance,

or

g) reacting a compound of the general formula

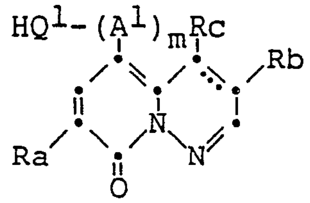

Ib

wherein $A^1$, $Q^1$, Ra, Rb, Rc, m and the dotted line have the above significance,

in the presence of an acid-binding agent with a reactive derivative of a carboxylic acid of the general formula

$R^1$—COOH XIII

wherein $R^1$ has the above significance,

or

h) reacting a compound of the general formula

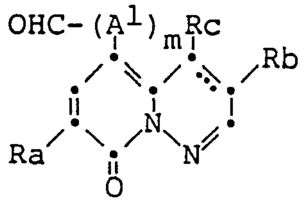

Ic

wherein $A^1$, Ra, Rb, Rc, m and the dotted line have the above significance,

in the presence of a reducing agent with an amine of formula IX or X above, or

i) reducing a compound of the general formula

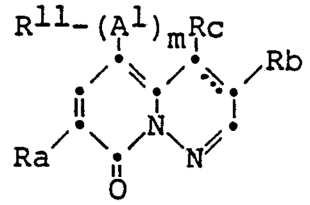

Id

wherein $R^{11}$ signifies nitro, cyano or lower alkoxycarbonyl and $A^1$, Ra, Rb, Rc, m and the dotted line have the above significance,

or a compound of formula Ia above or a reactive derivative thereof, or

j) oxidizing an alcohol of formula Ib′ above or an alcohol of the general formula

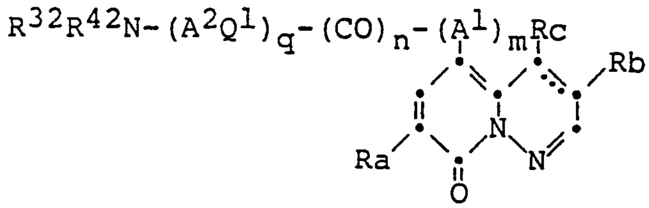

Ie

wherein $A^1$, $A^2$, $Q^1$, Ra, Rb, Rc, m, n, q and the dotted line have the above significance and $R^{32}$ and $R^{42}$ together with the nitrogen atom signify a 3- to 7-membered saturated N-heterocycle which is optionally substituted by one or two lower alkyl groups and which is substituted by a hydroxy group and which can contain as a ring member an oxygen or sulphur atom or the group >N—$R^5$, and $R^5$ has the above significance,

or

k) reacting an isocyanate of the general formula

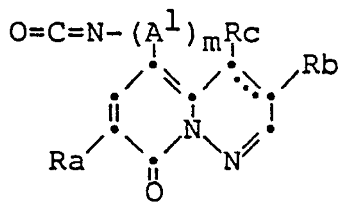

VIIIb or O=C-N-$R^{33}$ XIV

wherein $A^1$, Ra, Rb, Rc, m and the dotted line have the above significance and $R^{33}$ signifies hydrogen, lower alkyl or ($C_{3-7}$)-cycloalkyl,

with, respectively, a lower alcohol or an amine of formula X above or with a compound of formula Ib above, or

l) reacting a compound of the general formula

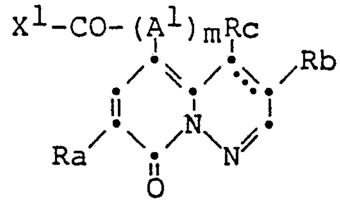

VIIIc

wherein $X^1$ signifies a halogen atom and $A^1$, Ra, Rb, Rc, m and the dotted line have the above significance,

with a lower alkylmagnesium halide, or

m) halogenating a compound of the general formula

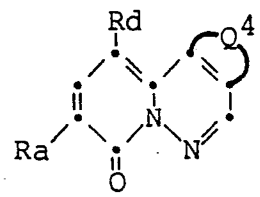

Ih

wherein $Q^4$ signifies the group (a) or (b) and Ra and Rd have the above significance,

on the thiophene ring, or

n) reacting a compound of formula VIIIc above in the presence of a base with a compound of the general formula

$$HYN{=}C(NH_2){-}R'', \qquad XV$$

$$H_2N{-}CHR''{-}CHR'''{-}Y'H \text{ or} \qquad XVI$$

$$H_2N{-}NH{-}C(R''){=}Y'' \qquad XVII$$

wherein Y signifies an oxygen atom or the group —NR'''—, Y' signifies an oxygen atom or the group —NH—, Y'' signifies an oxygen or sulphur atom and R'' and R''' each signify hydrogen or lower alkyl,

and cyclizing the product obtained, or

o) reacting a compound of the general formula

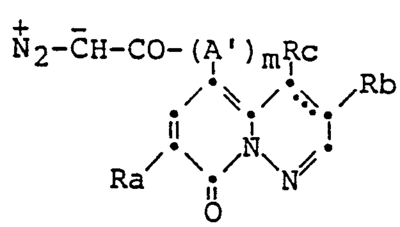

VIIId

wherein A' signifies $C_{1-6}$-alkylene and Ra, Rb, Rc, the dotted line and m have the above significance,

with a lower alcohol, or

p) decarboxylating a carboxylic acid of formula Ia in which m signifies the number 0, or

q) halogenating a compound of formula I in which Rd signifies hydrogen on the pyridone ring, or

r) cleaving the acetal group in a compound of the general formula

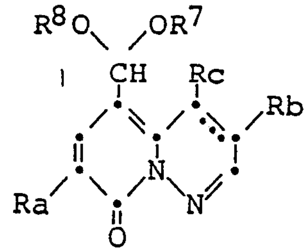

VIIIe

wherein $R^7$ and $R^8$ each signify lower alkyl or together signify lower alkylene and Ra, Rb, Rc and the dotted line have the above significance,

s) reacting a compound of the general formula

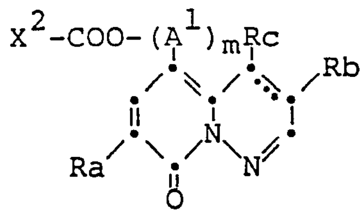

VIIIf

wherein $X^2$ signifies phenoxy and $A^1$, Ra, Rb, Rc, the dotted line and m have the above significance, with an amine of formula X above, and

t) if desired, converting a compound of formula I obtained which has a basic substituent into a pharmaceutically acceptable acid addition salt.

2. A process according to claim 1, wherein Ra signifies phenyl.

3. A process according to claim 1 or 2, wherein Rb and Rc together with the carbon atom denoted by α signify a group of the formula $>C_\alpha$—S—CH=CH— or $>C_\alpha$—CH=CH—CH=CH— which is optionally substituted by halogen and the dotted line signifies an additional bond.

4. A process according to claim 3, wherein Rb and Rc together with the carbon atom denoted by α signify the group of the formula $>C_\alpha$—S—CH=CH— or $>C_\alpha$—CH=CCl—CH=CH—.

5. A process according to any one of claims 1—4, wherein $Q^1$ signifies an oxygen atom, $A^2$ signifies the group —CO—, $R^1$ signifies the group —$NR^3R^4$, $R^3$ signifies lower alkoxyalkyl and $R^4$ signifies hydrogen or lower alkyl or $R^3$ and $R^4$ together with the nitrogen atom signify a 4-, 5- or 6-membered saturated N-heterocycle which is optionally substituted by one or two lower alkyl groups and optionally substituted by a hydroxy, lower alkoxy, lower hydroxyalkyl or lower alkoxyalkyl group and which can contain as a ring member an oxygen atom and either m and q signify the number 0 and n signifies the number 1 or m and q signify the number 1 and n signifies the number 0.

6. A process according to claim 5, wherein $A^1$ signifies methylene and $R^3$ signifies lower alkoxyalkyl and $R^4$ signifies hydrogen or lower alkyl or $R^3$ and $R^4$ together with the nitrogen atom signify a 1-azetidinyl, 1-pyrrolidinyl, 1-piperidinyl or 4-morpholinyl group which is optionally substituted by one or two lower alkyl groups and optionally substituted by a hydroxyl, lower alkoxy, lower hydroxyalkyl or lower alkoxyalkyl group.

7. A process according to claim 6, wherein $R^3$ signifies 2-(lower alkoxy)ethyl and $R^4$ signifies hydrogen or lower alkyl or $R^3$ and $R^4$ together with the nitrogen atom signify 3-(lower alkoxy)-1-azetidinyl, 3-(lower alkoxy)-1-pyrrolidinyl, 2-(lower alkoxyalkyl)-1-pyrrolidinyl, 2-(lower hydroxyalkyl)-1-pyrrolidinyl, 4-hydroxy-1-piperidinyl, 4-(lower alkoxy)-1-piperidinyl, 4-morpholinyl, or 2,6-di(lower alkyl)-4-morpholinyl.

8. A process according to any one of claims 1—7, wherein m and q signify the number 0 and n signifies the number 1.

9. A process according to any one of claims 1—4, wherein m and q signify the number 0, n signifies the number 1 and $R^1$ signifies hydroxy or lower alkoxy.

10. A process according to claim 1, characterized in that 3-methoxy-1-[(4-oxo-3-phenyl-4H-pyrido[2,1-a]phthalazin-1-yl)carbonyl]azetidine is manufactured.

11. A process for the manufacture of medicaments, especially of medicaments having muscle relaxant, sedative-hypnotic, anxiolytic and/or anticonvulsive activity, characterized by bringing a compound of formula I defined in claim 1 or a pharmaceutically acceptable acid addition salt of a compound of formula I which has one or more basic substituents and, if desired, another therapeutically active substance into a galenical administration form together with a therapeutically inert carrier material.

12. The use of compounds of formula I defined in claim 1 for the manufacture of medicaments having muscle relaxant, sedative-hypnotic, anxiolytic and/or anti-convulsive activity.

13. Compounds of the general formula

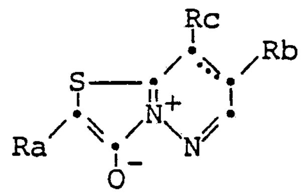

II

wherein Ra, Rb, Rc and the dotted line have the significance given in claim 1, with the proviso that Ra has a significance different from phenyl when Rb and Rc together signify the group —CH=CH—CH=CH— and the dotted line signifies an additional bond.

14. Compounds of the general formula

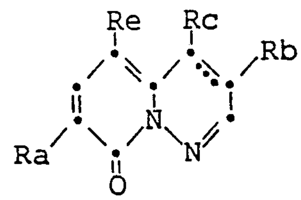

VIII

wherein Re signifies the group

$$—(A^1)_m—OOC—X^2,\ —(A^1)_m—(CO)_n—(Q^1A^{22})_q—NR^{31}R^{41},\ —(A^1)_m—N=C=O,\ —(A^1)_m—CO—X^1,$$

$$—(A')_m—(CO)—CH^-N_2^+ \text{ or } —CH(OR^7)OR^8, \text{ and}$$

$A^1$, $Q^1$, Ra, Rb, Rc, m, n, q and the dotted line as well as A', $A^{22}$, $R^7$, $R^8$, $R^{31}$, $R^{41}$, $X^1$ and $X^2$ have the significance given in claim 1.

15. Compounds of the general formulae

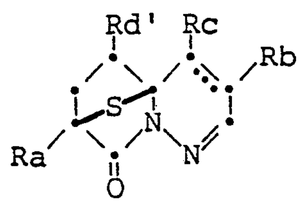

XVIII and

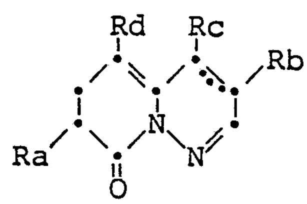

XIX

wherein Ra, Rb, Rc, Rd and the dotted line as well as Rd' have the significance given in claim 1, with the proviso that Rd' has a significance different from acetyl and ethoxycarbonyl when Ra signifies phenyl, Rb and Rc together signify the group —CH=CH—CH=CH— and the dotted line signifies an additional bond.

**Claims for the Contracting State: AT ES**

1. A process for the manufacture of compounds of the general formula

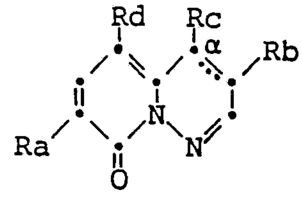

I

wherein Ra signifies a phenyl, pyridyl or thienyl group optionally substituted by halogen, trifluoromethyl, nitro, lower alkyl or lower alkoxy, Rb and Rc together with the carbon atom denoted by α signify a group of the formula $>C_α—S—CH=CH—$ (a), $>C_α—CH=CH—S—$ (b) or $>C_α—CH=CH—CH=CH—$ (c) which is optionally substituted by halogen, trifluoromethyl, lower alkyl, lower alkoxy, nitro, amino or mono- or di(lower alkyl)amino, the dotted line signifies an additional bond, Rd signifies the group of the formula $—(A^1)_m—(CO)_n—(Q^1A^2)_q—R^1$, m, n and q each signify the number 0 or 1, $A^1$ signifies lower alkylene, $A^2$ signifies lower alkylene, a direct bond or the group

—CO—, $Q^1$ signifies an oxygen atom or the group —$NR^2$—, $R^1$ signifies hydrogen, hydroxy, cyano, nitro, halogen, lower alkoxy, lower alkyl, lower alkoxycarbonyl, aryl, a group of the formula —$NR^3R^4$ or a 5-membered saturated, partially unsaturated or aromatic heterocycle which is attached via a carbon atom and which is optionally substituted by one or two lower alkyl groups and optionally substituted by a ($C_{3-6}$)-cycloalkyl, hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or alkylenedioxy group, $R^2$ signifies hydrogen, lower alkyl or aryl, $R^3$ and $R^4$ each signify hydrogen, lower alkyl, lower alkoxyalkyl, lower dialkoxyaryl, lower alkylenedioxyalkyl, lower cyanoalkyl, lower haloalkyl, lower hydroxyalkyl, lower dihydroxyalkyl, lower alkanoyl, lower alkoxycarbonyl or a ($C_{3-7}$)-cycloalkyl group which is optionally substituted by hydroxy, lower alkoxy, lower alkanoyloxy, lower hydroxyalkyl, lower alkoxyalkyl, lower alkanoyloxyalkyl, oxo, carbamoyl, mono- or di(lower alkyl)carbamoyl or by lower alkylenedioxy or $R^3$ and $R^4$ together with the nitrogen atom signify a 3- to 7-membered saturated N-heterocycle which is optionally substituted by one or two lower alkyl groups and optionally substituted by one or two hydroxy, lower alkoxy, lower alkanoyloxy, lower hydroxyalkyl, lower alkoxyalkyl, lower alkanoyloxyalkyl, lower alkoxycarbonyl, lower alkanoyl, carbamoyl, mono- or di(lower alkyl)carbamoyl, oxo or lower alkylenedioxy groups and which can contain as a ring member an oxygen or sulphur atom or the group >N—$R^5$, and $R^5$ signifies hydrogen, lower alkyl, lower hydroxyalkyl, lower alkoxyalkyl, lower alkanoyloxyalkyl, lower alkanoyl, lower alkoxycarbonyl, carbamoyl or mono- or di(lower alkyl)carbamoyl, with the proviso that n signifies the number 0 when q signifies the number 1 and $A^2$ signifies the group —CO—, that $R^1$ has a significance different from cyano, nitro, halogen or lower alkoxycarbonyl when q signifies the number 0 and n signifies the number 1 or when q signifies the number 1 and $A^2$ signifies the group —CO—, that $R^1$ has a significance different from hydroxy, cyano, nitro, halogen, lower alkoxycarbonyl, lower alkoxy and —$NR^3R^4$ when q signifies the number 1 and $A^2$ signifies a direct bond, that Rd has a significance different from acetyl when Ra signifies phenyl and Rb and Rc together signify the group —CH=CH—CH=CH—, that the residues denoted as lower have a maximum of seven carbon atoms and that aryl signifies a phenyl group which is optionally substituted by halogen, trifluoromethyl, lower alkyl, lower alkoxy, nitro, amino or mono- or di(lower alkyl)amino,

and of pharmaceutically acceptable acid addition salts of compounds of formula I which have one or more basic substituents characterized by

a) reacting a compound of the general formula

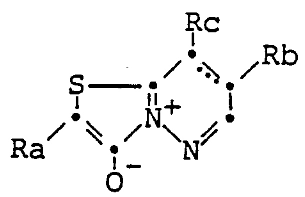

II

wherein Ra, Rb, Rc and the dotted line have the above significance,

at an elevated temperature with a compound of the general formula

$$HC \equiv C—Rd' \text{ or} \qquad III$$

$$H_2C=CH—Rd' \qquad IV$$

wherein Rd' signifies cyano, nitro or the group of the formula —CO—$(Q^1A^2)_q$—$R^1$ and q, $A^2$, $Q^1$ and $R^1$ have the above significance,

or with phenylvinyl sulphoxide and, if necessary, treating the cycloaddition product obtained with a strong base, or

b) reacting a compound of the general formula

$$ROOC—C(Ra)=CHR' \qquad V$$

wherein R signifies lower alkyl, R' signifies hydrogen or lower alkoxy and Ra has the above significance, at an elevated temperature when R' signifies hydrogen or in the presence of a strong base when R' signifies lower alkoxy with a compound of the general formula

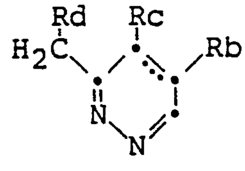

VI

wherein Rb, Rc, and Rd and the dotted line have the above significance,
and dehydrogenating the cyclocondensation product obtained when R′ signifies hydrogen, or

c) hydrolzying a compound of formula I which contains an esterified carboxy group, or

d) esterifying a carboxylic acid of the general formula

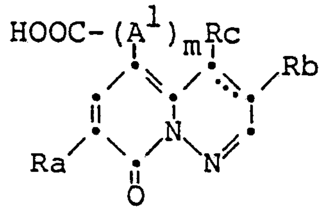

Ia

wherein $A^1$, Ra, Rb, Rc, m and the dotted line have the above significance,
with an alcohol of the general formula

$$HO—A^{21}—R^1 \qquad VII$$

wherein $A^{21}$ signifies lower alkylene or a direct bond and $R^1$ has the above significance,
or

e) converting a carboxylic acid of formula Ia above or a carboxylic acid of the general formula

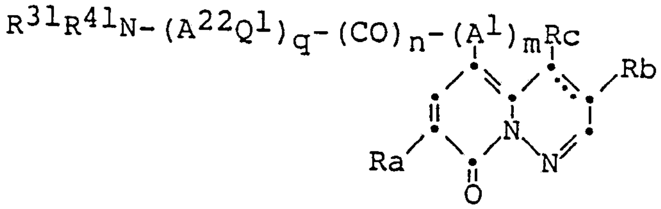

VIIIa

wherein $A^{22}$ signifies lower alkylene or the group —CO— and $R^{31}$ and $R^{41}$ together with the nitrogen atom signify a 3- to 7-membered saturated N-heterocycle which is optionally substituted by one or two lower alkyl groups and which is substituted by a carboxy group and which can contain as a ring member an oxygen or sulphur atom or the group >N—$R^5$, and $A^1$, $Q^1$, Ra, Rb, Rc, $R^5$, m, n, q and the dotted line have the above significance,
or a reactive derivative thereof into the corresponding amide with, respectively, an amine of the general formula

$$HNR^2—A^{21}—R^1 \text{ or} \qquad IX$$

$$HNR^3R^4 \qquad X$$

wherein $A^{21}$, $R^1$, $R^2$, $R^3$ and $R^4$ have the above significance,
or with ammonia or a mono- or di(lower alkyl)amine, or

f) reacting a compound of the general formula

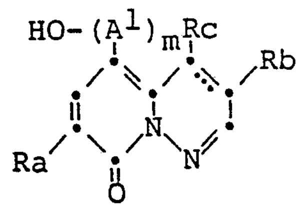

Ib'

wherein $A^1$, Ra, Rb, Rc, m and the dotted line have the above significance,
in the presence of a base with a compound of the general formula

$$X—A^{21}—R^1 \qquad XI$$

wherein X signifies a leaving group, $A^{21}$ has the above significance and $R^1$ has the above significance,
or reacting a compound of formula I which contains a free hydroxy group with a compound of the general formula

R—X XII

wherein R signifies lower alkyl and X has the above significance,

or

g) reacting a compound of the general formula

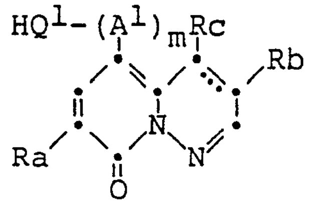

Ib

wherein $A^1$, $Q^1$, Ra, Rb, Rc, m and the dotted line have the above significance, in the presence of an acid-binding agent with a reactive derivative of a carboxylic acid of the general formula

$R^1$—COOH XIII

wherein $R^1$ has the above significance,

or

h) reacting a compound of the general formula

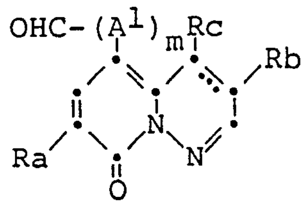

Ic

wherein $A^1$, Ra, Rb, Rc, m and the dotted line have the above significance, in the presence of a reducing agent with an amine of formula IX or X above, or

i) reducing a compound of the general formula

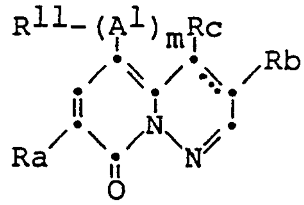

Id

wherein $R^{11}$ signifies nitro, cyano or lower alkoxycarbonyl and $A^1$, Ra, Rb, Rc, m and the dotted line have the above significance,

or a compound of formula Ia above or a reactive derivative thereof, or

j) oxidizing an alcohol of formula Ib' above or an alcohol of the general formula

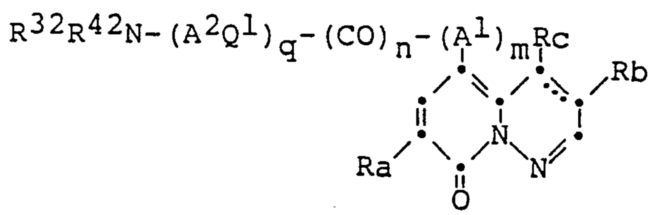

Ie

wherein $A^1$, $A^2$, $Q^1$, Ra, Rb, Rc, m, n, q and the dotted line have the above significance and $R^{32}$ and $R^{42}$ together with the nitrogen atom signify a 3- to 7-membered saturated N-heterocycle which is optionally substituted by one or two lower alkyl groups and which is substituted by a hydroxy group and which can contain as a ring member an oxygen or sulphur atom or the group >N—$R^5$, and $R^5$ has the above significance,

or

k) reacting an isocyanate of the general formula

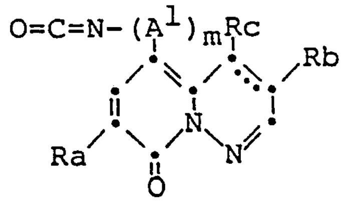

VIIIb oder $O{=}C{-}N{-}R^{33}$ XIV

wherein $A^1$, Ra, Rb, Rc, m and the dotted line have the above significance 1 and $R^{33}$ signifies hydrogen, lower alkyl or $(C_{3-7})$-cycloalkyl,

with, respectively, a lower alcohol or an amine of formula X above or with a compound of formula Ib above, or

l) reacting a compound of the general formula

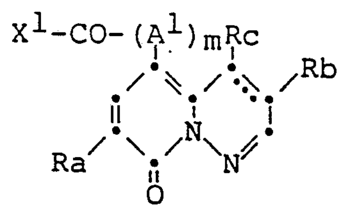

VIIIc

wherein $X^1$ signifies a halogen atom and $A^1$, Ra, Rb, Rc, m and the dotted line have the above significance,

with a lower alkylmagnesium halide, or

m) halogenating a compound of the general formula

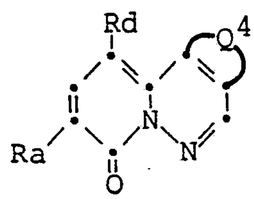

Ih

wherein $Q^4$ signifies the group (a) or (b) and Ra and Rd have the above significance,

on the thiophene ring, or

n) reacting a compound of formula VIIIc above in the presence of a base with a compound of the general formula

$$HYN{=}C(NH_2){-}R'', \qquad XV$$

$$H_2N{-}CHR''{-}CHR'''{-}Y'H \text{ or} \qquad XVI$$

$$H_2N{-}NH{-}C(R''){=}Y'' \qquad XVII$$

wherein Y signifies an oxygen atom or the group —NR'''—, Y' signifies an oxygen atom or the group —NH—, Y'' signifies an oxygen or sulphur atom and R'' and R''' each signify hydrogen or lower alkyl,

and cyclizing the product obtained, or

o) reacting a compound of the general formula

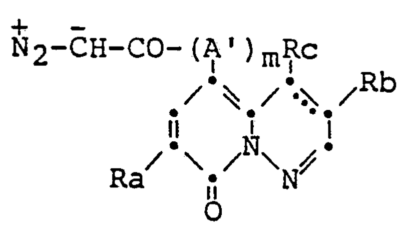

VIIId

wherein A' signifies $C_{1-6}$-alkylene and Ra, Rb, Rc, the dotted line and m have the above significance,

with a lower alcohol, or

p) decarboxylating a carboxylic acid of formula Ia in which m signifies the number 0, or

q) halogenating a compound of formula I in which Rd signifies hydrogen on the pyridone ring, or

r) cleaving the acetal group in a compound of the general formula

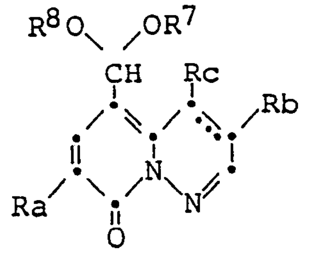

VIIIe

wherein $R^7$ and $R^8$ each signify lower alkyl or together signify lower alkylene and Ra, Rb, Rc and the dotted line have the above significance,

s) reacting a compound of the general formula

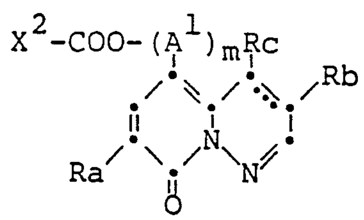

VIIIf

wherein $X^2$ signifies phenoxy and $A^1$, Ra, Rb, Rc, the dotted line and m have the above significance, with an amine of formula X above, and

t) if desired, converting a compound of formula I obtained which has a basic substituent into a pharmaceutically acceptable acid addition salt.

2. A process according to claim 1, wherein Ra signifies phenyl.

3. A process according to claim 1 or 2, wherein Rb and Rc together with the carbon atom denoted by α signify a group of the formula $>C_\alpha$—S—CH=CH— or $>C_\alpha$—CH=CH—CH=CH— which is optionally substituted by halogen and the dotted line signifies an additional bond.

4. A process according to claim 3, wherein Rb and Rc together with the carbon atom denoted by α signify the group of the formula $>C_\alpha$—S—CH=CH— or $>C_\alpha$—CH=CCl—CH=CH—.

5. A process according to any one of claims 1—4, wherein $Q^1$ signifies an oxygen atom, $A^2$ signifies the group —CO—, $R^1$ signifies the group $—NR^3R^4$, $R^3$ signifies lower alkoxyalkyl and $R^4$ signifies hydrogen or lower alkyl or $R^3$ and $R^4$ together with the nitrogen atom signify a 4-, 5- or 6-membered saturated N-heterocycle which is optionally substituted by one or two lower alkyl groups and optionally substituted by a hydroxy, lower alkoxy, lower hydroxyalkyl or lower alkoxyalkyl group and which can contain as a ring member an oxygen atom and either m and q signify the number 0 and n signifies the number 1 or m and q signify the number 1 and n signifies the number 0.

6. A process according to claim 5, wherein $A^1$ signifies methylene and $R^3$ signifies lower alkoxyalkyl and $R^4$ signifies hydrogen or lower alkyl or $R^3$ and $R^4$ together with the nitrogen atom signify a 1-azetidinyl, 1-pyrrolidinyl, 1-piperidinyl or 4-morpholinyl group which is optionally substituted by one or two lower alkyl groups and optionally substituted by a hydroxyl, lower alkoxy, lower hydroxyalkyl or lower alkoxyalkyl group.

7. A process according to claim 6, wherein $R^3$ signifies 2-(lower alkoxy)ethyl and $R^4$ signifies hydrogen or lower alkyl or $R^3$ and $R^4$ together with the nitrogen atom signify 3-(lower alkoxy)-1-azetidinyl, 3-(lower alkoxy)-1-pyrrolidinyl, 2-(lower alkoxyalkyl)-1-pyrrolidinyl, 2-(lower hydroxyalkyl)-1-pyrrolidinyl, 4-hydroxy-1-piperidinyl, 4-(lower alkoxy)-1-piperidinyl, 4-morpholinyl, or 2,6-di(lower alkyl)-4-morpholinyl.

8. A process according to any one of claims 1—7, wherein m and q signify the number 0 and n signifies the number 1.

9. A process according to any one of claims 1—4, wherein m and q signify the number 0, n signifies the number 1 and $R^1$ signifies hydroxy or lower alkoxy.

10. A process according to claim 1, characterized in that 3-Methoxy-1 [(4-oxo-3-phenyl-4H-pyrido[2,1-a]phthalazin-1-yl)carbonyl]azetidine is manufactured.

11. A process for the manufacture of medicaments, especially of medicaments having muscle relaxant, sedative-hypnotic, anxiolytic and/or anticonvulsive activity, characterized by bringing a compound of formula I defined in claim 1 or a pharmaceutically acceptable acid addition salt of a compound of formula I which has one or more basic substituents and, if desired, another therapeutically active substance into a galenical administration form together with a therapeutically inert carrier material.

12. The use of compounds of formula I defined in claim 1 for the manufacture of medicaments having muscle relaxant, sedative-hypnotic, anxiolytic and/or anti-convulsive activity.